# EUROPEAN PATENT APPLICATION

(11) **EP 4 647 432 A1**
(43) Date of publication of application: **12.11.2025**
(21) Application number: 24750327.9
(22) Date of filing: 31.01.2024
(51) Int. Cl.: C07D 495/04, H01L 29/786, H10K 10/46

(54) **AROMATIC COMPOUND, ORGANIC SEMICONDUCTOR LAYER, AND ORGANIC THIN FILM TRANSISTOR**

(30) Priority: 01.02.2023 JP 2023014302; 22.11.2023 JP 2023197948
(71) Applicant: Tosoh Corporation, Yamaguchi 746-8501 (JP)
(72) Inventor: MIYASHITA, Masato, Yokkaichi-shi, Mie 510-8540 (JP); HARADA, Shotaro, Yokkaichi-shi, Mie 510-8540 (JP); WATANABE, Makoto, Yokkaichi-shi, Mie 510-8540 (JP); OKU Shinya, Yokkaichi-shi, Mie 510-8540 (JP)
(74) Representative: Novagraaf International SA
(86) International application number: PCT/JP2024/003008
(87) International publication number: WO 2024/162378

(57) **Abstract**

Provided is an aromatic compound that is a coating-type organic semiconductor material having high carrier mobility, high heat resistance and high solubility. The aromatic compound is indicated by formula (1-I) or (1-II) below, where Ar denotes, for example, a monocyclic ring, X¹ and X² denote, for example, oxygen atoms, Y¹ and Y² denote, for example, CR⁶, R¹ to R⁶ denote, for example, hydrogen atoms, and at least one of R¹ to R⁶ is a group represented by formula (2) below. k and m denote 0 or 1, n denotes an integer of 1 to 8, l denotes an integer of 1 to 20, and Z¹ and Z² denote, for example, hydrogen atoms.

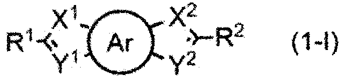

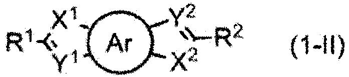

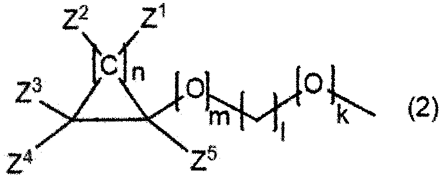

## Description

### Technical Field

The present invention relates to a novel aromatic compound that can be expanded to electronic materials, such as organic semiconductor materials, and to an organic semiconductor layer and an organic thin film transistor made using it. In particular, the present invention relates to an aromatic compound having particular substituents that is applicable to various device fabrication processes by virtue of its excellent solubility and heat resistance and to an organic semiconductor layer and an organic thin film transistor made using it.

### Background Art

In recent years, organic semiconductor devices, typified by organic thin film transistors, have been attracting attention because of their features not seen in inorganic semiconductor devices, such as energy conservation, low cost and flexibility. Organic semiconductor devices are composed of several materials, such as an organic semiconductor layer, a substrate, an insulating layer and electrodes. In particular, the organic semiconductor layer, which is responsible for charge carrier movement, plays a central role in the device. Since the performance of an organic semiconductor device is influenced by the carrier mobility of the organic semiconductor material constituting this organic semiconductor layer, furthermore, there is a need for the emergence of an organic semiconductor material that provides high carrier mobility.

Well-known methods for producing the organic semiconductor layer include, for example, vacuum deposition, which is conducted by atomizing an organic material in a vacuum at high temperatures, and coating, in which an organic material is dissolved in a suitable solvent and the resulting solution is applied. Of these, coating can be performed using printing techniques without using high-temperature and high-vacuum conditions. A significant reduction in manufacturing costs in device fabrication, therefore, is expected, making coating an economically favorable process.

The organic semiconductor material used in the coating method preferably has heat resistance of 130°C or above and a solubility at room temperature of 0.1% by weight or more for high carrier mobility and in consideration of the device fabrication process. In the case of transistors for use in electronic paper applications, furthermore, it is preferred that the carrier mobility be 0.1 cm²/V·sec or more.

Here, it is generally known that small-molecule semiconductors having a rod-shaped molecular major axis formed by a fused ring system are more likely to exhibit high carrier mobility because of their higher crystallinity compared with polymeric semiconductors. Examples of currently proposed small-molecule materials include 2,7-dialkyl-substituted benzothienobenzothiophenes (e.g., see PTL 1 and NPL 1), 2,7-diphenyl[1]benzothieno[3,2-b][1]benzothiophene (e.g., see NPL 2) and dithienobiphenylene derivatives (e.g., see PTL 2).

### Citation List

### Patent Literature

PTL 1: International Publication No. WO 2008/047896
PTL 2: International Publication No. WO 2021/177417

### Non Patent Literature

NPL 1: Journal of the American Chemical Society, 2007, vol. 129, pp. 15732 to 15733
NPL 2: Journal of the American Chemical Society, 2006, vol. 128, pp. 12604 to 12605

### Summary of Invention

### Technical Problem

Small-molecule semiconductors, however, generally have the problem of low solubility. To address this, some reported semiconductors contain an introduced alkyl group to improve solubility. This, however, involves the disadvantage that carrier mobility and heat resistance decrease. There are also reports of semiconductors to which an aromatic substituent has been introduced to develop high carrier mobility resulting from π-stacking, but the development of high mobility is reportedly achieved at the cost of a significant decrease in solubility. The status quo is that few known small-molecule organic semiconductor materials combine high carrier mobility, high heat resistance and high solubility.

For example, the dialkyl-substituted benzothienobenzothiophenes described in PTL 1 and NPL 1 are disadvantageous in terms of heat resistance; heating to 130°C or above causes the transistor operation to be lost.

2,7-diphenyl[1]benzothieno[3,2-b] [1]benzothiophene, which is described in NPL 2, has a problem with solubility; it is generally sparingly soluble in organic solvents.

The alkyl-substituted dithienobiphenylene derivatives described in PTL 2 are suitable for use in organic semiconductors as they combine high heat resistance and adequate solubility. There is, however, a need for a compound having even higher solubility to enable a wider range of device fabrication processes.

The present invention was made in light of these problems, and an object thereof is to provide a novel coating-type organic semiconductor material having high carrier mobility with high heat resistance and high solubility.

### Solution to Problem

After extensive research to solve the above problems, the inventors found that a novel aromatic compound having particular substituents functions as an organic semiconductor material that provides high carrier mobility and also has high heat resistance and high solubility, thereby completing the present invention.

That is, the present invention relates to an aromatic compound indicated by either formula (1-1) or formula (1-II) below, an organic semiconductor layer containing the aromatic compound and an organic thin film transistor including the organic semiconductor layer. 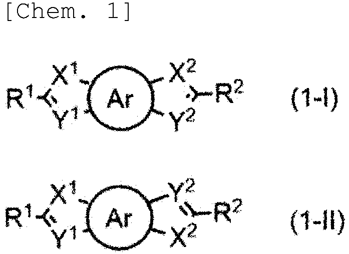 [(In the formula, Ar denotes a monocyclic ring or a fused ring system of two to six rings. X¹ and X² each independently denote one selected from the group consisting of an oxygen atom, a sulfur atom, a selenium atom, NR³ and CR⁴=CR⁵. Y¹ and Y² each independently denote either CR⁶ or a nitrogen atom. R¹ to R⁶ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) below, with at least one of R¹ to R⁶ being a group indicated by formula (2) below.) 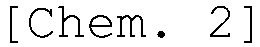 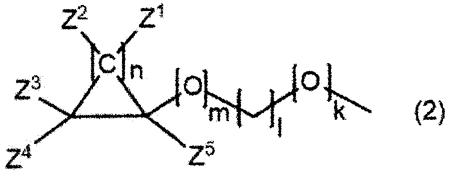 (In the formula, k and m each independently denote 0 or 1, n denotes an integer of 1 to 8, and l denotes an integer of 1 to 20. Z¹ to Z⁵ indicate one selected from the group consisting of a hydrogen atom, a halogen atom and a C1 to C20 alkyl group, being the same or different at each occurrence.)]

### Advantageous Effects of Invention

The novel aromatic compound according to the present invention provides high carrier mobility and also has high heat resistance and high solubility. Accordingly, it enables the provision of an organic thin film transistor that exhibits excellent semiconductor properties through coating.

### Brief Description of Drawings

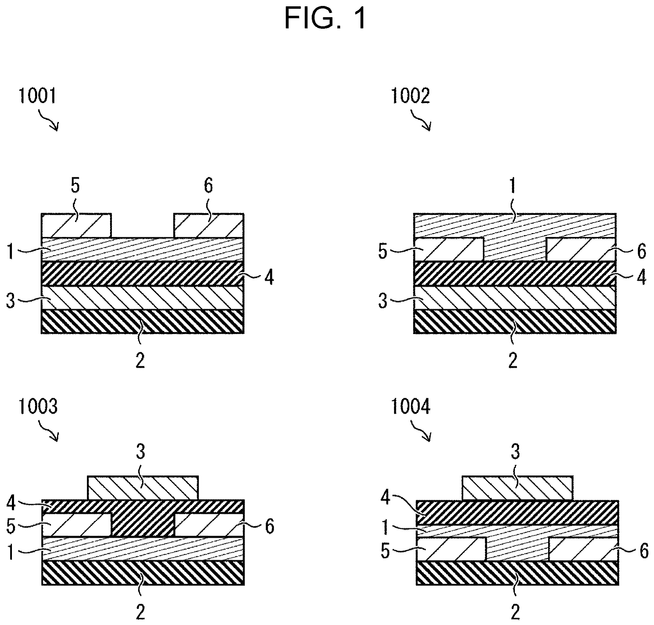

[Fig. 1] Diagrams illustrating structures of organic thin film transistors categorized by cross-sectional shape.

### Description of Embodiments

The present invention will now be described in detail.

### [1. Aromatic Compound]

The present invention is an aromatic compound indicated by either formula (1-I) or formula (1-II) above (Hereinafter referred to as "the compound of the present invention.).

Ar in formula (1-I) and formula (1-II) denotes a monocyclic ring or a fused ring system of two to six rings. Preferably, Ar is a fused ring system of two to four rings because in that case the compound of the present invention provides higher carrier mobility. More preferably, furthermore, Ar is a fused ring system of two or three rings because in that case the compound of the present invention exhibits higher solubility.

The monocyclic ring or each ring constituting the fused ring system of two to six rings is a four- to eight-membered ring. Preferably, it is a four- to six-membered ring because this facilitates π-stacking of the compound of the present invention.

Specific examples of monocyclic rings or fused ring systems of two to six rings denoted by Ar include monocyclic rings such as a cyclobutene ring, a thiophene ring, a furan ring, a selenophene ring, a thiazole ring, an oxazole ring, a pyrrole ring, an imidazole ring, a benzene ring and a pyridine ring and fused ring systems such as a thienothiophene ring system, a naphthalene ring system, a biphenylene ring system, an anthracene ring system, a dithienothiophene ring system, a dithienobenzo ring system, a benzothienobenzothiophene ring system, a tetracene ring system, a bis(dithieno)benzo ring system and a bis(benzothieno)benzo ring system. The fused ring systems of two to four rings, i.e., a thienothiophene ring system, a naphthalene ring system, a biphenylene ring system, an anthracene ring system, a dithienothiophene ring system and a benzothienobenzothiophene ring system, are preferred because they impart higher carrier mobility to the compound of the present invention. The fused ring systems of two or three rings, i.e., a thienothiophene ring system, a naphthalene ring system, a biphenylene ring system and an anthracene ring system, are more preferred.

X¹ and X² in formula (1-I) and formula (1-II) each independently denote one selected from the group consisting of an oxygen atom, a sulfur atom, a selenium atom, NR³ and CR⁴=CR⁵. Preferably, at least one of X¹ or X² is either a sulfur atom or CR⁴=CR⁵ because in that case the compound of the present invention exhibits higher stability. More preferably, both are sulfur atoms.

Y¹ and Y² in formula (1-I) and formula (1-II) each independently denote either CR⁶ or a nitrogen atom. Preferably, at least one of Y¹ or Y² is CR⁶ because in that case the compound of the present invention exhibits higher stability.

R¹ to R⁶ in formula (1-I) and formula (1-II) each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2), with at least one of R¹ to R⁶ being a group indicated by formula (2).

A halogen atom at the R¹ to R⁶ indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the R¹ to R⁶ include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C14 alkyl groups are particularly preferred because they make the compound of the present invention an aromatic compound that provides higher carrier mobility and also exhibits higher solubility. The C1 to C14 linear-chain alkyl groups, which are a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group and an n-tetradecyl group, are more preferred.

Examples of C2 to C20 alkenyl groups at the R¹ to R⁶ include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonenyl group, a 2-ethylheptenyl group, an n-decenyl group, an n-dodecenyl group, a cyclopentenyl-1- group, a cyclohexenyl-1- group and a cycloheptenyl-1- group.

Examples of C2 to C20 alkynyl groups at the R¹ to R⁶ include an ethynyl group, a propynyl group, a butynyl group, an n-pentynyl group, an n-hexynyl group, an n-heptynyl group, an n-octynyl group, an n-nonynyl group, an n-decynyl group and an n-dodecynyl group.

Examples of C4 to C22 alkadienyl groups at the R¹ to R⁶ include a butadienyl group, a pentadienyl group, a hexadienyl group, an n-heptadienyl group, an n-octadienyl group, an n-nonadienyl group, an n-decadienyl group, an n-dodecadienyl group and an n-tridecadienyl group. Preferably, such a group is a C4 to C22 alka-1,3-dienyl group. A hexa-1,3-dienyl group, an n-hepta-1,3-dienyl group, an n-octa-1,3-dienyl group, an n-nona-1,3-dienyl group and an n-deca-1,3-dienyl group are more preferred.

Examples of C4 to C22 alkadiynyl groups at the R¹ to R⁶ include a butadiynyl group, a pentadiynyl group, a hexadiynyl group, an n-heptadiynyl group, an n-octadiynyl group, an n-nonadiynyl group, an n-decadiynyl group, an n-dodecadiynyl group and an n-tridecadiynyl group. Preferably, such a group is a C4 to C22 1,3-alkadiynyl group. A hexa-1,3-diynyl group, an n-hepta-1,3-diynyl group, an n-octa-1,3-diynyl group, an n-nona-1,3-diynyl group and an n-deca-1,3-diynyl group are more preferred.

C4 to C26 aryl groups at the R¹ to R⁶ include C4 to C24 heteroaryl groups. Examples of such C4 to C26 aryl groups include a phenyl group; alkyl-substituted phenyl groups, such as a p-tolyl group, a p-(n-hexyl)phenyl group, a p-(n-octyl)phenyl group and a p-(2-ethylhexyl)phenyl group; a 2-furyl group and a 2-thienyl group; and alkyl-substituted heteroaryl groups, such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group and a 5-(2-ethylhexyl)-2-thienyl group. It should be noted that when an aryl group includes an alkyl substituent herein, a C4 to C26 aryl group means that the group is "C4 to C26" including the carbon atoms in the alkyl moiety.

k and m in formula (2) each independently denote 0 or 1. Preferably, k is 0 for the ease of synthesis of the compound of the present invention. More preferably, furthermore, k and m are both 0 because this makes the compound of the present invention one that provides higher carrier mobility.

n in formula (2) denotes 1 to 8. Preferably, n is from 3 to 6 for the stability of the compound of the present invention. More preferably, furthermore, n is 3 or 4 because this makes the compound of the present invention one that provides higher carrier mobility.

l in formula (2) denotes an integer of 1 to 20. Preferably, l is an integer of 1 to 3 because in that case the compound of the present invention exhibits higher solubility. More preferably, furthermore, l is an integer of 1 or 2 for higher heat resistance of the compound of the present invention. Moreover, even more preferably, l is 2 because this makes the compound of the present invention one that provides higher carrier mobility.

Z¹ to Z⁵ in formula (2) indicate one selected from the group consisting of a hydrogen atom, a halogen atom and a C1 to C20 alkyl group, being the same or different at each occurrence.

A halogen atom at the Z¹ to Z⁵ indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the Z¹ to Z⁵ include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C8 alkyl groups are particularly preferred because they make the compound of the present invention an aromatic compound that provides higher carrier mobility and also exhibits higher solubility. A methyl group, an ethyl group and an n-propyl group are more preferred.

Preferably, the Z¹ to Z⁵ are either hydrogen atoms or halogen atoms because this makes the compound of the present invention one that provides higher carrier mobility. More preferably, the Z¹ to Z⁵ are hydrogen atoms.

In formula (2), it is preferred that k and m be both 0, n be an integer of 3 or 4, l be an integer of 1 to 3, and Z¹ to Z⁵ be either hydrogen atoms or halogen atoms. This makes the compound of the present invention exhibit higher carrier mobility.

The group represented by formula (2) is preferably a cyclohexylmethyl group, a 2-cyclohexylethyl group, a 3-cyclohexylpropyl group, a cyclopentylmethyl group, a 2-cyclopentylethyl group or a 3-cyclopentylpropyl group because this makes the compound of the present invention one that provides higher carrier mobility. More preferably, the group is a 2-cyclohexylethyl group or a 2-cyclopentylethyl group because in that case the compound of the present invention achieves higher heat resistance and higher solubility.

Of these R¹ to R⁶, each of R¹ to R⁶ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, furthermore, each of R¹ to R⁶ is any of a hydrogen atom, a C1 to C20 alkyl group or a group represented by formula (2) because in that case the compound of the present invention exhibits higher solubility. Moreover, even more preferably, only one or only both of R¹ and R² denote groups represented by formula (2), and R³ to R⁶ are hydrogen atoms, because in that case the compound of the present invention provides higher carrier mobility.

The aromatic compound of formula (1-I) or formula (1-II) is preferably an aromatic compound indicated by one selected from the group consisting of formulae (3-1) to (3-6) below because of the ease of synthesis. 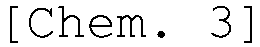 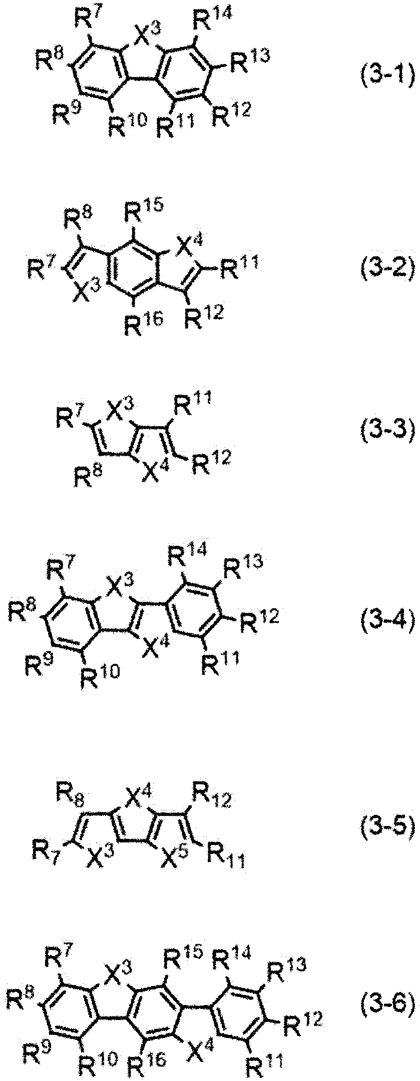 [(In the formulae, X³, X⁴ and X⁵ each independently denote one selected from the group consisting of an oxygen atom, a sulfur atom, a selenium atom, a single bond, NR¹⁷ and CR¹⁸=CR¹⁹. Of the combinations of adjacent two of R⁷ to R¹⁰, only one constitutes formula (4) below, and of the combinations of adjacent two of R¹¹ to R¹⁴, only one constitutes formula (4-2) below, each forming a five-membered ring or a six-membered ring. Those of R⁷ to R¹⁴ that do not constitute formula (4) below or formula (4-2) below, and R¹⁵ to R¹⁹, each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above.) 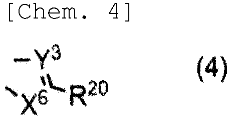 (In the formula, X⁶ denotes one selected from the group consisting of an oxygen atom, a sulfur atom, a selenium atom, CR²¹=CR²² and NR²³. Y³ denotes either CR²⁴ or a nitrogen atom. R²¹ to R²⁴ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above, and R²⁰ is a group represented by formula (2) above.) 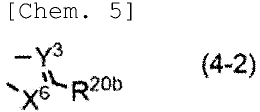 (In the formula, X⁶ and Y³ are synonymous with X⁶ and Y³ in formula (4) above, and R^{20b} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above.)]

In formulae (3-1) to (3-6), only one of the combinations of adjacent two of R⁷ to R¹⁰ constitutes formula (4) above, and only one of the combinations of adjacent two of R¹¹ to R¹⁴ constitutes formula (4-2) above, each forming a five-membered ring or a six-membered ring.

Those of R⁷ to R¹⁴ that do not constitute formula (4) or formula (4-2), R¹⁵ to R¹⁹ and R²¹ to R²⁴ in formula (4) and formula (4-2) each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group or a C4 to C26 aryl group and a group represented by formula (2).

R²⁰ is a group represented by formula (2).

R^{20b} is a group represented by one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2). Preferably, R^{20b} is one selected from the group consisting of a hydrogen atom, a fluorine atom and a group represented by formula (2) because this makes the compound of the present invention one that provides higher carrier mobility.

For the compound indicated by one selected from the group consisting of formulae (3-1) to (3-6), it is preferred that formula (4-2) be formula (4-3) below. 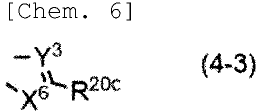 (In the formula, X⁶ and Y³ are synonymous with X⁶ and Y³ in formula (4) above, and R^{20c} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a group represented by a C4 to C26 aryl group.)

R^{20c} is one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a group represented by a C4 to C26 aryl group. Preferably, R^{20c} is a hydrogen atom or a fluorine atom because this makes the compound of the present invention one that provides higher carrier mobility. More preferably, R^{20c} is a hydrogen atom.

For the compound indicated by one selected from the group consisting of formulae (3-1) to (3-6), it is also preferred that formula (4-2) be formula (4-4) below. 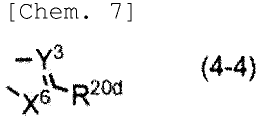 (In the formula, X⁶ and Y³ are synonymous with X⁶ and Y³ in formula (4) above, and R^{20d} is a group represented by formula (2) above.)

It should be noted that the definition of a group represented by formula (2) in formulae (3-1) to (3-6) is the same as the definition of formula (2) in formula (1-I) and formula (1-II), described earlier.

A halogen atom at the R⁷ to R¹⁹, R²¹ to R²⁴, R^{20b} and R^{20c} indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the R⁷ to R¹⁹, R²¹ to R²⁴, R^{20b} and R^{20c} include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C14 alkyl groups are particularly preferred because they make the compound of the present invention an aromatic compound that provides higher carrier mobility and also exhibits higher solubility. The C1 to C14 linear-chain alkyl groups, which are a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group and an n-tetradecyl group, are more preferred.

Examples of C2 to C20 alkenyl groups at the R⁷ to R¹⁹, R²¹ to R²⁴, R^{20b} and R^{20c} include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonenyl group, a 2-ethylheptenyl group, an n-decenyl group, an n-dodecenyl group, a cyclopentenyl-1- group, a cyclohexenyl-1- group and a cycloheptenyl-1- group.

Examples of C2 to C20 alkynyl groups at the R⁷ to R¹⁹, R²¹ to R²⁴, R^{20b} and R^{20c} include an ethynyl group, a propynyl group, a butynyl group, an n-pentynyl group, an n-hexynyl group, an n-heptynyl group, an n-octynyl group, an n-nonynyl group, an n-decynyl group and an n-dodecynyl group.

Examples of C4 to C22 alkadienyl groups at the R⁷ to R¹⁹, R²¹ to R²⁴, R^{20b} and R^{20c} include a butadienyl group, a pentadienyl group, a hexadienyl group, an n-heptadienyl group, an n-octadienyl group, an n-nonadienyl group, an n-decadienyl group, an n-dodecadienyl group and an n-tridecadienyl group. Preferably, such a group is a C4 to C22 alka-1,3-dienyl group. A hexa-1,3-dienyl group, an n-hepta-1,3-dienyl group, an n-octa-1,3-dienyl group, an n-nona-1,3-dienyl group and an n-deca-1,3-dienyl group are more preferred.

Examples of C4 to C22 alkadiynyl groups at the R⁷ to R¹⁹, R²¹ to R²⁴, R^{20b} and R^{20c} include a butadiynyl group, a pentadiynyl group, a hexadiynyl group, an n-heptadiynyl group, an n-octadiynyl group, an n-nonadiynyl group, an n-decadiynyl group, an n-dodecadiynyl group and an n-tridecadiynyl group. Preferably, such a group is a C4 to C22 1,3-alkadiynyl group. A hexa-1,3-diynyl group, an n-hepta-1,3-diynyl group, an n-octa-1,3-diynyl group, an n-nona-1,3-diynyl group and an n-deca-1,3-diynyl group are more preferred.

C4 to C26 aryl groups at the R⁷ to R¹⁹, R²¹ to R²⁴, R^{20b} and R^{20c} include C4 to C24 heteroaryl groups. Examples of such C4 to C26 aryl groups include a phenyl group; alkyl-substituted phenyl groups, such as a p-tolyl group, a p-(n-hexyl)phenyl group, a p-(n-octyl)phenyl group and a p-(2-ethylhexyl)phenyl group; a 2-furyl group and a 2-thienyl group; and alkyl-substituted heteroaryl groups, such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group and a 5-(2-ethylhexyl)-2-thienyl group.

Each of the R⁷ to R¹⁹ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, each of the R⁷ to R¹⁹ is a hydrogen atom or a C1 to C20 alkyl group because in that case the compound of the present invention exhibits higher solubility.

Each of the R²¹ to R²⁴ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, furthermore, each of R²¹ to R²⁴ is one selected from the group consisting of a hydrogen atom and a methyl group because this makes the compound of the present invention one that provides higher carrier mobility. Even more preferably, R²¹ to R²⁴ are hydrogen atoms.

X³, X⁴ and X⁵ in formulae (3-1) to (3-6) denote one selected from the group consisting of an oxygen atom, a sulfur atom, a selenium atom, a single bond, NR¹⁷ and CR¹⁸=CR¹⁹. Preferably, each of X³, X⁴ and X⁵ is any of a sulfur atom, a single bond or CR¹⁸=CR¹⁹ because this makes the compound of the present invention one that provides higher carrier mobility. More preferably, each of X³, X⁴ and X⁵ is a sulfur atom or CR¹⁸=CR¹⁹, even more preferably a sulfur atom.

X⁶ in formula (4), (4-2) and (4-3) denotes one selected from the group consisting of an oxygen atom, a sulfur atom, a selenium atom, CR²¹=CR²² and NR²³. Preferably, X⁶ is a sulfur atom or CR²¹=CR²² because this makes the compound of the present invention one that provides higher carrier mobility. More preferably, X⁶ is a sulfur atom.

Y³ in formulae (4), (4-2) and (4-3) denotes either CR²⁴ or a nitrogen atom. Preferably, Y³ is CR²⁴ for the stability of the compound of the present invention.

The aromatic compound indicated by formulae (3-1) to (3-6) is preferably in a structure having point symmetry or axial symmetry because this makes the compound of the present invention one that provides higher carrier mobility. More preferably, the compound has a structure with point symmetry.

The aromatic compound indicated by formulae (3-1) to (3-6) is preferably formula (3-1) or formula (3-2) because in that case the compound of the present invention achieves higher solubility and higher heat resistance.

The aromatic compound indicated by formula (1-I) or formula (1-II), furthermore, is preferably an aromatic compound indicated by formula (5) below or formula (5-2) below. 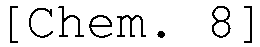 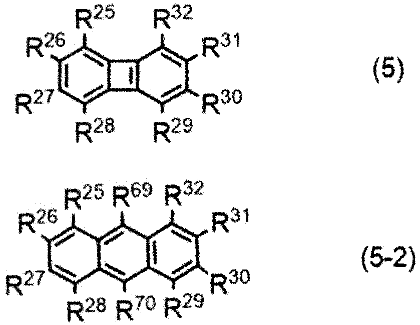 [(In the formula, only one of the combinations of adjacent two of R²⁵ to R²⁸ constitutes formula (6) below, and only one of the combinations of adjacent two of R²⁹ to R³² constitutes formula (6-2) below, each forming a five-membered ring or a six-membered ring. Those of R²⁵ to R³² that do not constitute formula (6) below or formula (6-2) below, and R⁶⁹ and R⁷⁰, each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above.) 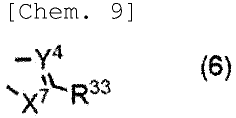 (In the formula, X⁷ denotes an oxygen atom, a sulfur atom, a selenium atom, CR³⁴=CR³⁵ or NR³⁶. Y⁴ denotes either CR³⁷ or a nitrogen atom. R³⁴ to R³⁷ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above, and R³³ is a group represented by formula (2) above.) 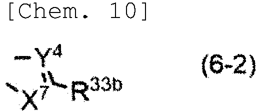 (In the formula, X⁷ and Y⁴ are synonymous with X⁷ and Y⁴ in formula (6) above, and R^{33b} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above.)]

In formulae (5) and (5-2), only one of the combinations of adjacent two of R²⁵ to R²⁸ constitutes formula (6) above, and only one of the combinations of adjacent two of R²⁹ to R³² constitutes formula (6-2) above, each forming a five-membered ring or a six-membered ring.

Those of R²⁵ to R³² that do not constitute formula (6) or formula (6-2), R⁶⁹ and R⁷⁰ and R³⁴ to R³⁷ in formula (6) and formula (6-2) each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group or a C4 to C26 aryl group and a group represented by formula (2).

R³³ is a group represented by formula (2).

R^{33b} is a group represented by one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2). Preferably, R^{33b} is one selected from the group consisting of a hydrogen atom, a fluorine atom and a group represented by formula (2) because this makes the compound of the present invention one that provides higher carrier mobility.

For the aromatic compound indicated by formula (5) or formula (5-2), it is preferred that formula (6-2) be formula (6-3) below. 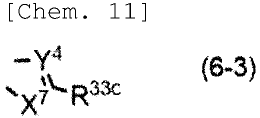 (In the formula, X⁷ and Y⁴ are synonymous with X⁷ and Y⁴ in formula (6) above, and R^{33c} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a C4 to C26 aryl group.)

R^{33c} is one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a group represented by a C4 to C26 aryl group. Preferably, R^{33c} is a hydrogen atom or a fluorine atom because this makes the compound of the present invention one that provides higher carrier mobility. More preferably, R^{33c} is a hydrogen atom.

For the aromatic compound indicated by formula (5) or formula (5-2), it is also preferred that formula (6-2) be formula (6-4) below. 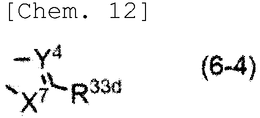 (In the formula, X⁷ and Y⁴ are synonymous with X⁷ and Y⁴ in formula (6) above, and R^{33d} is a group represented by formula (2) above.)

It should be noted that the definition of a group represented by formula (2) in formula (5) and formula (5-2) is the same as the definition of formula (2) in formula (1-I) and formula (1-II), described earlier.

A halogen atom at the R²⁵ to R³², R³⁴ to R³⁷, R⁶⁹, R⁷⁰, R^{33b} and R^{33c} indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the R²⁵ to R³², R³⁴ to R³⁷, R⁶⁹, R⁷⁰, R^{33b} and R^{33c} include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C14 alkyl groups are particularly preferred as such alkyl groups because they make the compound of the present invention an aromatic compound that provides higher carrier mobility and also exhibits higher solubility. The C1 to C14 linear-chain alkyl groups, which are a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group and an n-tetradecyl group, are more preferred.

Examples of C2 to C20 alkenyl groups at the R²⁵ to R³², R³⁴ to R³⁷, R⁶⁹, R⁷⁰, R^{33b} and R^{33c} include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonenyl group, a 2-ethylheptenyl group, an n-decenyl group, an n-dodecenyl group, a cyclopentenyl-1- group, a cyclohexenyl-1- group and a cycloheptenyl-1- group.

Examples of C2 to C20 alkynyl groups at the R²⁵ to R³², R³⁴ to R³⁷, R⁶⁹, R⁷⁰, R^{33b} and R^{33c} include an ethynyl group, a propynyl group, a butynyl group, an n-pentynyl group, an n-hexynyl group, an n-heptynyl group, an n-octynyl group, an n-nonynyl group, an n-decynyl group and an n-dodecynyl group.

Examples of C4 to C22 alkadienyl groups at the R²⁵ to R³², R³⁴ to R³⁷, R⁶⁹, R⁷⁰, R^{33b} and R^{33c} include a butadienyl group, a pentadienyl group, a hexadienyl group, an n-heptadienyl group, an n-octadienyl group, an n-nonadienyl group, an n-decadienyl group, an n-dodecadienyl group and an n-tridecadienyl group. Preferably, such a group is a C4 to C22 alka-1,3-dienyl group. A hexa-1,3-dienyl group, an n-hepta-1,3-dienyl group, an n-octa-1,3-dienyl group, an n-nona-1,3-dienyl group and an n-deca-1,3-dienyl group are more preferred.

Examples of C4 to C22 alkadiynyl groups at the R²⁵ to R³², R³⁴ to R³⁷, R⁶⁹, R⁷⁰, R^{33b} and R^{33c} include a butadiynyl group, a pentadiynyl group, a hexadiynyl group, an n-heptadiynyl group, an n-octadiynyl group, an n-nonadiynyl group, an n-decadiynyl group, an n-dodecadiynyl group and an n-tridecadiynyl group. Preferably, such a group is a C4 to C22 1,3-alkadiynyl group. A hexa-1,3-diynyl group, an n-hepta-1,3-diynyl group, an n-octa-1,3-diynyl group, an n-nona-1,3-diynyl group and an n-deca-1,3-diynyl group are more preferred.

C4 to C26 aryl groups at the R²⁵ to R³², R³⁴ to R³⁷, R⁶⁹, R⁷⁰, R^{33b} and R^{33c} include C4 to C24 heteroaryl groups. Examples of such C4 to C26 aryl groups include a phenyl group; alkyl-substituted phenyl groups, such as a p-tolyl group, a p-(n-hexyl)phenyl group, a p-(n-octyl)phenyl group and a p-(2-ethylhexyl)phenyl group; a 2-furyl group and a 2-thienyl group; and alkyl-substituted heteroaryl groups, such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group and a 5-(2-ethylhexyl)-2-thienyl group.

Each of the R²⁵ to R³², R⁶⁹ and R⁷⁰ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, each of the R²⁵ to R³², R⁶⁹ and R⁷⁰ is a hydrogen atom or a C1 to C20 alkyl group because in that case the compound of the present invention achieves higher solubility. Even more preferably, the R²⁵ to R³², R⁶⁹ and R⁷⁰ are selected from one in the group consisting of a hydrogen atom and a methyl group.

Each of the R³⁴ to R³⁷ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, each of the R³⁴ to R³⁷ is a hydrogen atom, a C1 to C20 alkyl group or a group represented by formula (2) because this makes the compound of the present invention one that provides higher carrier mobility. Even more preferably, R³⁴ to R³⁷ are hydrogen atoms.

X⁷ in formulae (6) and (6-2) to (6-4) denotes an oxygen atom, a sulfur atom, a selenium atom, CR³⁴=CR³⁵ or NR³⁶. Preferably, X⁷ is a sulfur atom, an oxygen atom, a sulfur atom or a selenium atom because in that case the compound of the present invention exhibits higher solubility. More preferably, X⁷ is a sulfur atom because this makes the compound of the present invention one that provides higher carrier mobility.

Y⁴ in formulae (6) and (6-2) to (6-4) denotes CR³⁷ or a nitrogen atom. Preferably, Y⁴ is CR³⁷ for the stability of the compound of the present invention.

The aromatic compound indicated by formula (5) and formula (5-2) is preferably in a structure having point symmetry or axial symmetry because this makes the compound of the present invention one that provides higher carrier mobility. More preferably, the compound has a structure with point symmetry.

The aromatic compound indicated by formula (5) and formula (5-2) is preferably an aromatic compound indicated by formula (5) because in that case the compound of the present invention exhibits higher solubility.

The aromatic compound indicated by formula (5) or formula (5-2), furthermore, is preferably one compound selected from the group consisting of formulae (7-1) to (7-6) below because of the ease of synthesis. Particularly preferably, the compound is a compound indicated by (7-5). 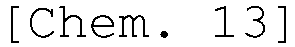 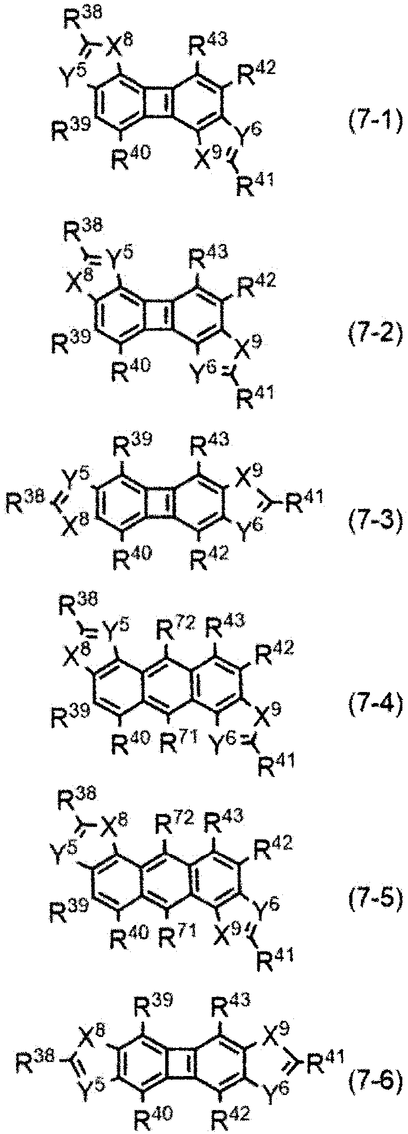 (In the formulae, X⁸ and X⁹ each independently denote one selected from the group consisting of an oxygen atom, a sulfur atom, a selenium atom and NR⁴⁴. Y⁵ and Y⁶ each independently denote either CR⁴⁵ or a nitrogen atom. R³⁸ to R⁴⁵, R⁷¹ and R⁷² each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2), with at least one of R³⁸ or R⁴¹ being a group represented by formula (2).)

X⁸ and X⁹ in formulae (7-1) to (7-6) each independently denote one selected from the group consisting of an oxygen atom, a sulfur atom, a selenium atom and NR⁴⁴. Preferably, each of X⁸ and X⁹ is a sulfur atom, an oxygen atom, a sulfur atom or a selenium atom for the stability of the compound of the present invention. More preferably, furthermore, X⁸ and X⁹ are sulfur atoms because this makes the compound of the present invention one that provides higher carrier mobility.

Y⁵ and Y⁶ in formulae (7-1) to (7-6) each independently denote either CR⁴⁵ or a nitrogen atom. Preferably, Y⁵ and Y⁶ are CR⁴⁵ for the stability of the compound of the present invention.

R³⁸ to R⁴⁵, R⁷¹ and R⁷² in formulae (7-1) to (7-6) each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2) above, with at least one of R³⁸ to R⁴⁵ being a group represented by formula (2) above.

For R³⁸ and R⁴¹ in formulae (7-1) to (7-6), only either one or both are groups represented by formula (2).

It should be noted that the definition of a group represented by formula (2) in formulae (7-1) to (7-6) is the same as the definition of formula (2) in formula (1-I) and formula (1-II), described earlier.

A halogen atom at the R³⁸ to R⁴⁵, R⁷¹ and R⁷² indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the R³⁸ to R⁴⁵, R⁷¹ and R⁷² include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C14 alkyl groups are particularly preferred as such alkyl groups because they make the compound of the present invention an aromatic compound that provides higher carrier mobility and also exhibits higher solubility. The C1 to C14 linear-chain alkyl groups, which are a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group and an n-tetradecyl group, are more preferred.

Examples of C2 to C20 alkenyl groups at the R³⁸ to R⁴⁵, R⁷¹ and R⁷² include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonenyl group, a 2-ethylheptenyl group, an n-decenyl group, an n-dodecenyl group, a cyclopentenyl-1- group, a cyclohexenyl-1- group and a cycloheptenyl-1- group.

Examples of C2 to C20 alkynyl groups at the R³⁸ to R⁴⁵, R⁷¹ and R⁷² include an ethynyl group, a propynyl group, a butynyl group, an n-pentynyl group, an n-hexynyl group, an n-heptynyl group, an n-octynyl group, an n-nonynyl group, an n-decynyl group and an n-dodecynyl group.

Examples of C4 to C22 alkadienyl groups at the R³⁸ to R⁴⁵, R⁷¹ and R⁷² include a butadienyl group, a pentadienyl group, a hexadienyl group, an n-heptadienyl group, an n-octadienyl group, an n-nonadienyl group, an n-decadienyl group, an n-dodecadienyl group and an n-tridecadienyl group. Preferably, such a group is a C4 to C22 alka-1,3-dienyl group. A hexa-1,3-dienyl group, an n-hepta-1,3-dienyl group, an n-octa-1,3-dienyl group, an n-nona-1,3-dienyl group and an n-deca-1,3-dienyl group are more preferred.

Examples of C4 to C22 alkadiynyl groups at the R³⁸ to R⁴⁵, R⁷¹ and R⁷² include a butadiynyl group, a pentadiynyl group, a hexadiynyl group, an n-heptadiynyl group, an n-octadiynyl group, an n-nonadiynyl group, an n-decadiynyl group, an n-dodecadiynyl group and an n-tridecadiynyl group. Preferably, such a group is a C4 to C22 1,3-alkadiynyl group. A hexa-1,3-diynyl group, an n-hepta-1,3-diynyl group, an n-octa-1,3-diynyl group, an n-nona-1,3-diynyl group and an n-deca-1,3-diynyl group are more preferred.

C4 to C26 aryl groups at the R³⁸ to R⁴⁵, R⁷¹ and R⁷² include C4 to C24 heteroaryl groups. Examples of such C4 to C26 aryl groups include a phenyl group; alkyl-substituted phenyl groups, such as a p-tolyl group, a p-(n-hexyl)phenyl group, a p-(n-octyl)phenyl group and a p-(2-ethylhexyl)phenyl group; a 2-furyl group and a 2-thienyl group; and alkyl-substituted heteroaryl groups, such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group and a 5-(2-ethylhexyl)-2-thienyl group.

Each of the R³⁸ to R⁴⁵, R⁷¹ and R⁷² is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. More preferably, furthermore, each of the R³⁸ to R⁴⁵, R⁷¹ and R⁷² is a hydrogen atom, a C1 to C20 alkyl group or a group represented by formula (2) because in that case the compound of the present invention exhibits higher solubility. Preferably, each of R³⁸ and R⁴¹ is one selected from the group consisting of a group represented by formula (2) above, a hydrogen atom and a fluorine atom for higher carrier mobility of the compound of the present invention. More preferably, each of R³⁸ and R⁴¹ is one selected from the group consisting of a group represented by formula (2) above and a hydrogen atom, and even more preferably both of R³⁸ and R⁴¹ are groups represented by formula (2) above. Still more preferably, R³⁹, R⁴⁰, R⁴² to R⁴⁵, R⁷¹ and R⁷² are selected from one in the group consisting of a hydrogen atom and a methyl group, and further preferably are hydrogen atoms.

Of formulae (7-1) to (7-6), the formula is preferably any of formula (7-1), (7-2) or (7-5) because in that case the compound of the present invention exhibits higher solubility. More preferably, furthermore, the formula is formula (7-5) because this makes the compound of the present invention one that provides higher carrier mobility.

A more preferred compound structure of the compound of the present invention is indicated by one selected from the group consisting of formulae (8-1) to (8-11) below in addition to formulae (7-1) to (7-6) above. Of these, the compound is preferably one aromatic compound selected from the group consisting of (7-1) to (7-6) and (8-2) to (8-9), in which the number of fused rings is four or five, for higher heat resistance and higher solubility of the compound of the present invention. More preferably, furthermore, the compound is one aromatic compound selected from the group consisting of formulae (7-1) to (7-6), (8-2), (8-3) and (8-5) to (8-8), which are in a structure with point symmetry, because in that case the compound of the present invention provides higher carrier mobility. 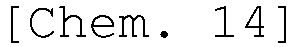 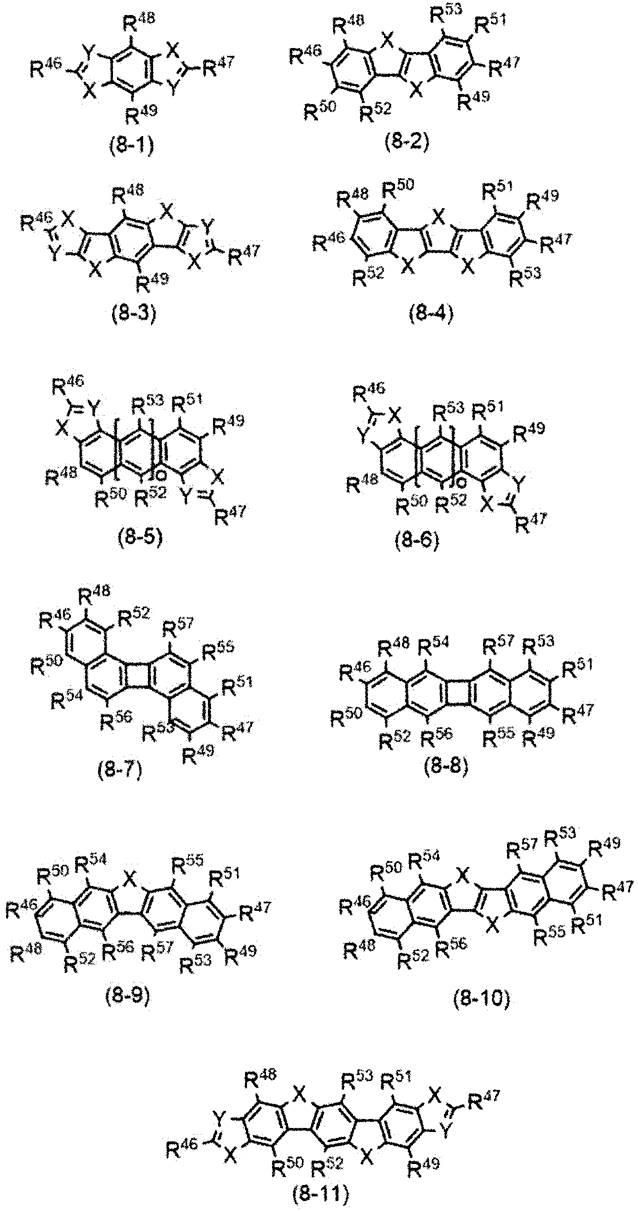 (In the formulae, X denotes one selected from the group consisting of an oxygen atom, a sulfur atom, a selenium atom, NR⁵⁸ and CR⁵⁹=CR⁶⁰. Y denotes either CR⁶¹ or a nitrogen atom. R⁴⁶ to R⁶¹ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2), with at least one of R⁴⁶ to R⁶¹ being a group represented by formula (2), and at least one of R⁴⁶ or R⁴⁷ being a group represented by formula (2). o denotes 0 or 1.)

X in formulae (8-1) to (8-11) denotes one selected from the group consisting of an oxygen atom, a sulfur atom, a selenium atom, NR⁵⁸ and CR⁵⁹=CR⁶⁰. Preferably, X is one in the group consisting of an oxygen atom, a sulfur atom and a selenium atom for the stability of the compound of the present invention. More preferably, furthermore, X is a sulfur atom because this makes the compound of the present invention one that provides higher carrier mobility.

Y in formulae (8-1) to (8-11) denotes either CR⁶¹ or a nitrogen atom. Preferably, Y is CR⁶¹ for the stability of the compound of the present invention.

R⁴⁶ to R⁶¹ in formulae (8-1) to (8-11) each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by formula (2), with at least one of R⁴⁶ to R⁶¹ being a group represented by formula (2).

It should be noted that the definition of a group represented by formula (2) in formulae (8-1) to (8-11) is the same as the definition in formula (1-I) and formula (1-II), described earlier.

A halogen atom at the R⁴⁶ to R⁶¹ indicates, for example, a fluorine atom, a chlorine atom, a bromine atom or an iodine atom. Preferably, it is either a fluorine atom or a chlorine atom because in that case the compound of the present invention is stable.

Examples of C1 to C20 alkyl groups at the R⁴⁶ to R⁶¹ include linear-chain, branched or cyclic alkyl groups, such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl, a 2-hexyldecyl group, a cyclopentyl group, a cyclohexyl group and a cycloheptyl group. Of these, furthermore, C1 to C14 alkyl groups are particularly preferred as such alkyl groups because they make the compound of the present invention an aromatic compound that provides higher carrier mobility and also exhibits higher solubility. The C1 to C14 linear-chain alkyl groups, which are a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group and an n-tetradecyl group, are more preferred.

Examples of C2 to C20 alkenyl groups at the R⁴⁶ to R⁶¹ include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonenyl group, a 2-ethylheptenyl group, an n-decenyl group, an n-dodecenyl group, a cyclopentenyl-1- group, a cyclohexenyl-1- group and a cycloheptenyl-1- group.

Examples of C2 to C20 alkynyl groups at the R⁴⁶ to R⁶¹ include an ethynyl group, a propynyl group, a butynyl group, an n-pentynyl group, an n-hexynyl group, an n-heptynyl group, an n-octynyl group, an n-nonynyl group, an n-decynyl group and an n-dodecynyl group.

Examples of C4 to C22 alkadienyl groups at the R⁴⁶ to R⁶¹ include a butadienyl group, a pentadienyl group, a hexadienyl group, an n-heptadienyl group, an n-octadienyl group, an n-nonadienyl group, an n-decadienyl group, an n-dodecadienyl group and an n-tridecadienyl group. Preferably, such a group is a C4 to C22 alka-1,3-dienyl group. A hexa-1,3-dienyl group, an n-hepta-1,3-dienyl group, an n-octa-1,3-dienyl group, an n-nona-1,3-dienyl group and an n-deca-1,3-dienyl group are more preferred.

Examples of C4 to C22 alkadiynyl groups at the R⁴⁶ to R⁶¹ include a butadiynyl group, a pentadiynyl group, a hexadiynyl group, an n-heptadiynyl group, an n-octadiynyl group, an n-nonadiynyl group, an n-decadiynyl group, an n-dodecadiynyl group and an n-tridecadiynyl group. Preferably, such a group is a C4 to C22 1,3-alkadiynyl group. A hexa-1,3-diynyl group, an n-hepta-1,3-diynyl group, an n-octa-1,3-diynyl group, an n-nona-1,3-diynyl group and an n-deca-1,3-diynyl group are more preferred.

C4 to C26 aryl groups at the R⁴⁶ to R⁶¹ include C4 to C24 heteroaryl groups. Examples of such C4 to C26 aryl groups include a phenyl group; alkyl-substituted phenyl groups, such as a p-tolyl group, a p-(n-hexyl)phenyl group, a p-(n-octyl)phenyl group and a p-(2-ethylhexyl)phenyl group; a 2-furyl group and a 2-thienyl group; and alkyl-substituted heteroaryl groups, such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group and a 5-(2-ethylhexyl)-2-thienyl group.

Each of the R⁴⁶ to R⁶¹ is preferably one selected from the group consisting of a hydrogen atom, a halogen atom, a C1 to C20 alkyl group, a C2 to C20 alkenyl group, a C2 to C20 alkynyl group, a C4 to C26 aryl group and a group represented by formula (2) for the stability of the compound of the present invention. Preferably, each of R⁴⁶ and R⁴⁷ is one selected from the group consisting of a group represented by formula (2), a hydrogen atom and a fluorine atom because in that case the compound of the present invention provides higher carrier mobility. More preferably, each of R⁴⁶ and R⁴⁷ is one selected from the group consisting of a group represented by formula (2) and a hydrogen atom, and even more preferably both of R⁴⁶ and R⁴⁷ are groups represented by formula (2). Preferably, R⁴⁸ to R⁶¹ are selected from one selected from the group consisting of a hydrogen atom and a methyl group, and more preferably are hydrogen atoms.

o in formulae (8-5) and (8-6) denotes 0 or 1. Preferably, o is 1 because this makes the compound of the present invention one that provides higher carrier mobility.

With the compound of the present invention, an aromatic compound that provides high carrier mobility and also has high heat resistance and high solubility can be provided. Its advantages are enormous. It should be noted that as used herein, "high carrier mobility" is intended to mean that the carrier mobility is 0.10 cm²/V·sec or more. Preferably, the carrier mobility is 0.15 cm²/V·sec or more, more preferably 0.20 cm²/V·sec or more. As used herein, furthermore, "high solubility" is intended to mean that the solubility in a solvent (e.g., toluene or n-octane) at room temperature is 0.1% by weight or more. Moreover, "high heat resistance" as used herein is intended to mean that the melting point is 130°C or above.

Specific examples of compounds according to the present invention include the following.

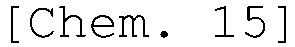

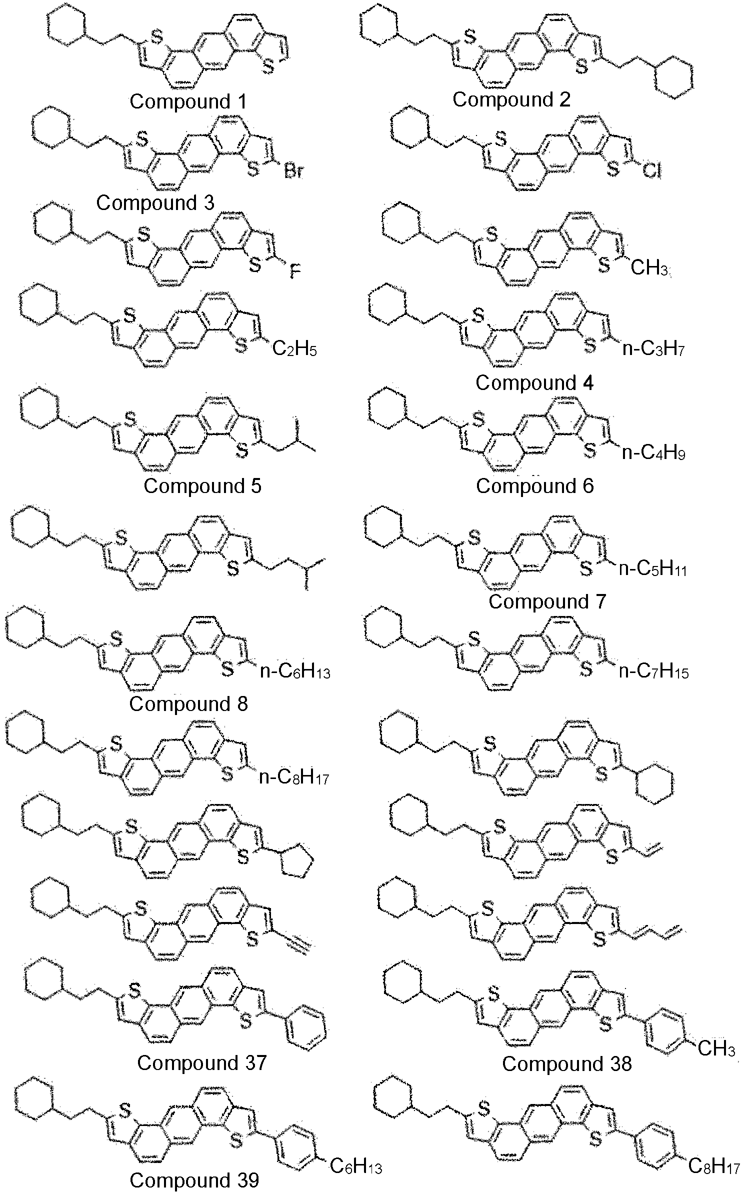

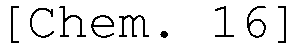

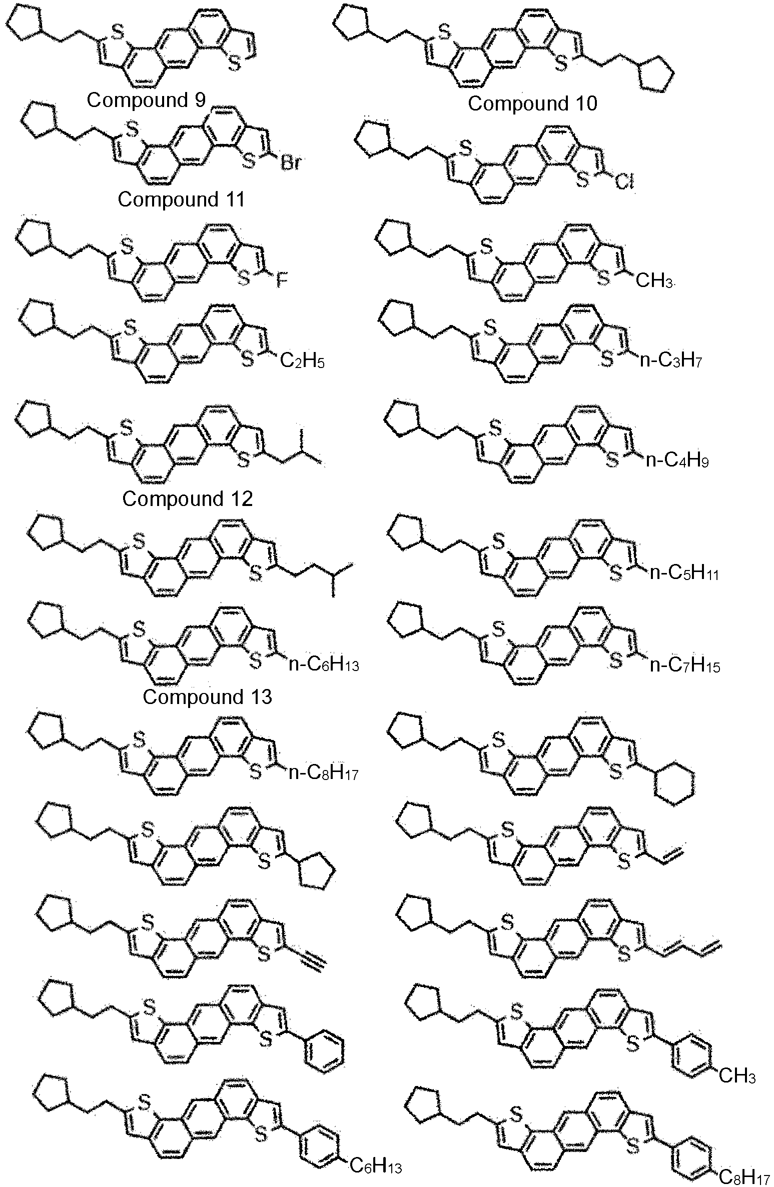

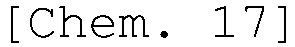

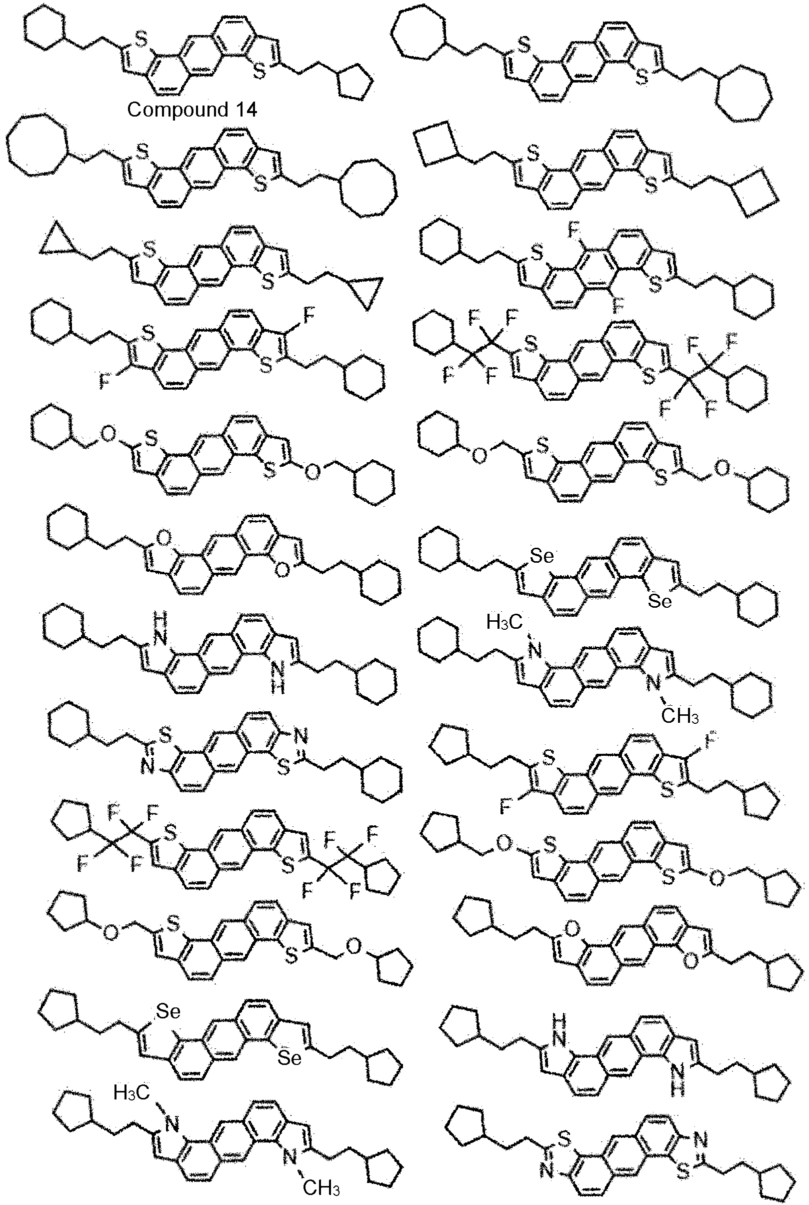

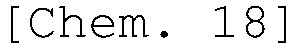

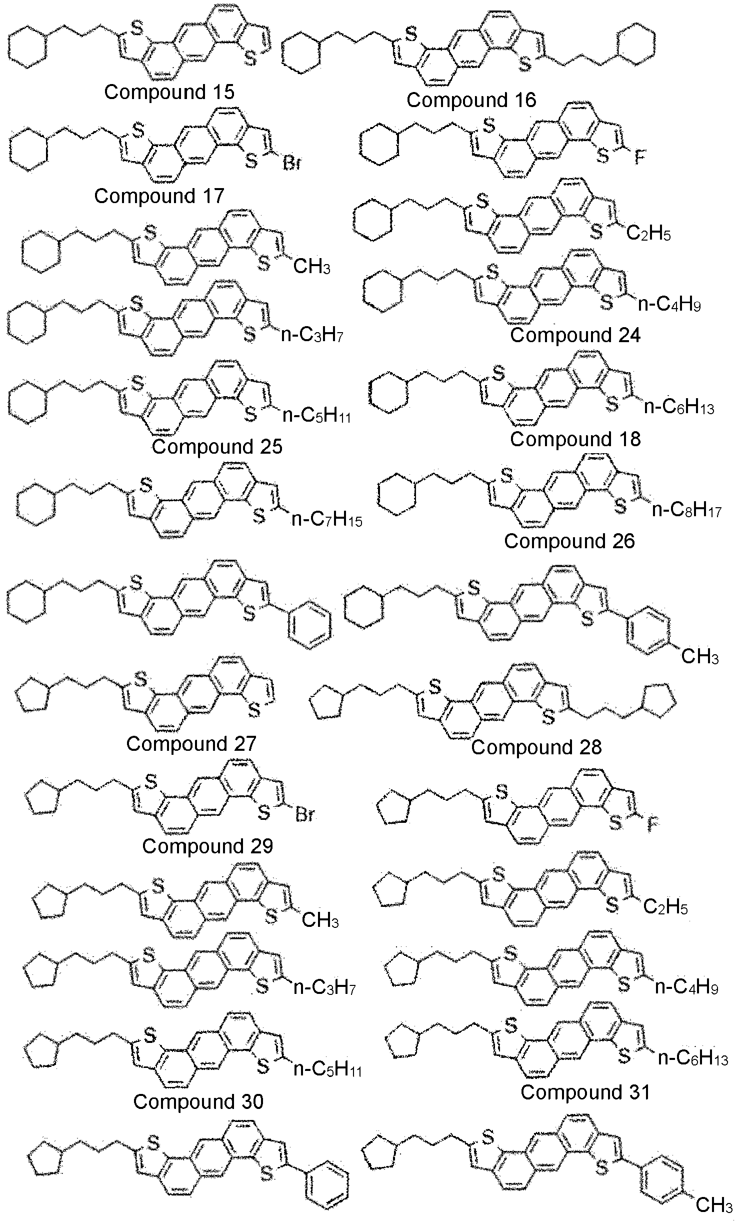

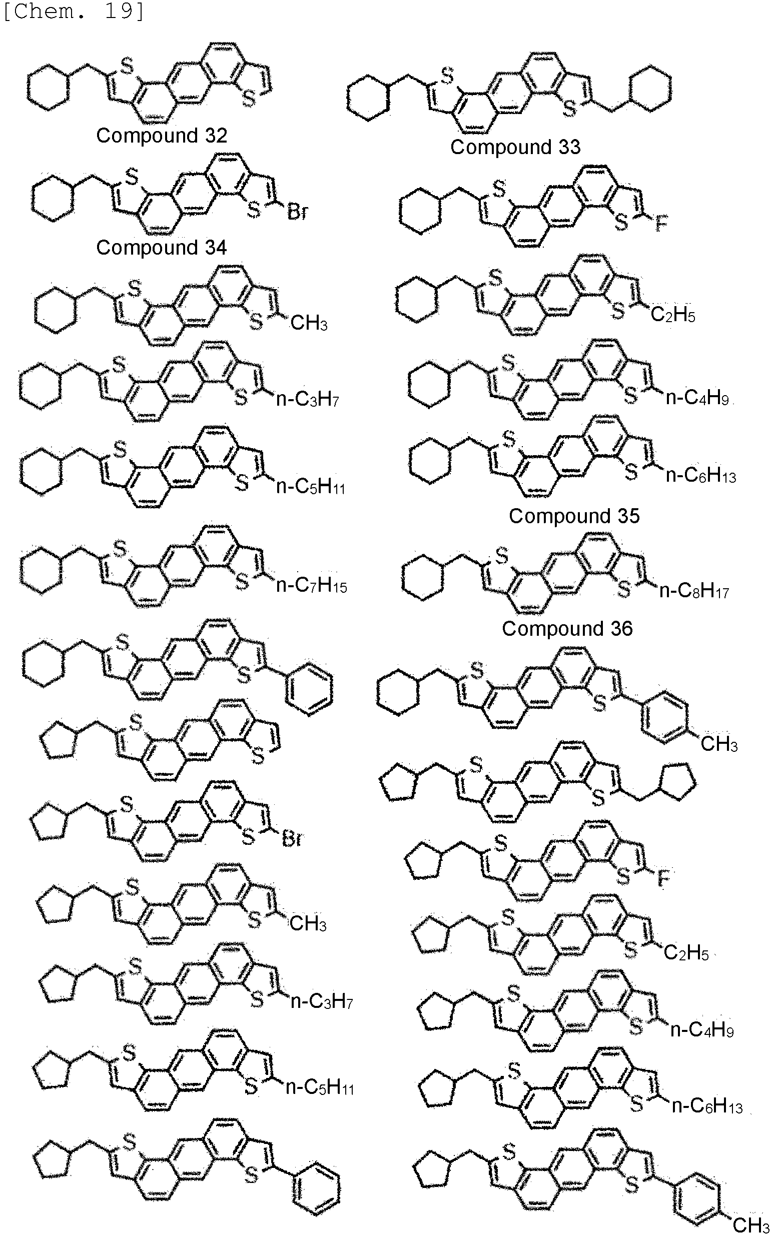

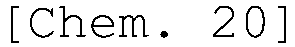

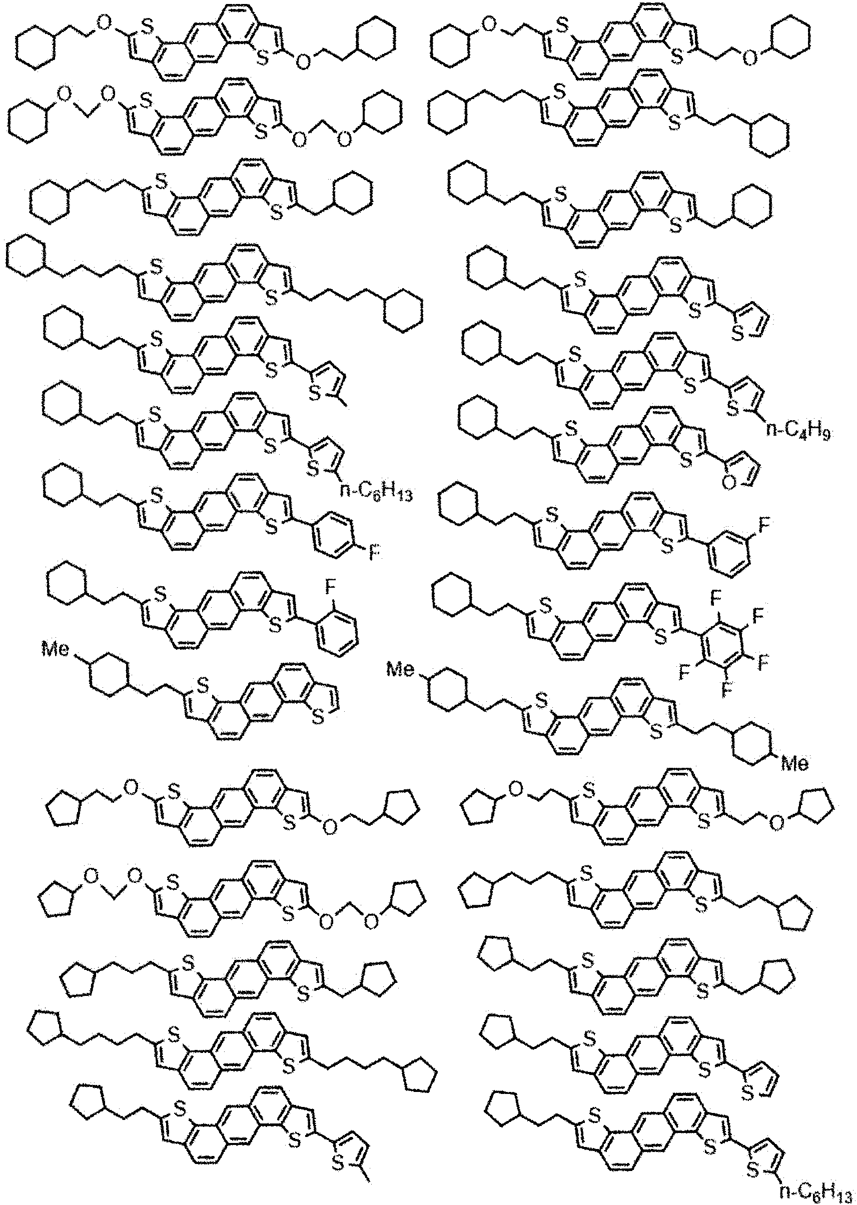

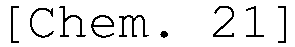

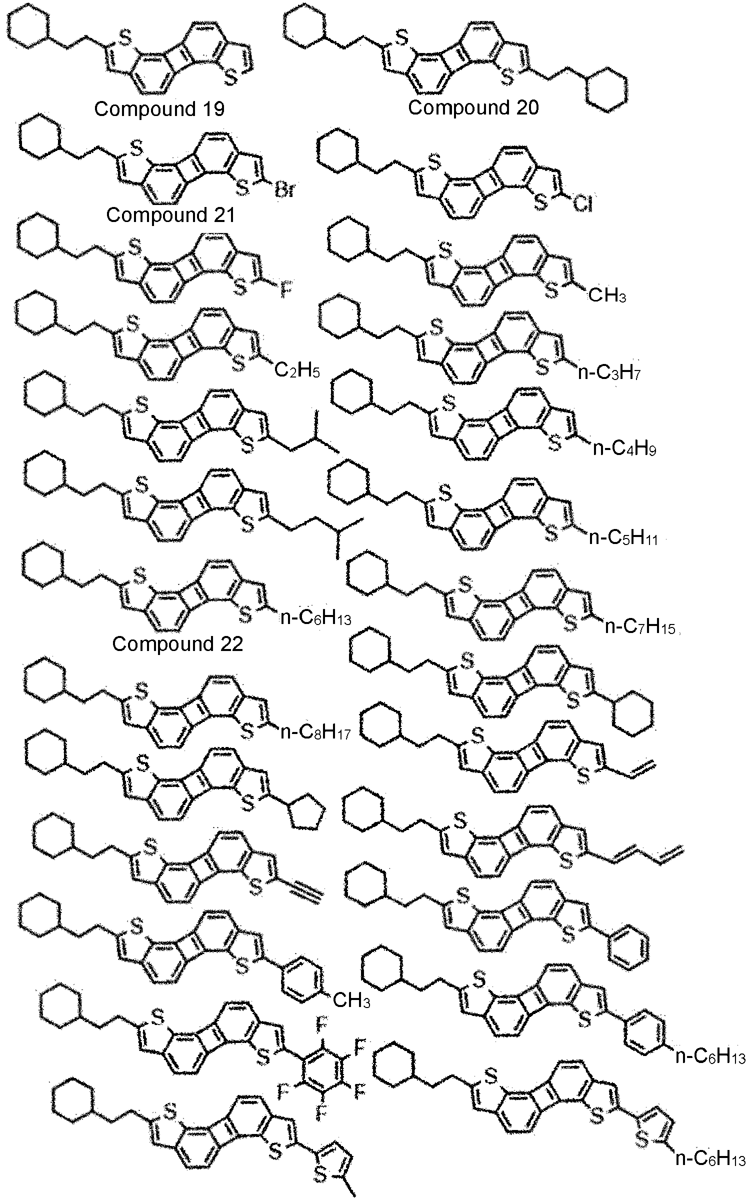

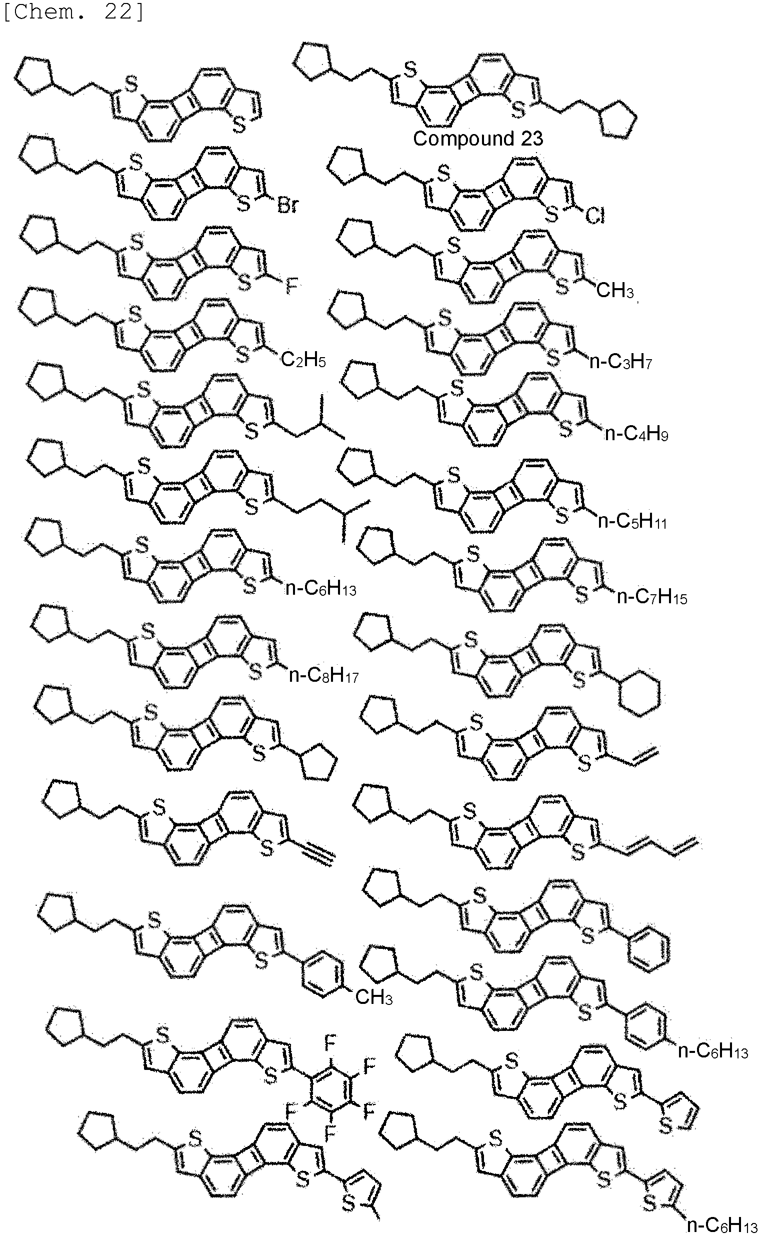

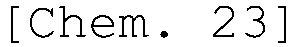

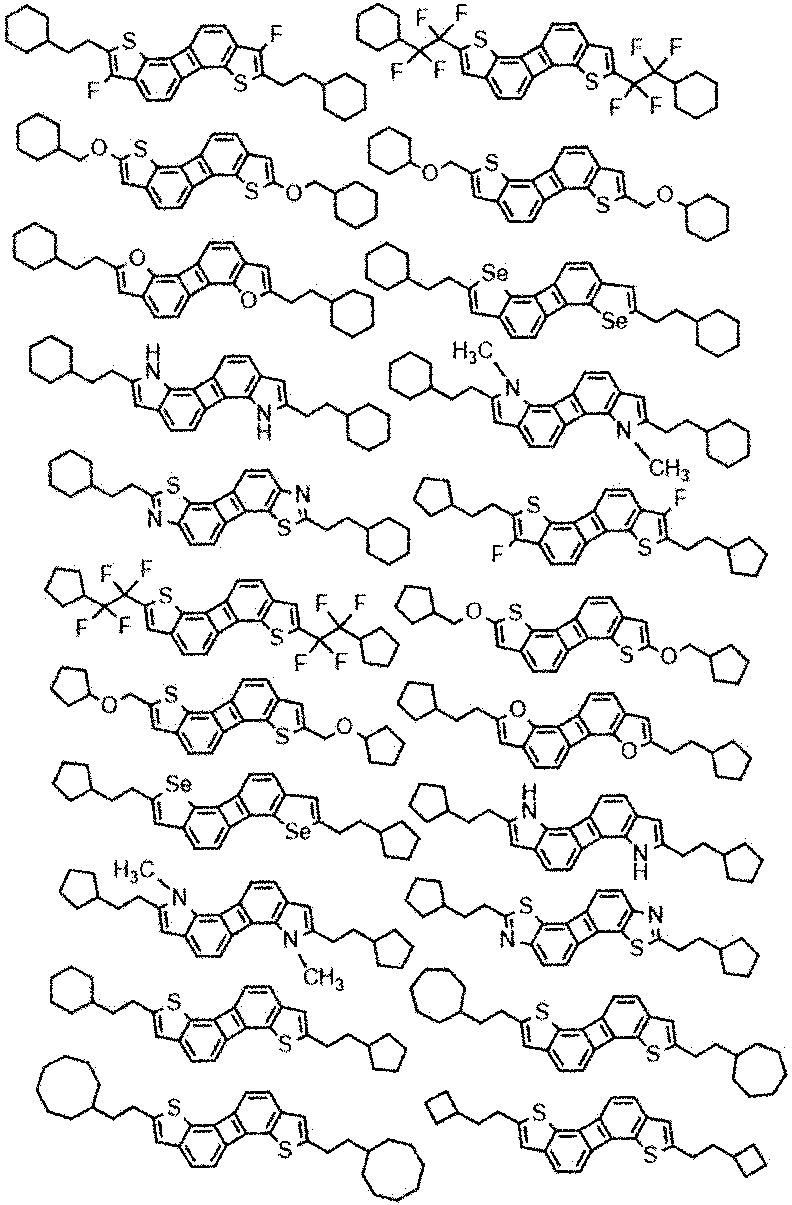

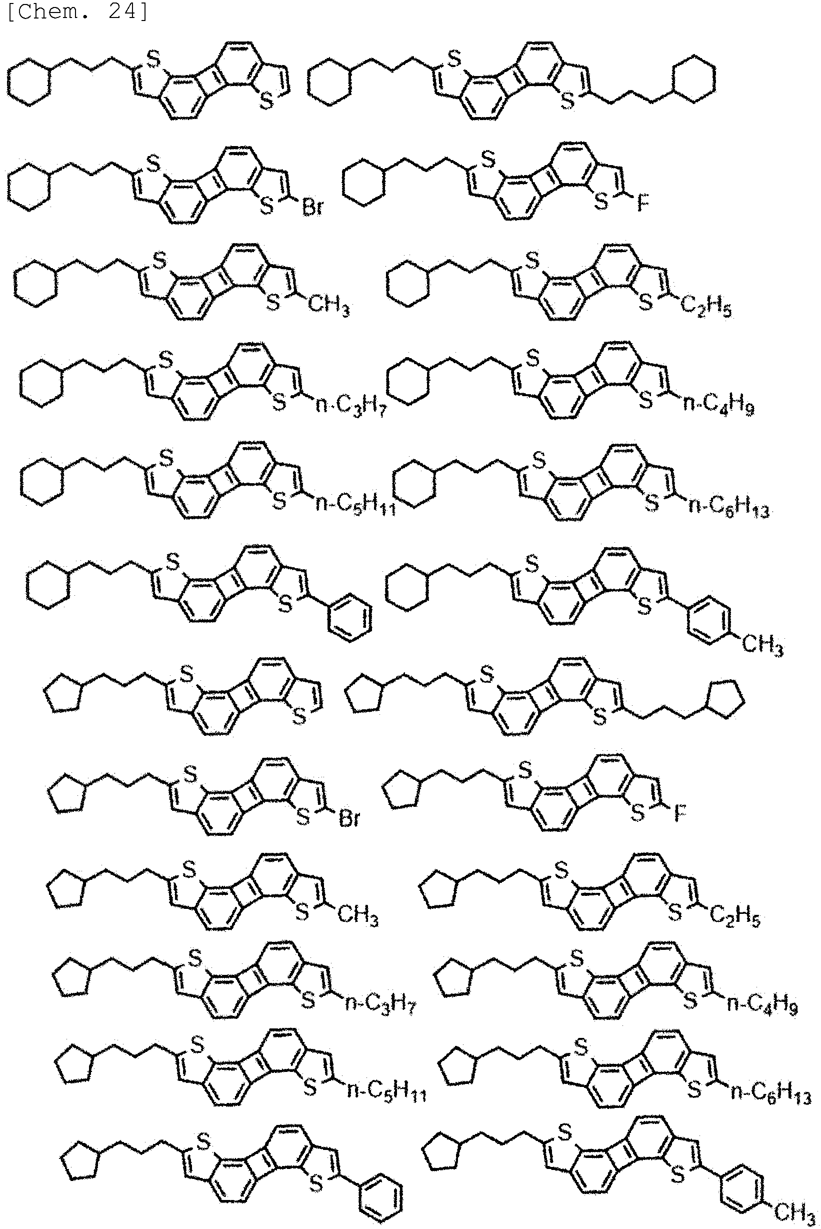

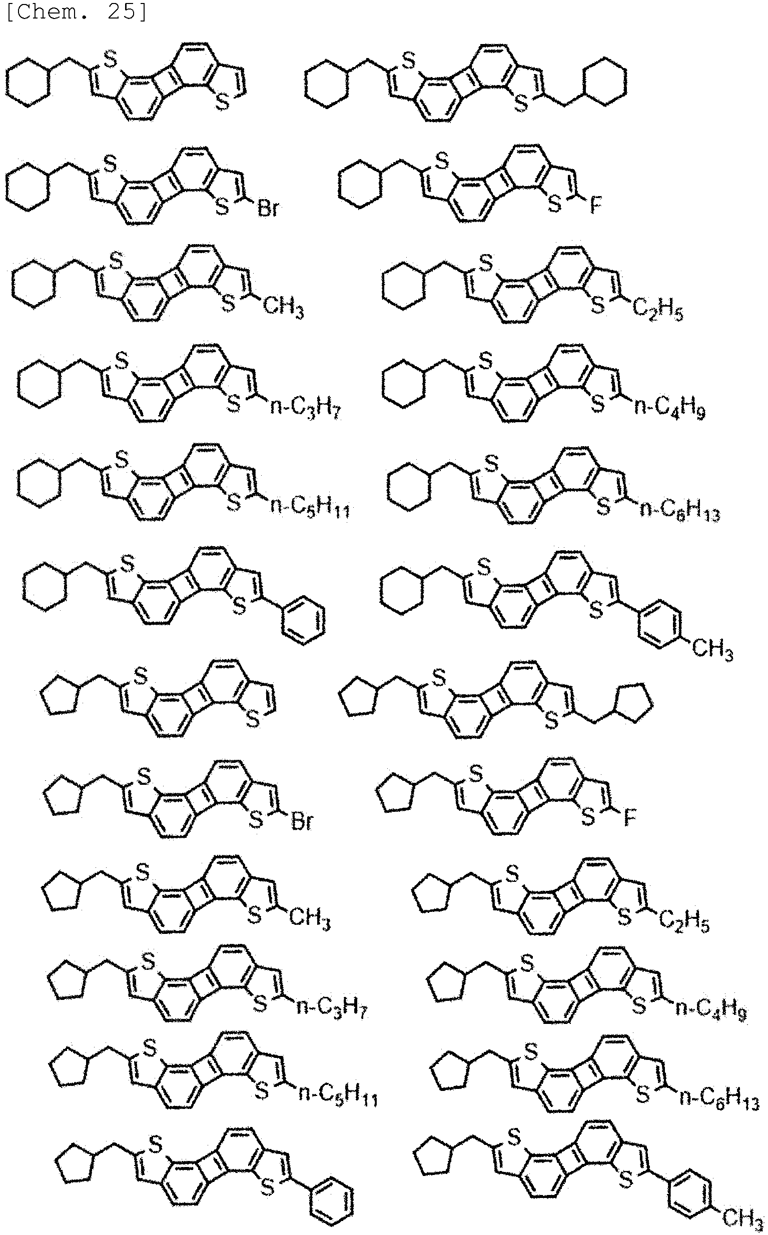

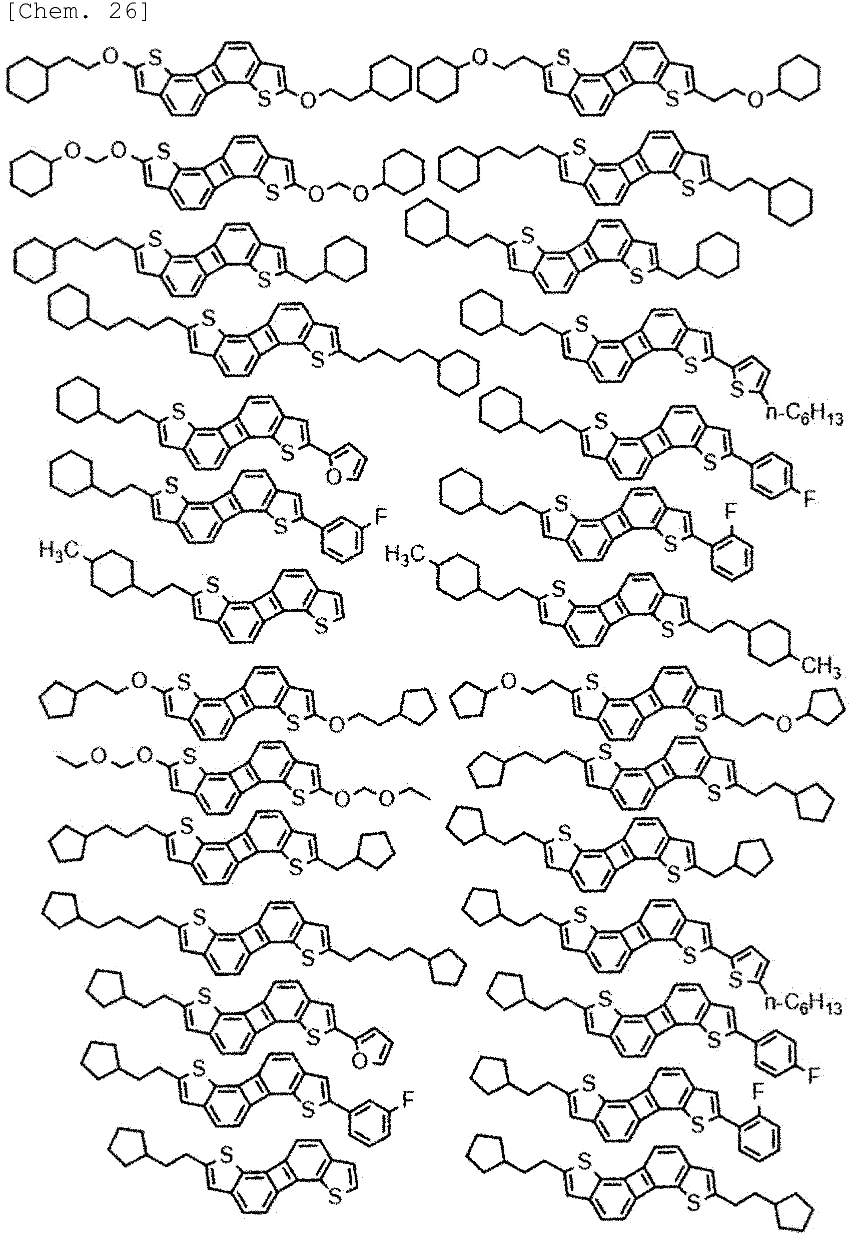

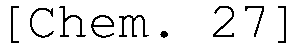

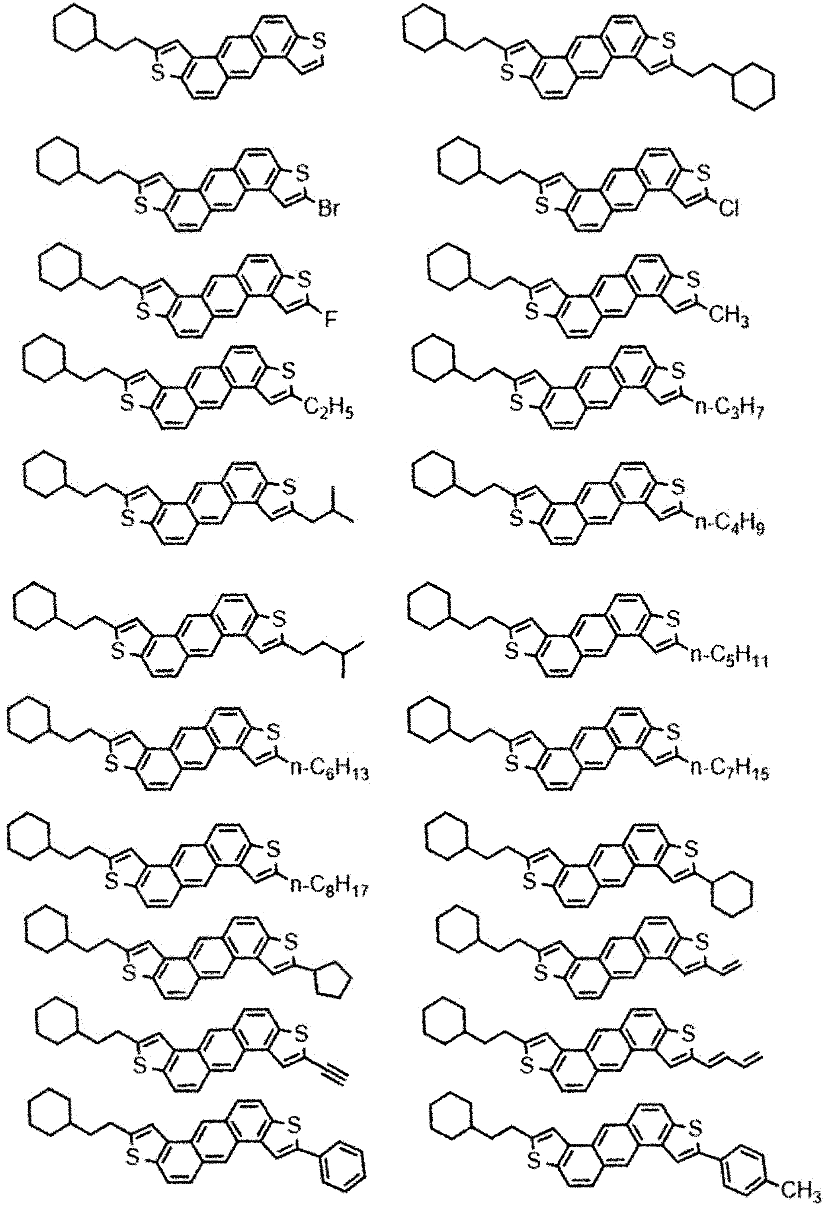

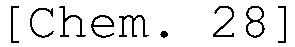

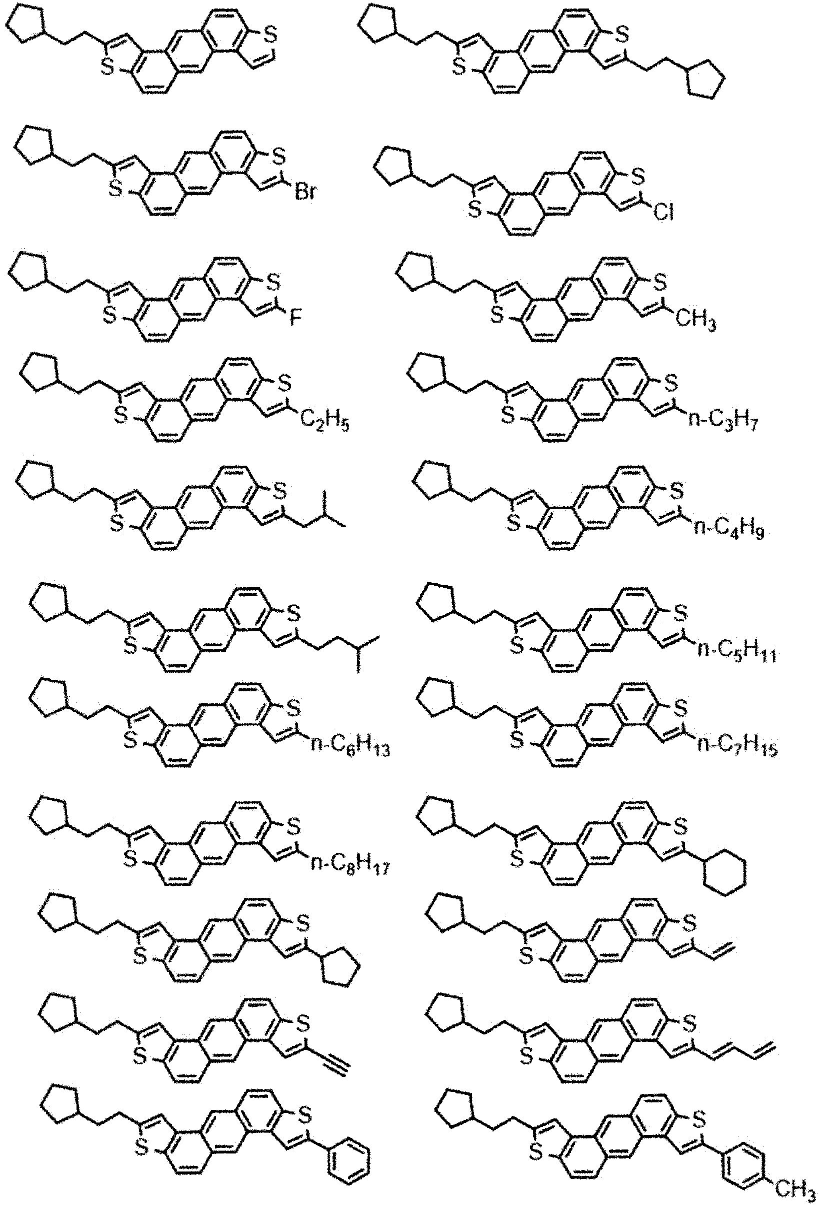

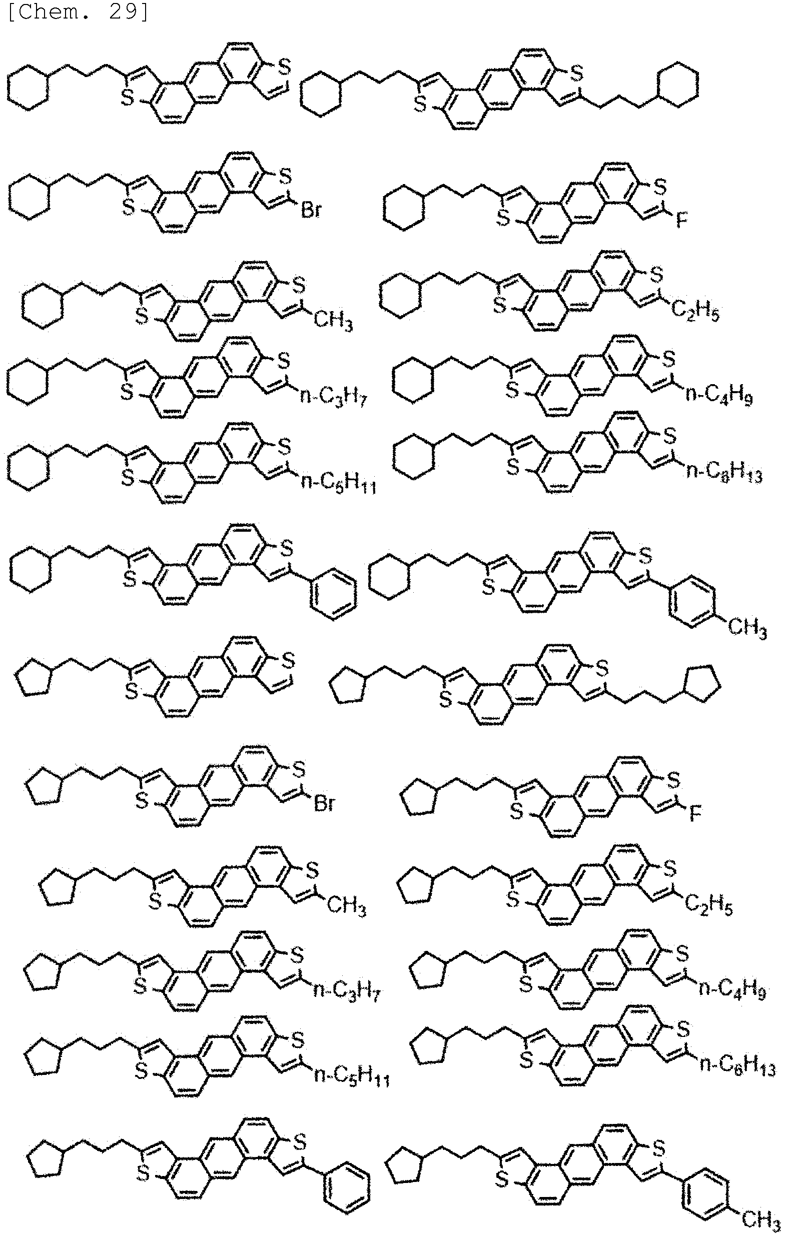

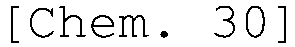

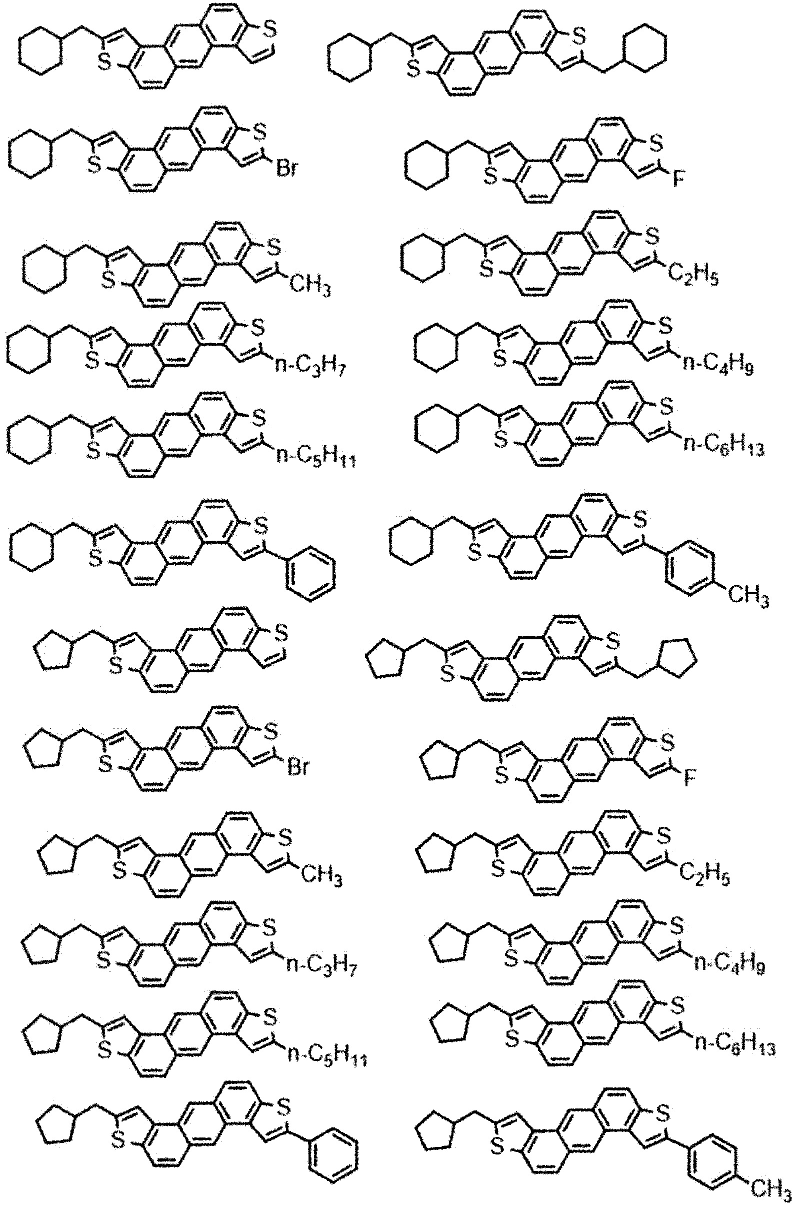

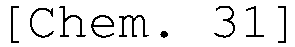

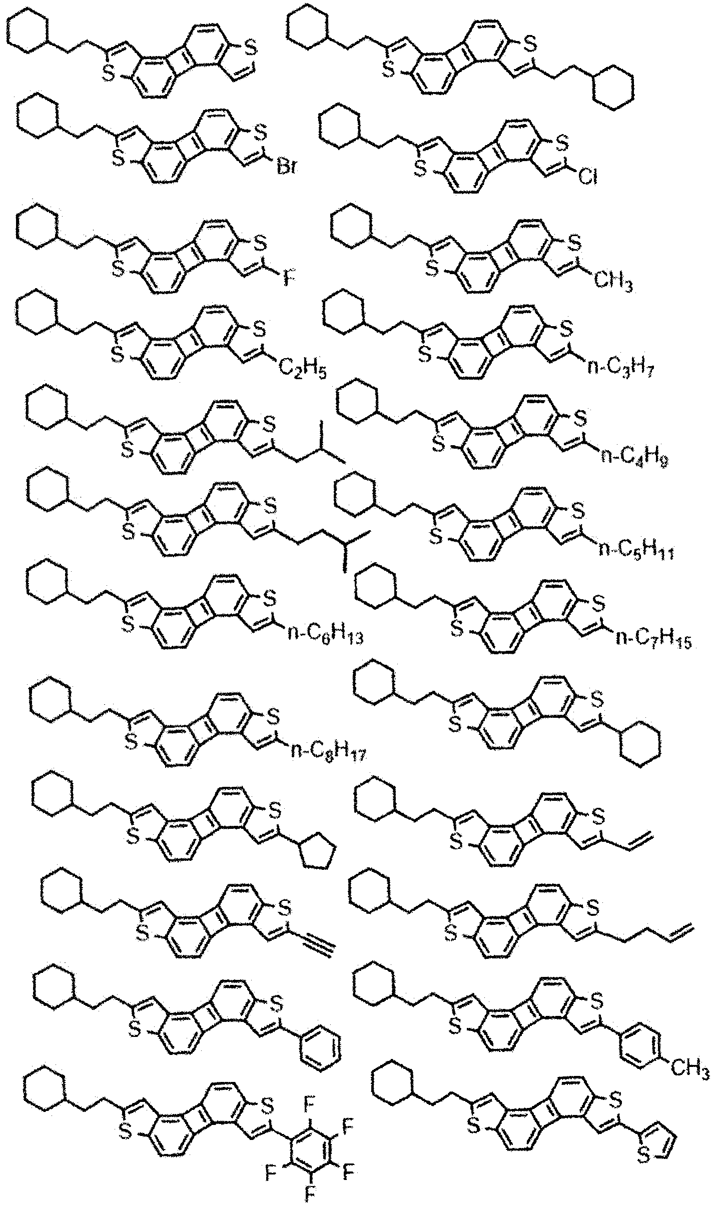

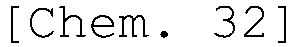

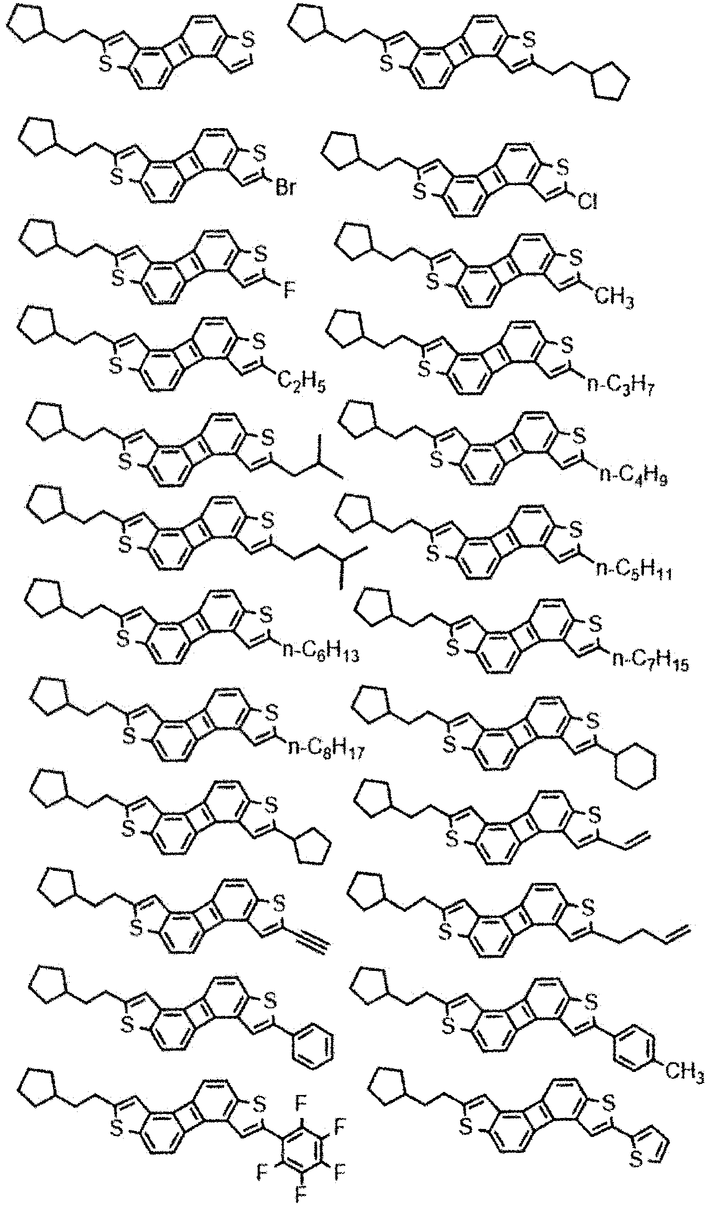

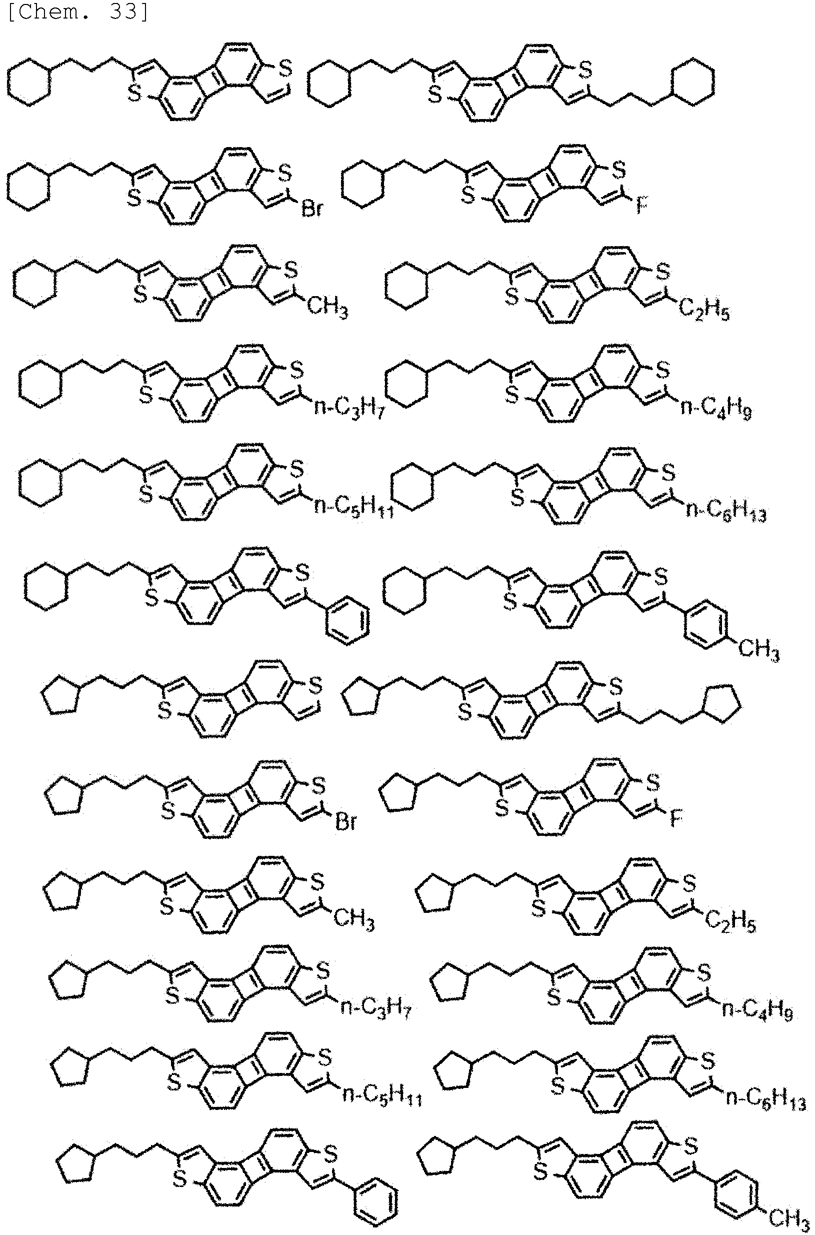

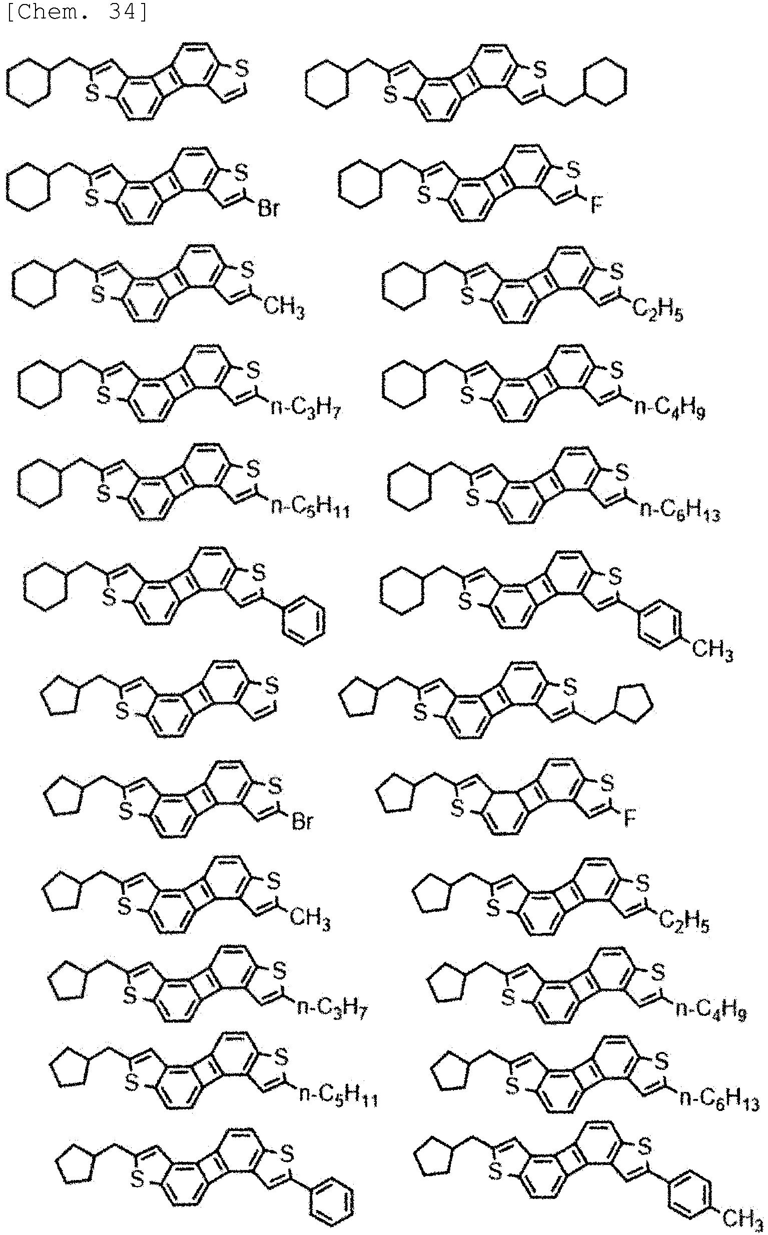

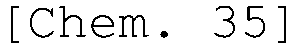

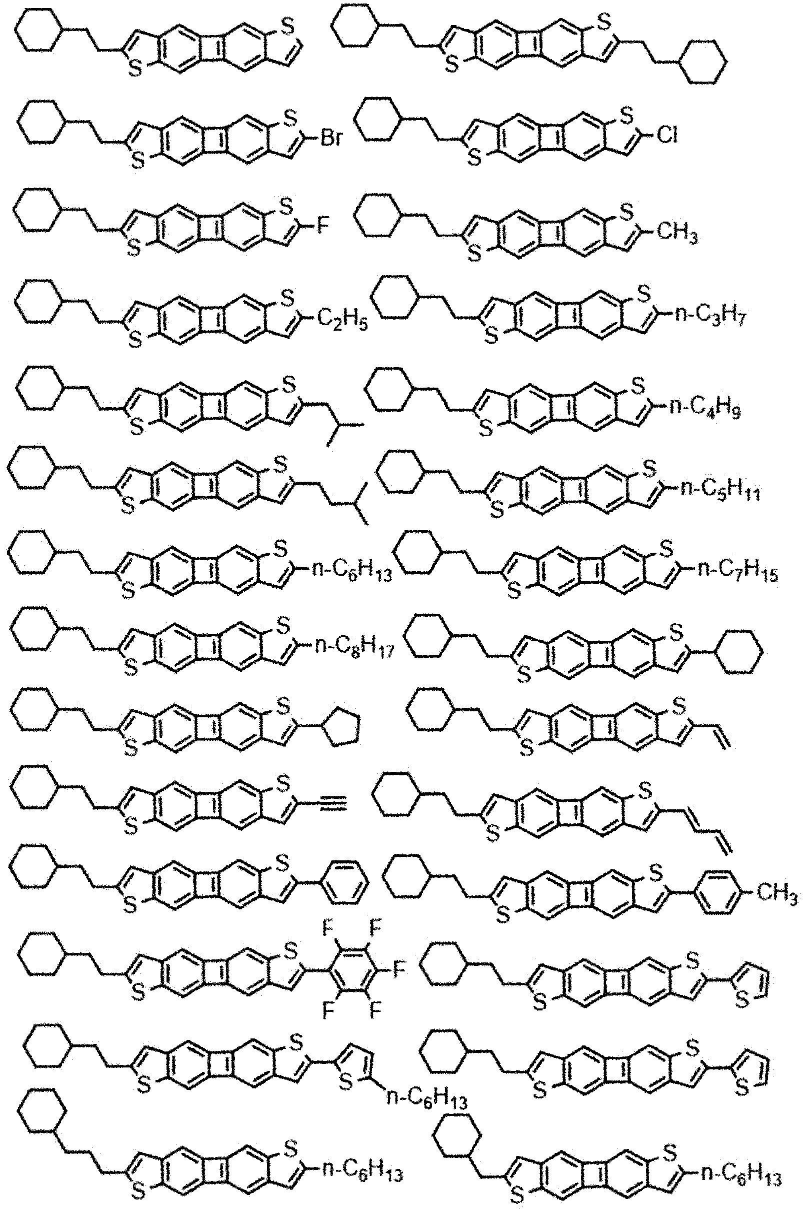

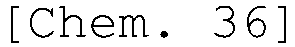

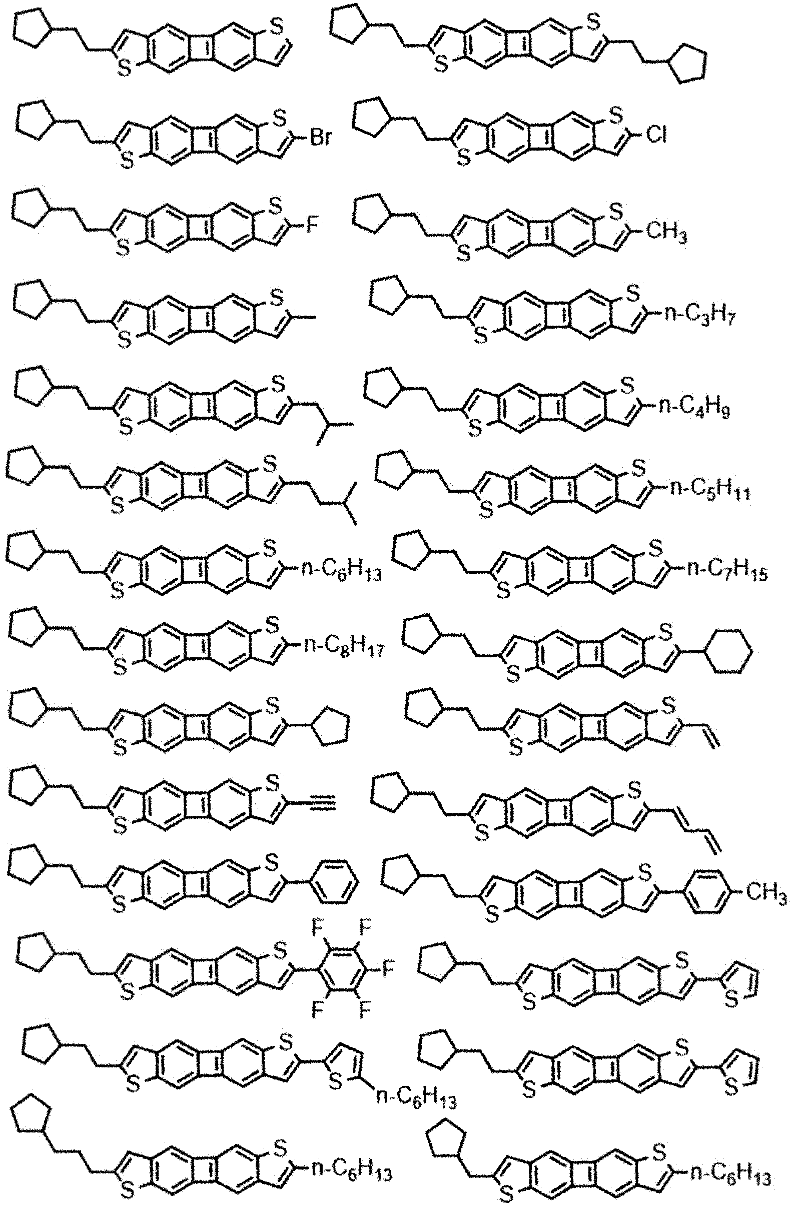

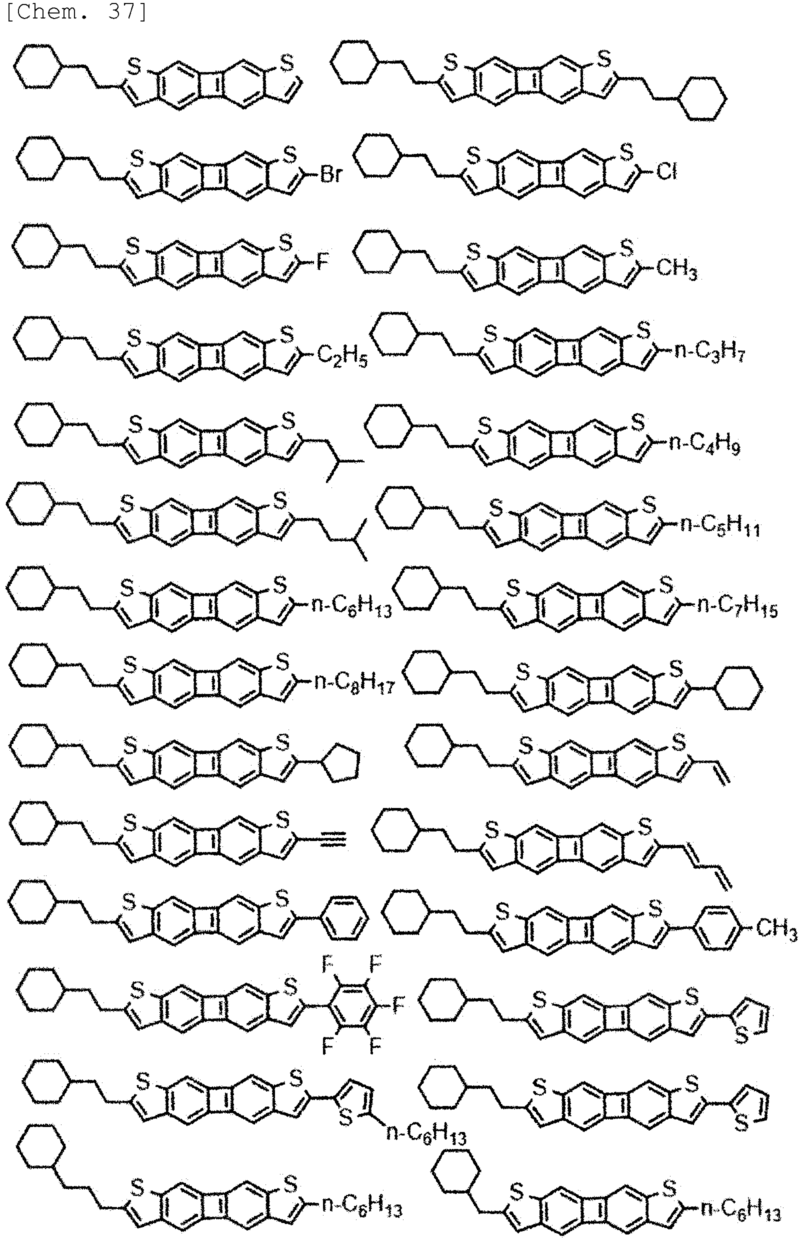

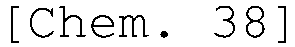

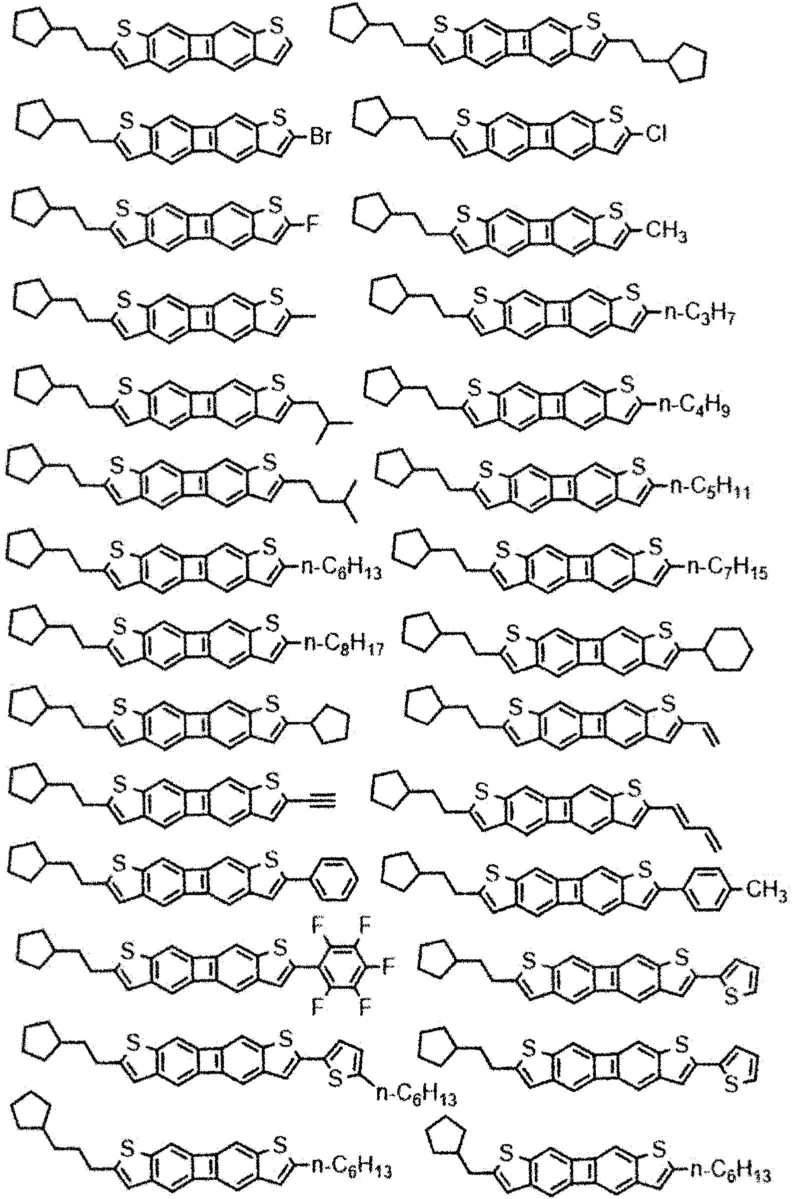

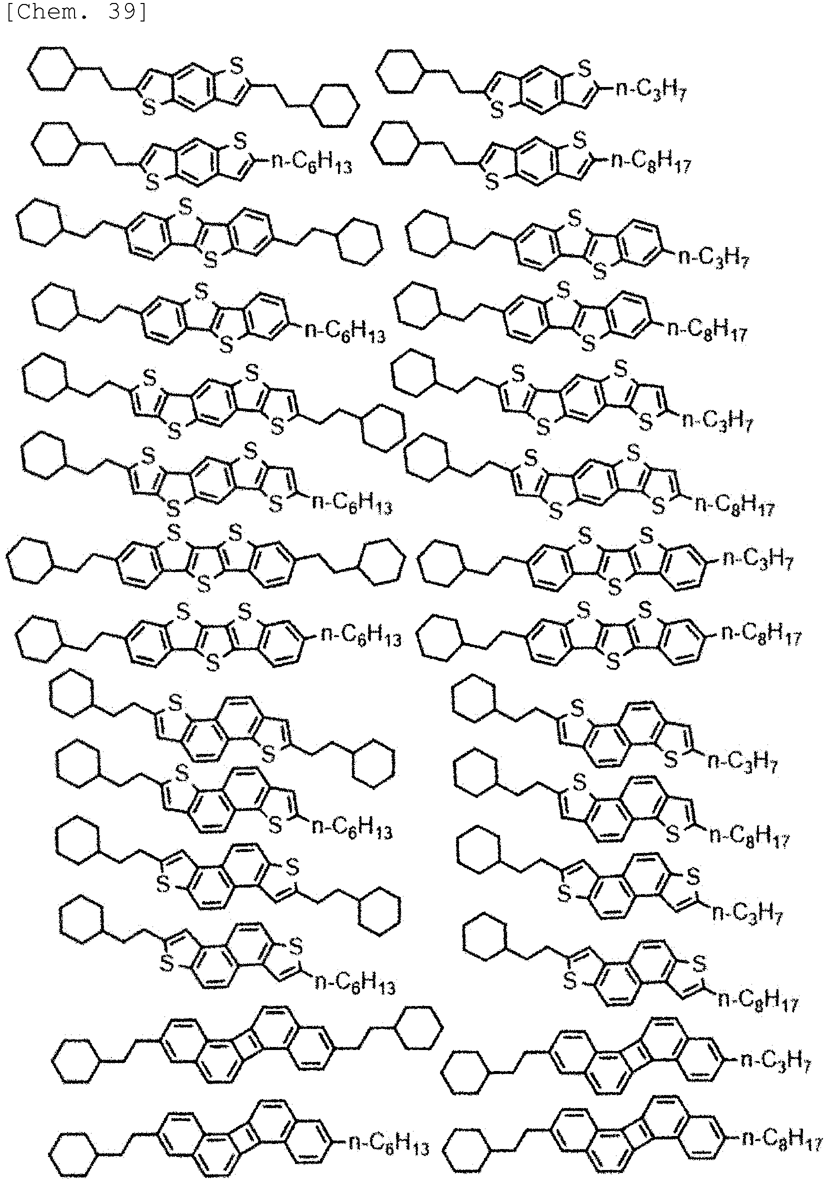

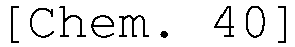

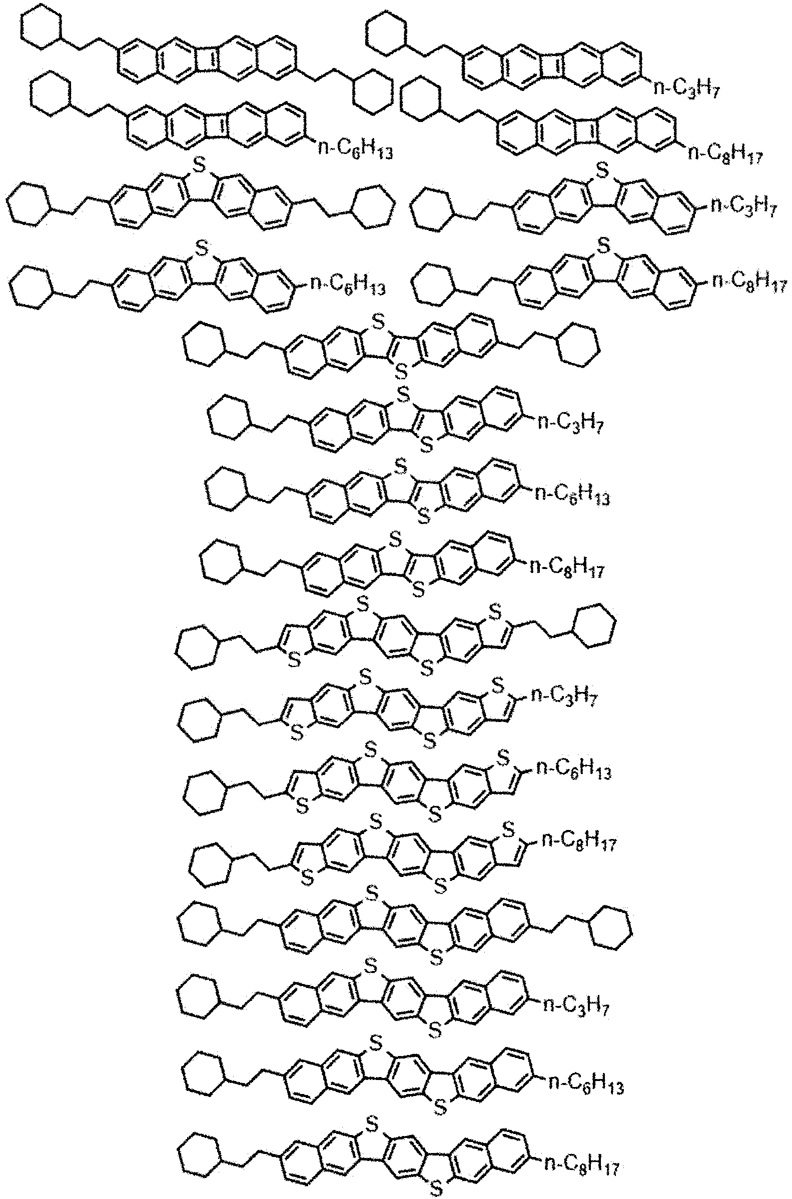

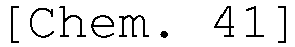

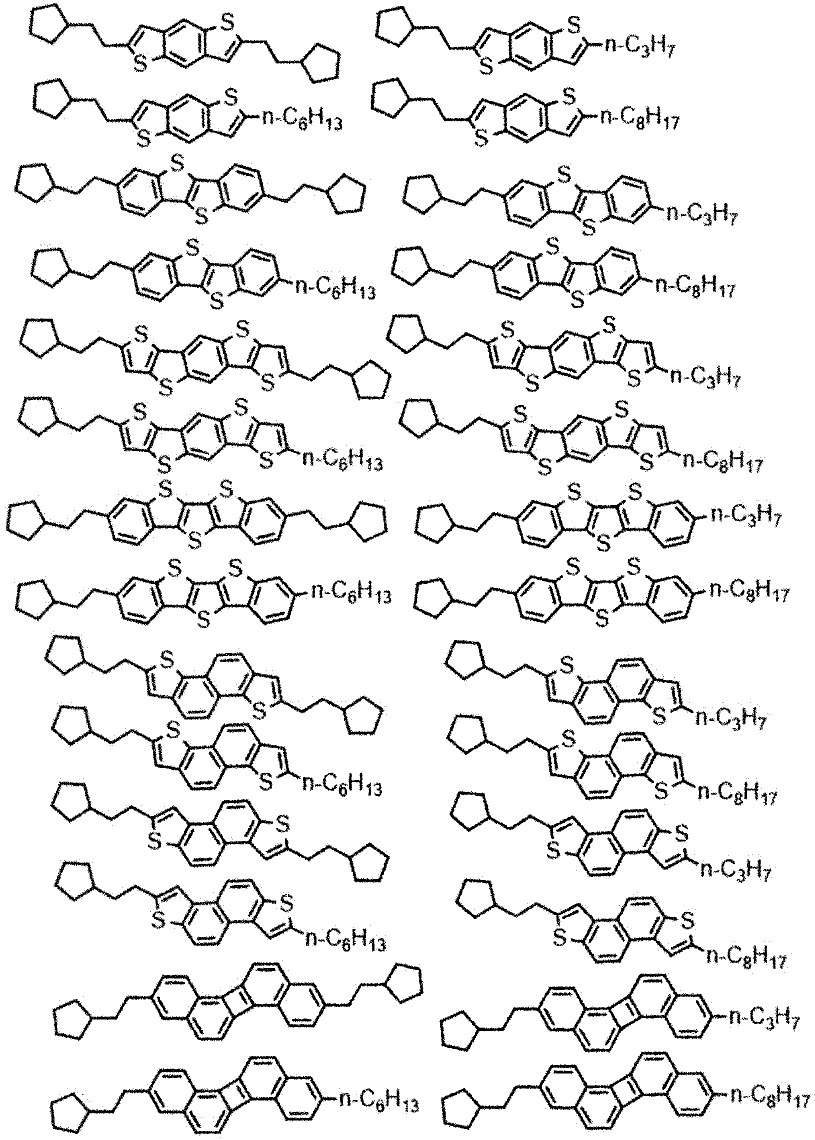

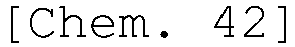

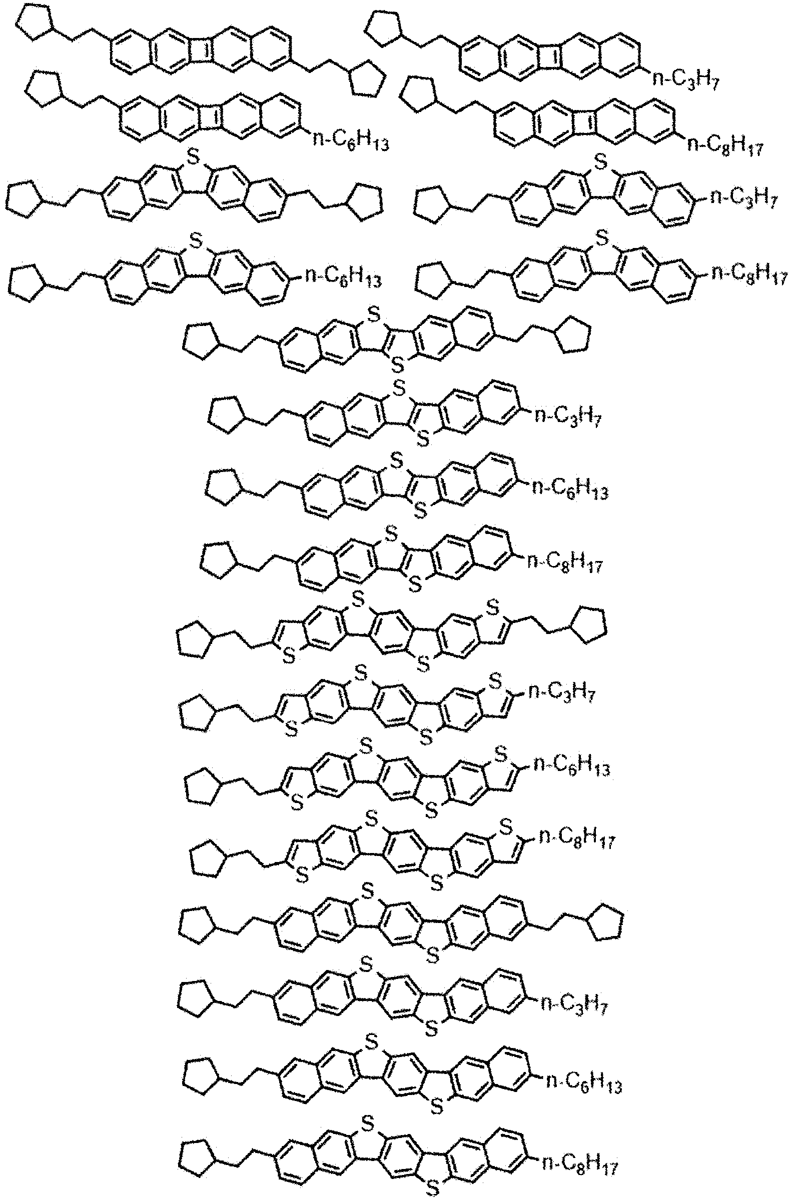

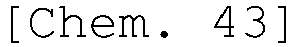

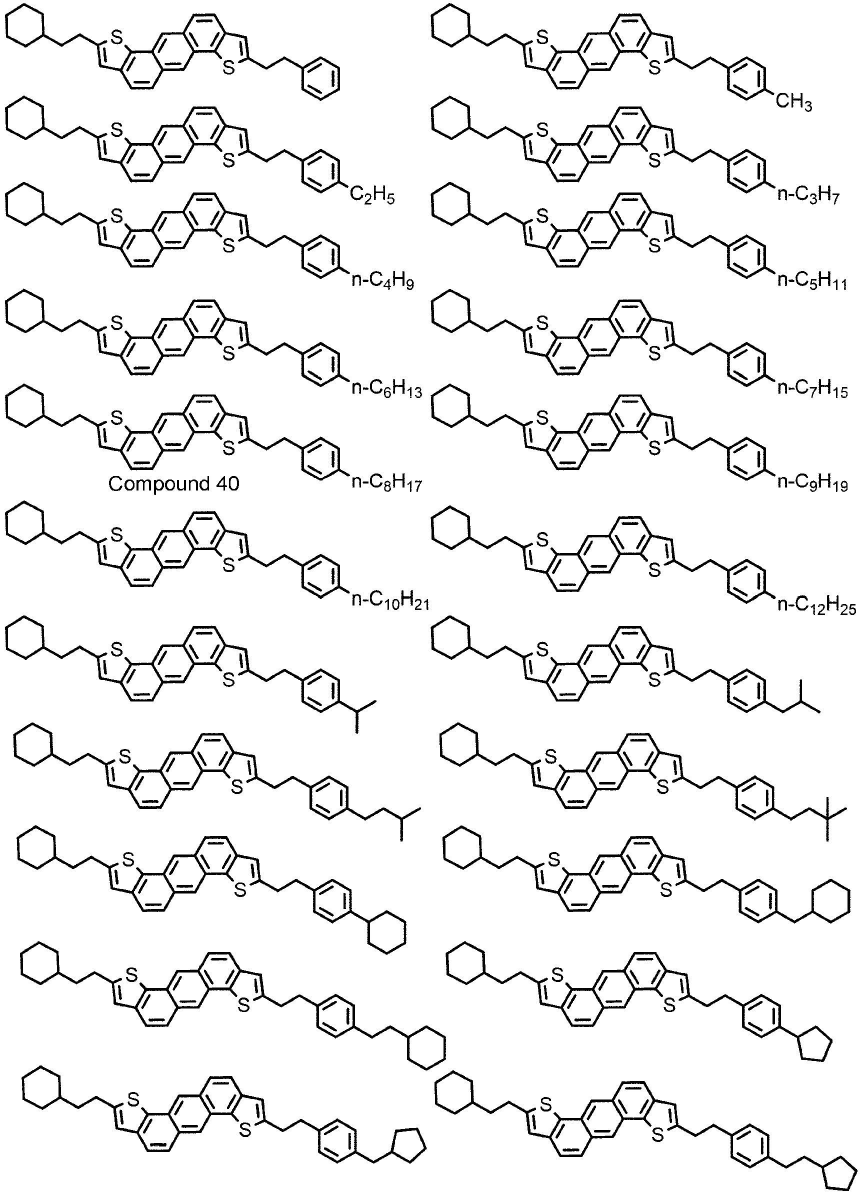

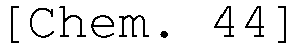

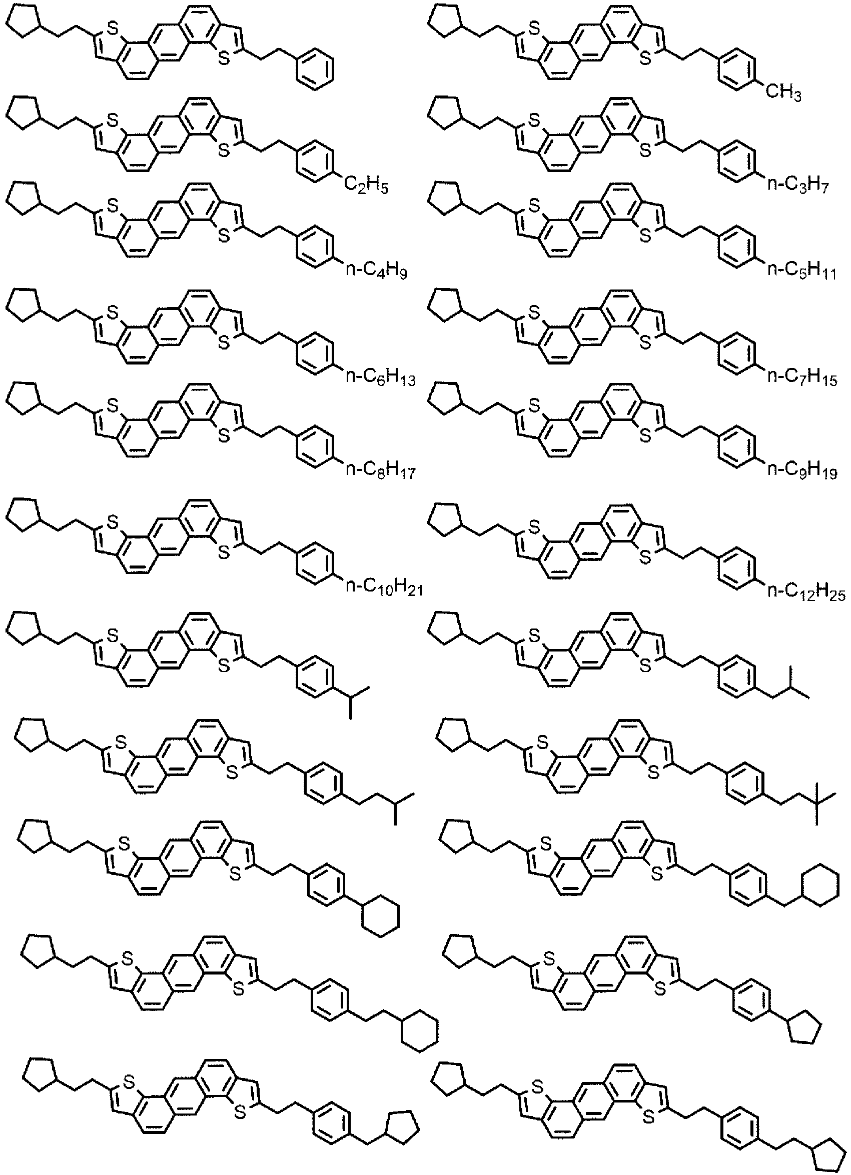

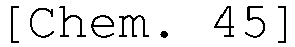

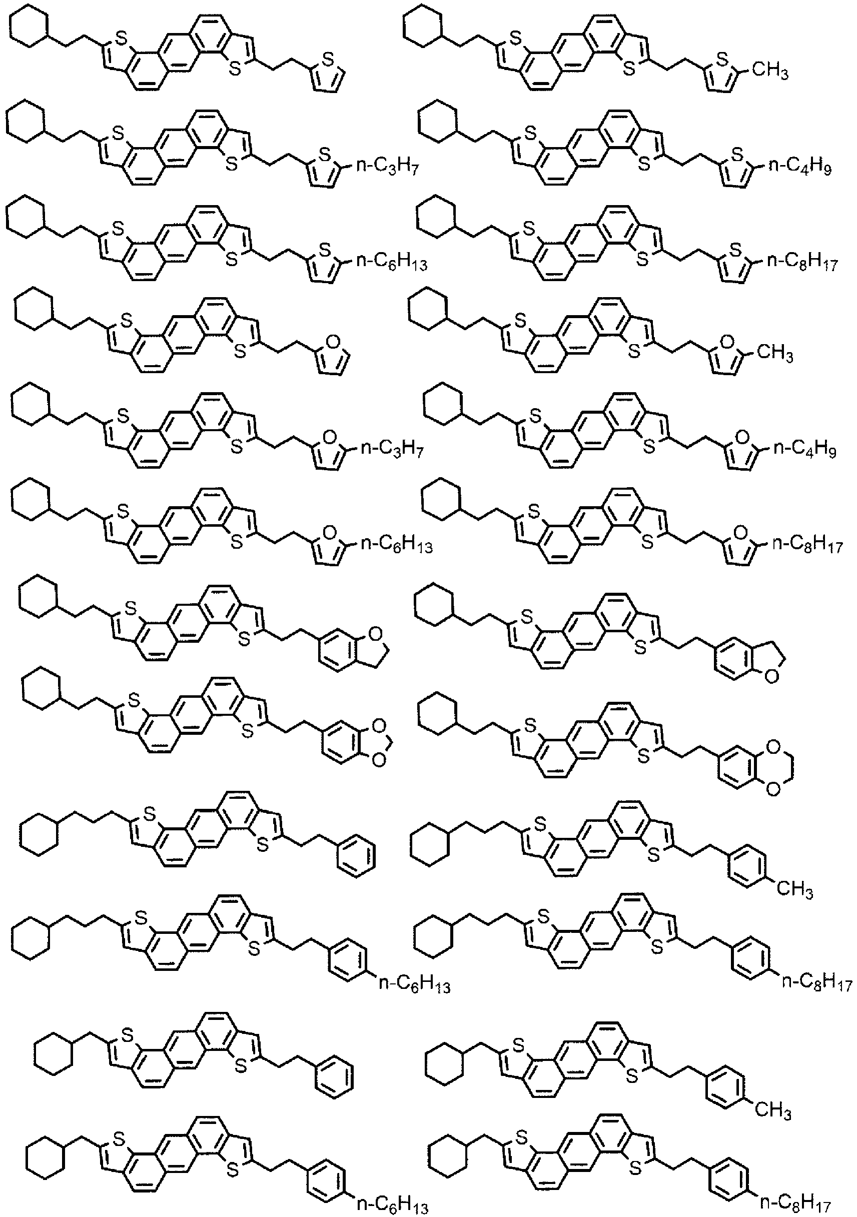

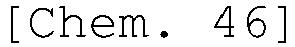

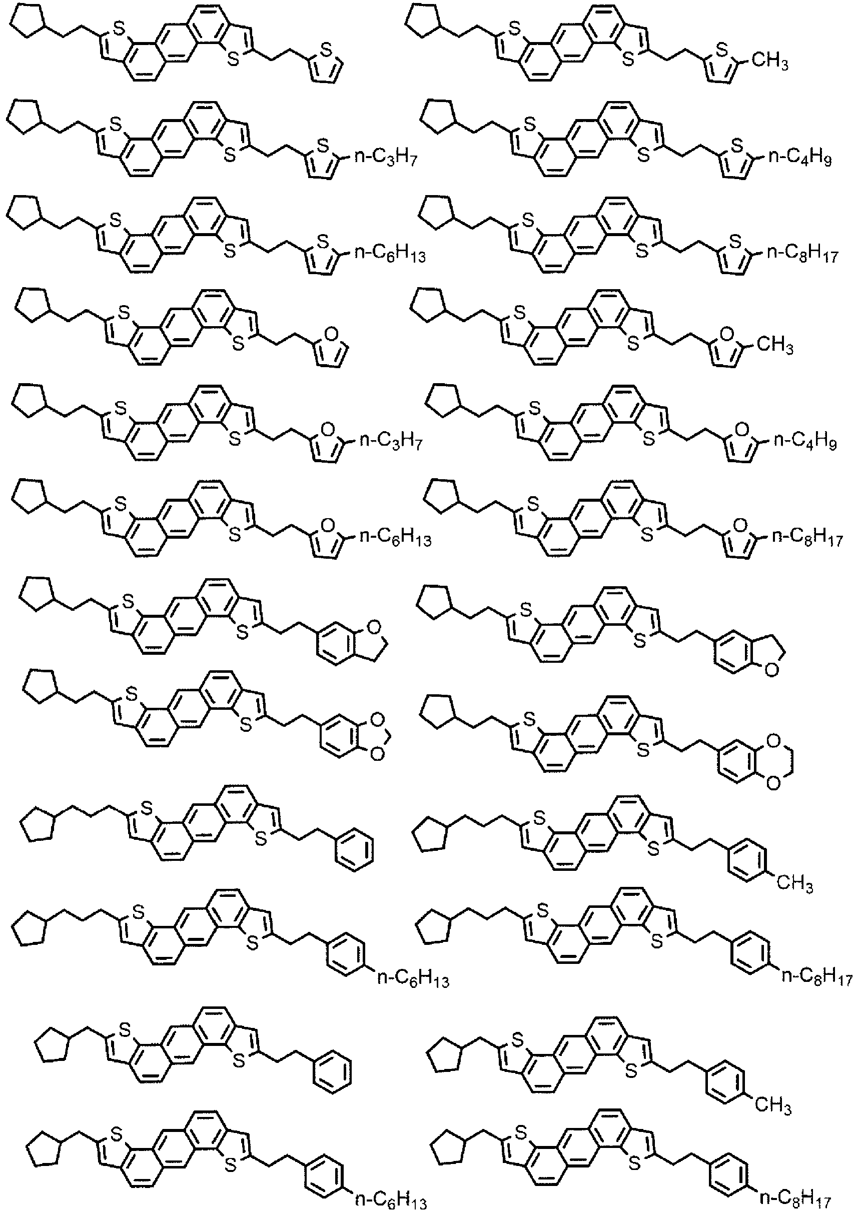

Of these, preferred compounds are the following compounds.

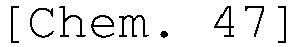

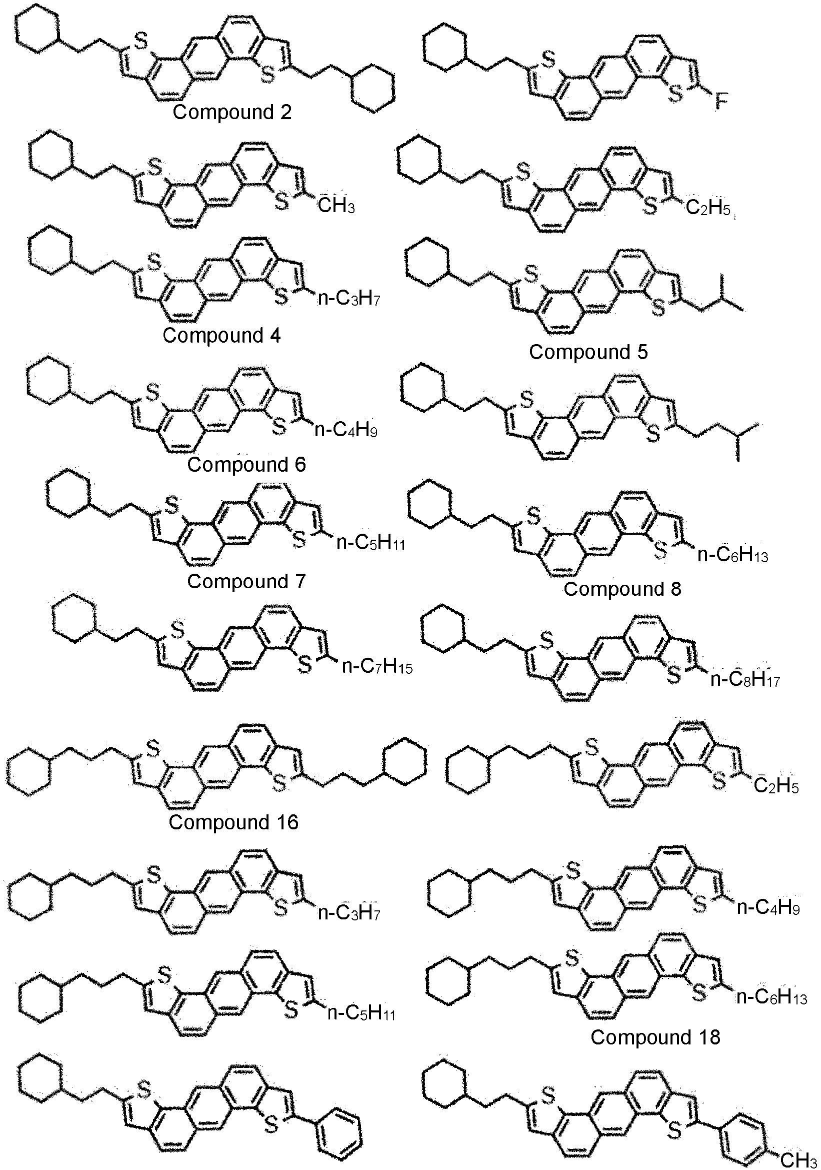

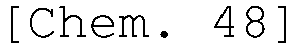

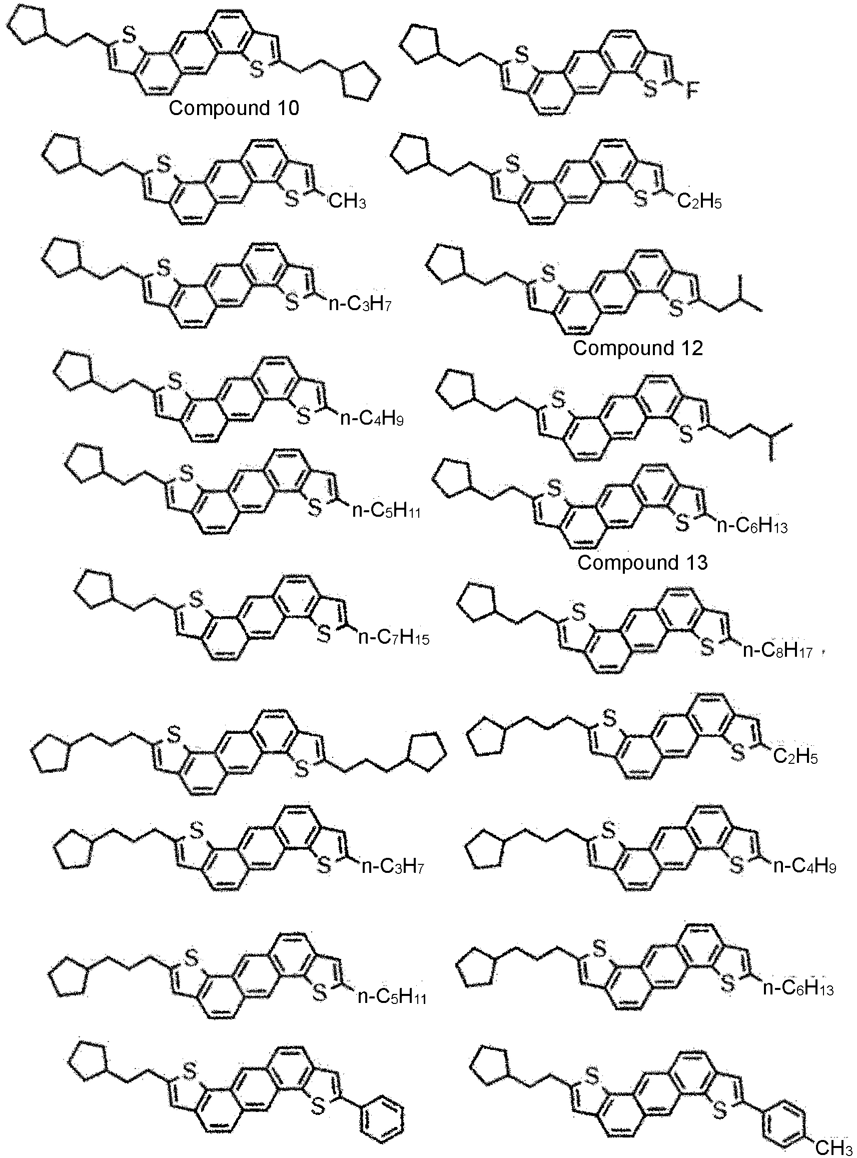

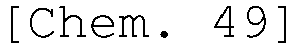

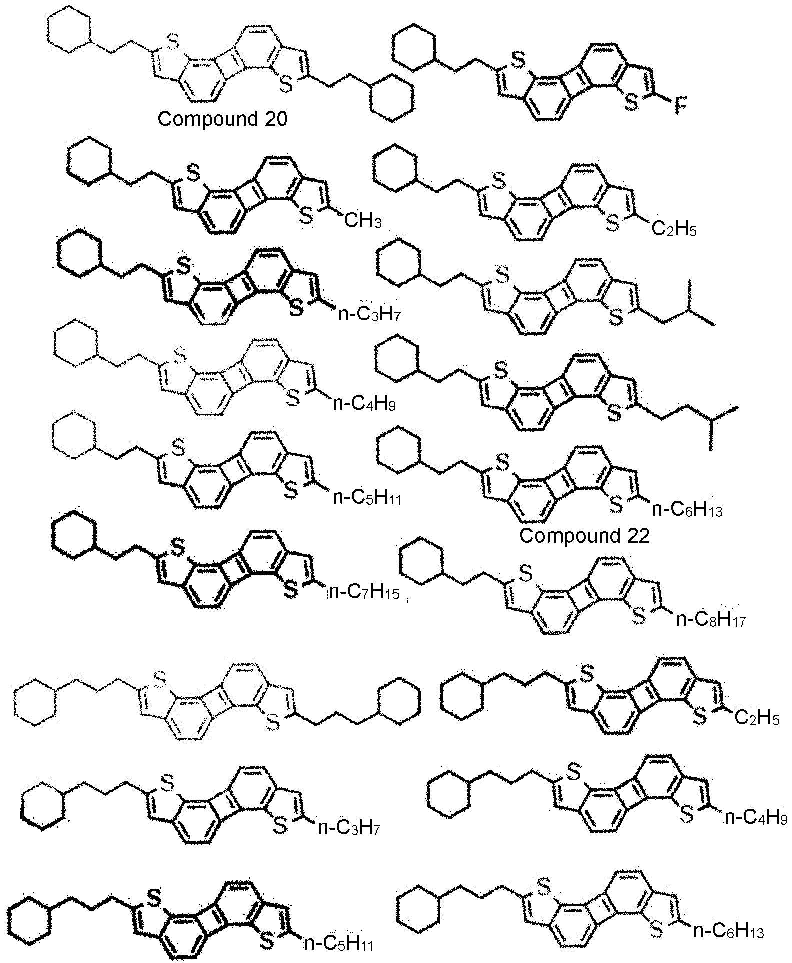

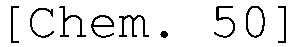

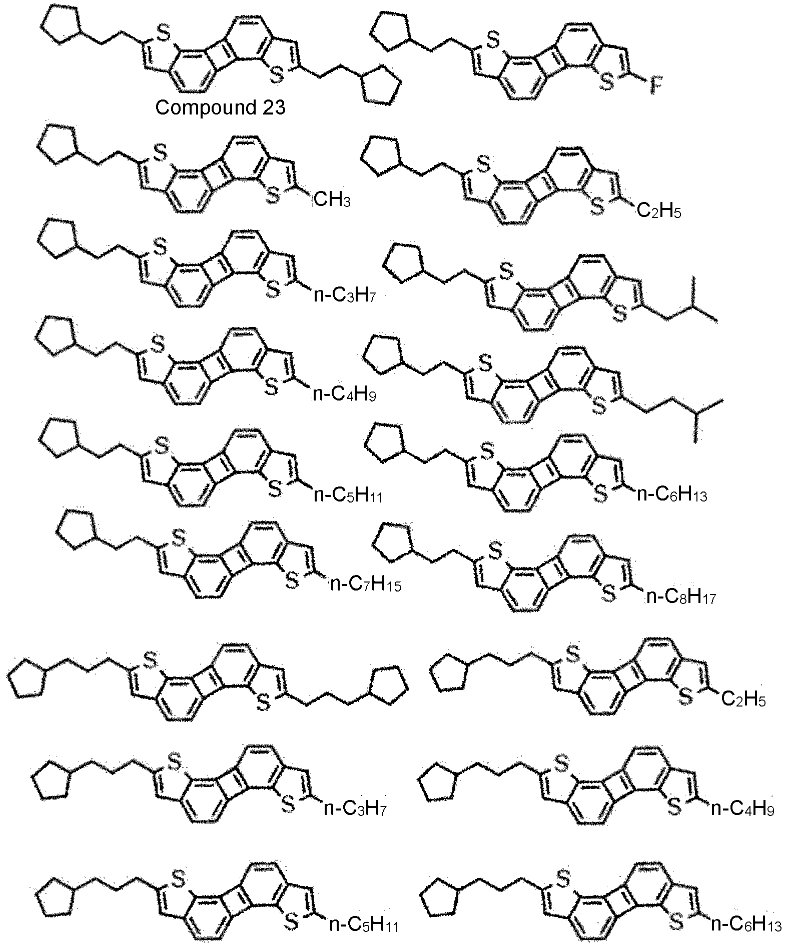

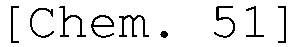

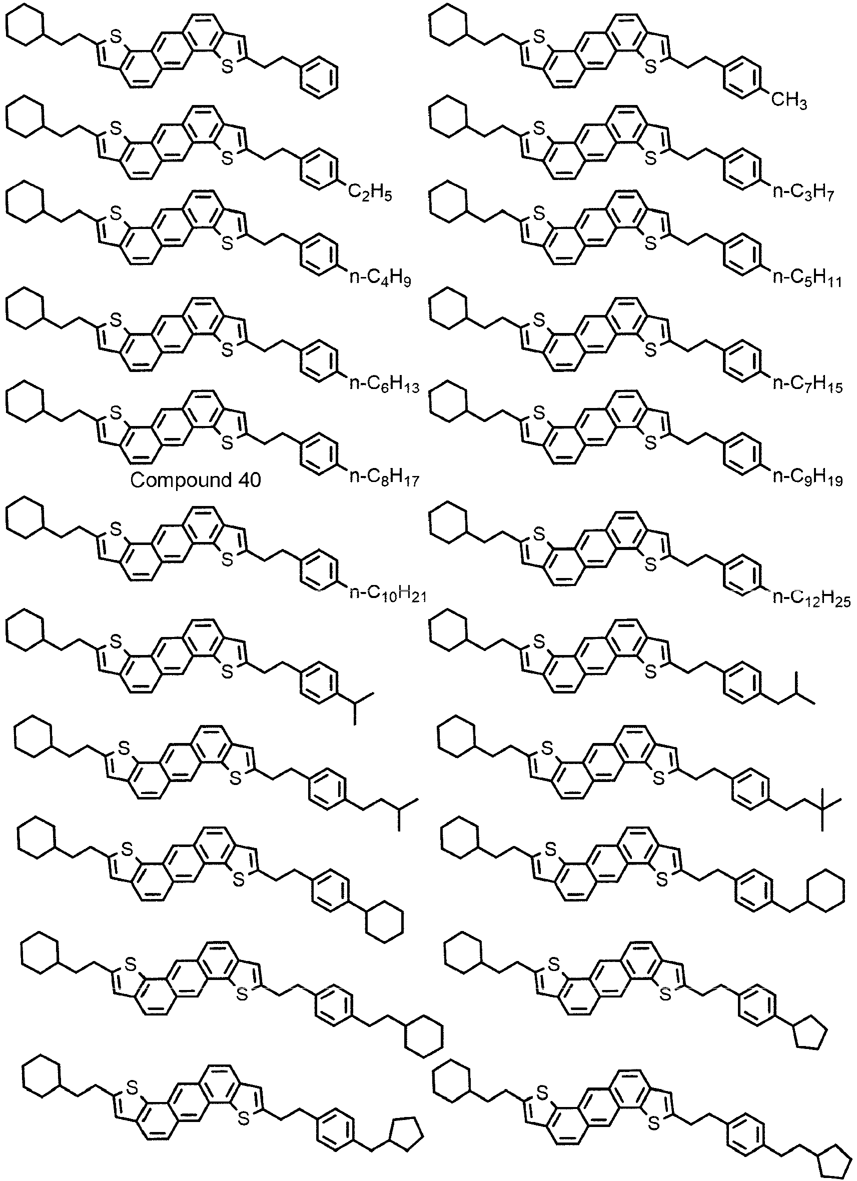

Of these, particularly preferred compounds are the following compounds. 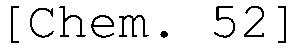 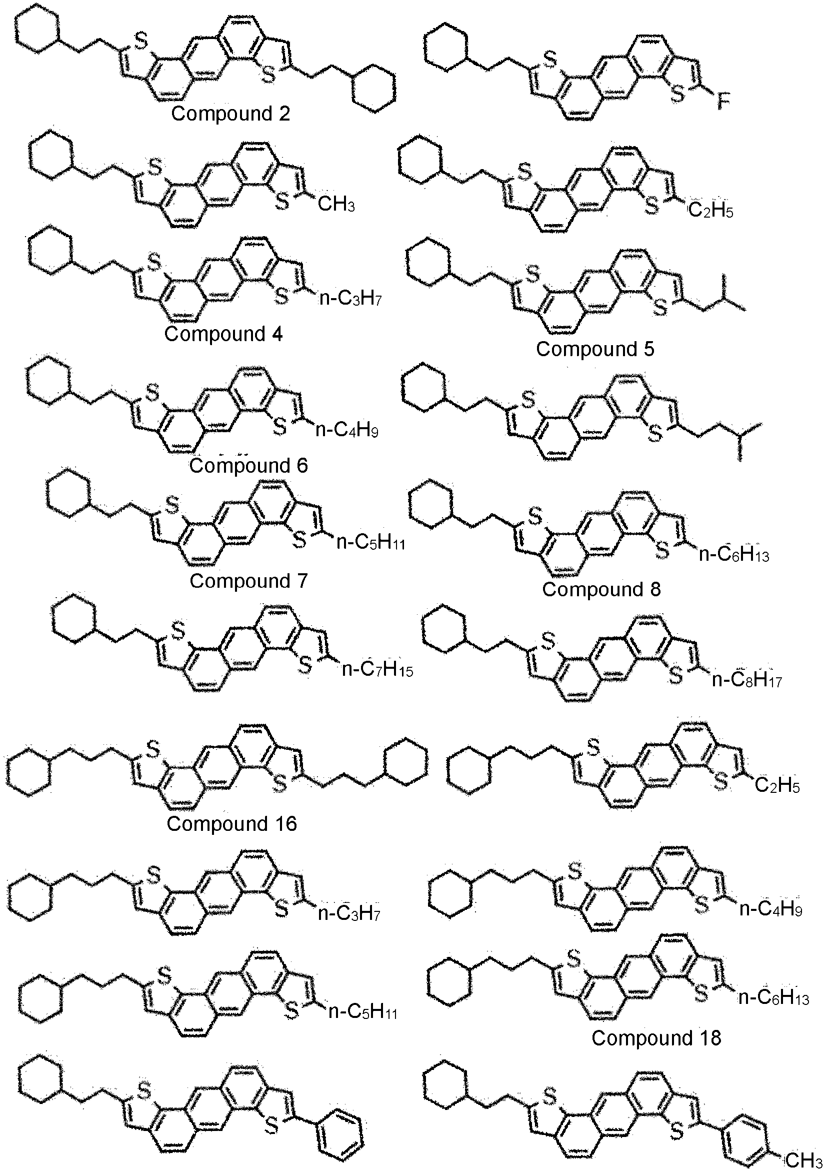 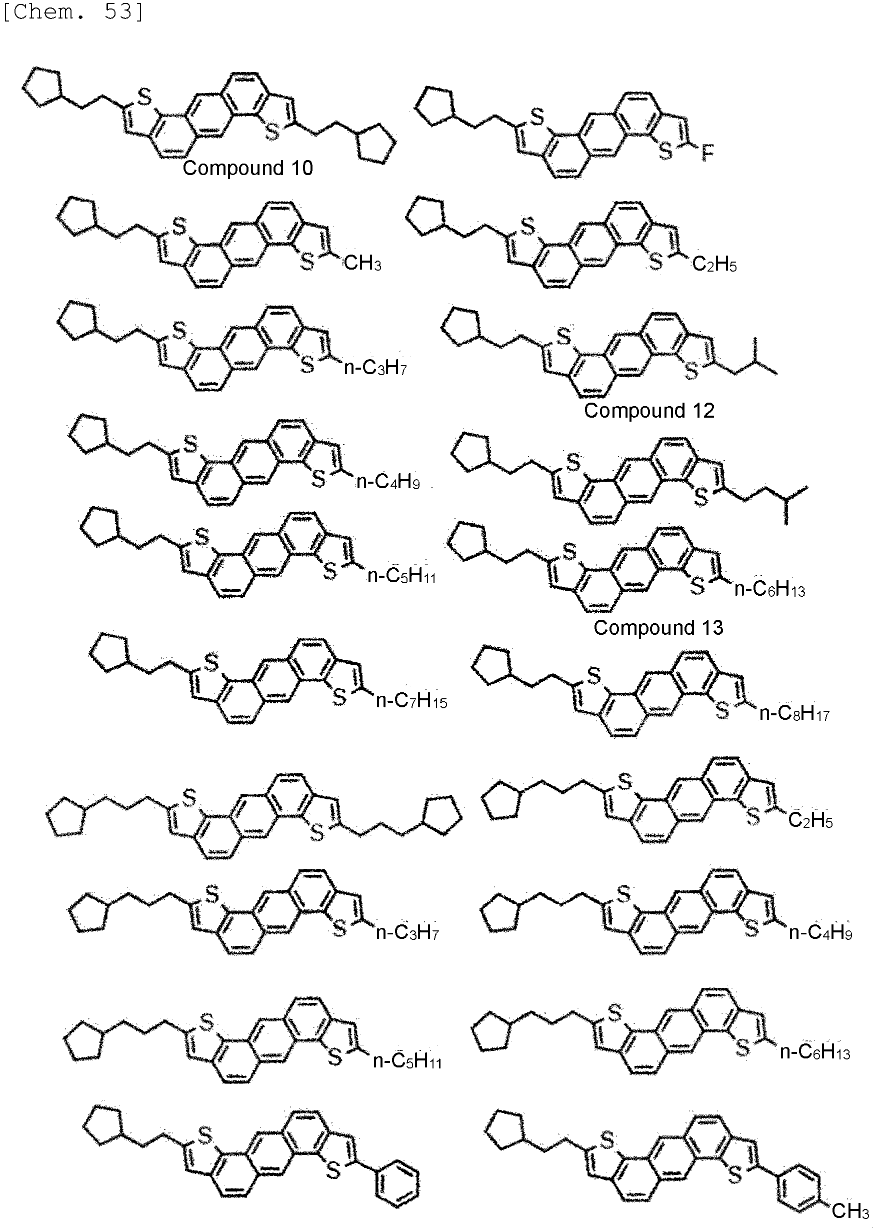 For the method for manufacturing the compound of the present invention, any manufacturing method can be used as long as the compound can be manufactured using it.

As an example of a method for manufacturing the aromatic compound according to the present invention, an aromatic compound (7-5a) in which X⁸ and X⁹ in formula (7-5) are sulfur atoms, Y⁵ and Y⁶ are CH, R³⁸ is a group represented by formula (2), R³⁹, R⁴⁰, R⁴¹, R⁴², R⁴³, R⁷¹ and R⁷² are hydrogen atoms, each of m and k in formula (2) is 0, and Z¹ to Z⁵ are hydrogen atoms, for example, can be manufactured using a method involving the steps of A1 or B1 and C1, described below.

(Step A1); A step of producing a boronate by allowing an alkyl bromide derivative to react with bis(pinacolato)diboron in the presence of a copper catalyst and a ligand.

(Step B1); A step of producing a boronate by allowing an alkene to react with borane and water to give a boronic acid and allowing it to react with pinacol.

(Step C1); A step of producing the aromatic compound (7-5a) by allowing the boronate obtained through step A1 or step B1 to react with 2-bromoanthra[1,2-b:5,6-b']dithiophene in the presence of a base and a palladium catalyst.

The details of each step are presented below.

### (Step A1)

The step A1 is a step of producing a boronate by allowing an alkyl bromide derivative to react with bis(pinacolato)diboron in the presence of a copper catalyst and a ligand.

Examples of copper catalysts in this step include copper(I) iodide, copper(I) bromide and copper(I) chloride. The copper catalyst may have a ligand, and examples of such ligands include Xantphos, Ruphos and Xphos.

Examples of alkyl bromide derivatives in step A1 include (2-bromoethyl)cyclohexane, (3-bromopropyl)cyclohexane, (2-bromoethyl)cyclopentane and (3-bromopropyl)cyclopentane.

### (Step B1)

The step B1 is a step of producing a boronate by allowing an alkene to react with borane and water to give a boronic acid and allowing it to react with pinacol.

For the conditions for the preparation of the boronic acid, the process can be performed, for example, within a temperature range of 0°C to 40°C in a solvent such as THF or diethyl ether using 1 to 3 equivalents of borane.

During the reaction between the boronic acid and pinacol, furthermore, magnesium sulfate or sodium sulfate, for example, can be added as a dehydrating agent.

Examples of alkenes in step B1 include vinylcyclohexane, allylcyclohexane, vinylcyclopentane and allylcyclopentane.

### (Step C1)

The step C1 is a step of producing the aromatic compound (7-5a) through a Suzuki coupling reaction between the boronate obtained in step A1 or B1 and 2-bromoanthra[1,2-b:5,6-b']dithiophene in the presence of a palladium catalyst.

The reaction can be performed, for example, within a temperature range of 20°C to 100°C in a solvent such as toluene, N,N-dimethylformamide (Hereinafter abbreviated as DMF.), N,N-dimethylacetamide (Hereinafter abbreviated as DMA.) or THF. In addition, water may be added as a solvent.

Examples of palladium catalysts in step C1 include palladium acetate, [1,1'-bis(diphenylphosphino)ferrocene]dichloropalladium(II), tetrakis(triphenylphosphine)palladium and dichlorobis(triphenylphosphine)palladium. The palladium catalyst may have a ligand, and Ruphos, Xantphos or Xphos, for example, can be added as the ligand.

Examples of bases usable in step C1 include potassium tert-butoxide, sodium tert-butoxide, sodium hydroxide and potassium hydroxide.

A more specific manufacturing method that is preferred because of the ease of synthesis, furthermore, is presented in the following reaction scheme. 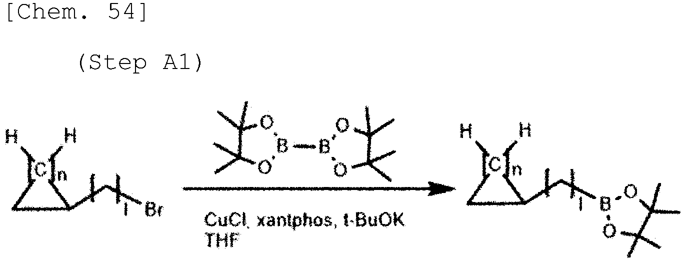 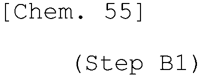 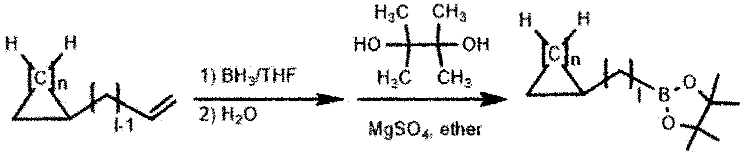 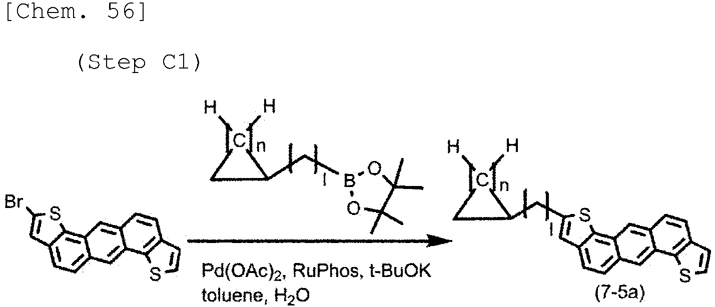 (In the formulae, n and l are synonymous with n and l indicated in formula (2).)

Moreover, as another example of a method for manufacturing the aromatic compound according to the present invention, an aromatic compound (7-5b) in which X⁸ and X⁹ in formula (7-5) are sulfur atoms, Y⁵ and Y⁶ are CH, R³⁸ and R⁴¹ are groups represented by formula (2), R³⁹, R⁴⁰, R⁴², R⁴³, R⁷¹ and R⁷² are hydrogen atoms, each of m and k in formula (2) is 0, and Z¹ to Z⁵ are hydrogen atoms, for example, can be manufactured using a method involving the steps of A1 or B1, described above, and C2, described below.

(Step C2); A step of producing the aromatic compound (7-5b) by allowing the boronate obtained through step A1 or step B1 to react with 2,8-dibromoanthra[1,2-b:5,6-b']dithiophene in the presence of a base and a palladium catalyst.

The details of each step are presented below.

### (Step C2)

The step C2 is a step of producing the aromatic compound (7-5b) through a Suzuki coupling reaction between the boronate obtained in step A1 or B1 and 2,8-bromoanthra[1,2-b:5,6-b']dithiophene in the presence of a base and a palladium catalyst.

The reaction can be performed, for example, within a temperature range of 20°C to 100°C in a solvent such as toluene, DMF, DMA or THF. In addition, water may be added as a solvent.

Examples of palladium catalysts in step C2 include palladium acetate, [1,1'-bis(diphenylphosphino)ferrocene]dichloropalladium(II), tetrakis(triphenylphosphine)palladium and dichlorobis(triphenylphosphine)palladium. The palladium catalyst may have a ligand, and Ruphos, Xantphos or Xphos, for example, can be added as the ligand.

Examples of bases usable in step C2 include potassium tert-butoxide, sodium tert-butoxide, sodium hydroxide and potassium hydroxide.

A more specific reaction in step C2 that is preferred because of the ease of synthesis, furthermore, is presented in the following reaction scheme. 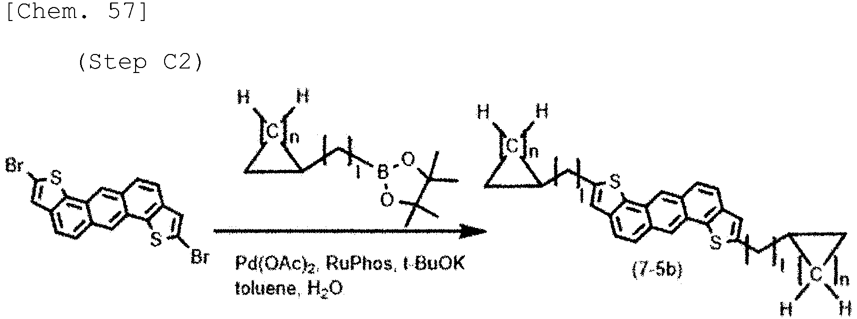 (In the formula, n and l are synonymous with n and l indicated in formula (2).)

As another example of a method for manufacturing the aromatic compound according to the present invention, furthermore, an aromatic compound (7-5d) in which X⁸ and X⁹ in formula (7-5) are sulfur atoms, Y⁵ and Y⁶ are CH, R³⁸ is a group represented by formula (2), R³⁹, R⁴⁰, R⁴², R⁴³, R⁷¹ and R⁷² are hydrogen atoms, each of m and k in formula (2) is 0, and Z¹ to Z⁵ are hydrogen atoms, for example, can be manufactured using a method involving step D1 and step C3, described below, or a method involving step C4.

(Step D1); A step of turning an anthradithiophene derivative (7-5a) into a monolithium salt using butyllithium and then synthesizing a monobromoanthradithiophene derivative (7-5c) using a brominating agent.

(Step C3); A step of producing the aromatic compound (7-5d) by allowing a boronate or boronic acid to react with the monobromoanthradithiophene derivative (7-5c) obtained in step D1 in the presence of a base and a palladium catalyst.

(Step C4); A step of producing the aromatic compound (7-5d) by allowing a boronate obtained through step A1 or step B1, described above, to react with a monobromoanthradithiophene derivative in the presence of a base and a palladium catalyst.

The details of each step are presented below.

### (Step D1)

The step D1 is a method of turning an anthradithiophene derivative into a monolithium salt using 1 equivalent of butyllithium and allowing it to react with a brominating agent to produce a monobromo derivative.

For the conditions for the preparation of the monolithium salt, the process can be performed, for example, within a temperature range of -80°C to 30°C in a solvent such as THF or diethyl ether using 0.5 to 1.5 equivalents of n-butyllithium or tert-butyllithium.

The brominating agent can be, for example, tetrabromomethane or 1,2-dibromotetrachloroethane.

### (Step C3)

The step C3 is a step of producing the aromatic compound (7-5d) through a Suzuki coupling reaction between a boronate or boronic acid and the monobromoanthradithiophene derivative (7-5c) obtained in step D1 in the presence of a base and a palladium catalyst.

The reaction can be performed, for example, within a temperature range of 20°C to 100°C in a solvent such as toluene, DMF, DMA or THF. In addition, water may be added as a solvent.

Examples of palladium catalysts in step C3 include palladium acetate, [1,1'-bis(diphenylphosphino)ferrocene]dichloropalladium(II), tetrakis(triphenylphosphine)palladium and dichlorobis(triphenylphosphine)palladium. The palladium catalyst may have a ligand, and Ruphos, Xantphos or Xphos, for example, can be added as the ligand.

Examples of bases usable in step C3 include potassium tert-butoxide, sodium tert-butoxide, sodium hydroxide and potassium hydroxide.

### (Step C4)

The step C4 is a step of producing the aromatic compound (7-5d) through a Suzuki coupling reaction between a boronate obtained through step A1 or step B1, described above, and a monobromoanthradithiophene derivative in the presence of a base and a palladium catalyst.

The reaction can be performed, for example, within a temperature range of 20°C to 100°C in a solvent such as toluene, DMF, DMA or THF. In addition, water may be added as a solvent.

Examples of palladium catalysts in step C4 include palladium acetate, [1,1'-bis(diphenylphosphino)ferrocene]dichloropalladium(II), tetrakis(triphenylphosphine)palladium and dichlorobis(triphenylphosphine)palladium. The palladium catalyst may have a ligand, and Ruphos, Xantphos or Xphos, for example, can be added as the ligand.

Examples of bases usable in step C4 include potassium tert-butoxide, sodium tert-butoxide, sodium hydroxide and potassium hydroxide.

More specific manufacturing methods with step D1, step C3 and step C4 that are preferred because of the ease of synthesis, furthermore, are presented in the following reaction schemes. 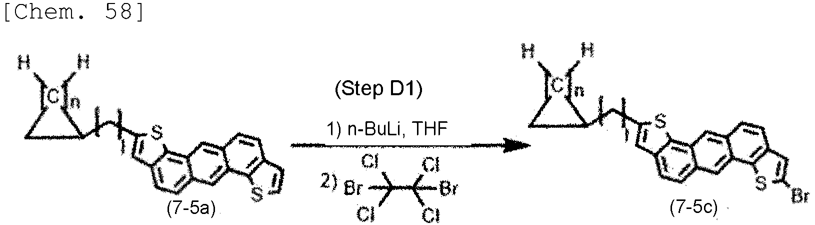 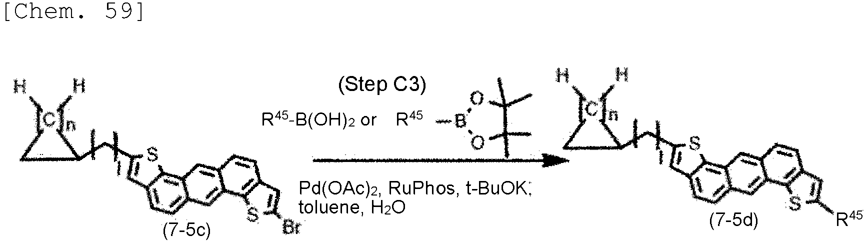 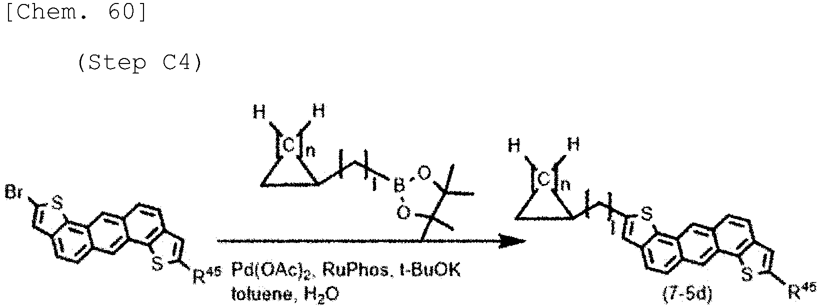 (In the formulae, n and l are synonymous with n and l indicated in formula (2), and R⁴⁵ is synonymous with R⁴⁵ indicated in formula (7-5).)

Moreover, as another example of a method for manufacturing the aromatic compound according to the present invention, an aromatic compound (7-1a) in which X⁸ and X⁹ in formula (7-1) are sulfur atoms, Y⁵ and Y⁶ are CH, R³⁸ is a group represented by formula (2), R³⁹, R⁴⁰, R⁴¹, R⁴² and R⁴³ are hydrogen atoms, each of m and k in formula (2) is 0, and Z¹ to Z⁵ are hydrogen atoms, for example, can be manufactured through the steps of E1 and F1, described below.

(Step E1); A step of producing an alkylmagnesium bromide by allowing an alkyl bromide derivative to react with magnesium.

(Step F1); A step of producing the aromatic compound (7-1a) by allowing an alkylzinc chloride derived from the alkylmagnesium bromide obtained through step E1 to react with 2-bromobiphenyleno[1,2-b:5,6-b']dithiophene in the presence of a palladium catalyst.

The details of each step are presented below.

### (Step E1)

The step E1 is a step of producing an alkylmagnesium bromide by allowing an alkyl bromide derivative to react with magnesium.

For the conditions for the preparation of the magnesium salt, namely the alkylmagnesium bromide, the process can be performed, for example, within a temperature range of 25°C to 60°C in a solvent such as THF or diethyl ether using 1 to 2 equivalents of magnesium.

Examples of alkyl bromide derivatives in step E1 include (2-bromoethyl)cyclohexane, (3-bromopropyl)cyclohexane, (2-bromoethyl)cyclopentane and (3-bromopropyl)cyclopentane.

### (Step F1)

The step F1 is a step of producing the aromatic compound (7-1a) by allowing an alkylzinc chloride derived from the alkylmagnesium bromide obtained through step E1 to react with 2-bromobiphenyleno[1,2-b:5,6-b']dithiophene in the presence of a palladium catalyst.

For the conditions for the preparation of the alkylzinc chloride from the alkylmagnesium bromide, the process can be performed using zinc chloride, for example within a temperature range of 0°C to 25°C in a solvent such as THF or diethyl ether.

Examples of palladium catalysts in step F1 include [1,1'-bis(diphenylphosphino)ferrocene]dichloropalladium(II), tetrakis(triphenylphosphine)palladium and dichlorobis(triphenylphosphine)palladium. The reaction temperature can be, for example, within a range of 20°C to 60°C.

A more specific manufacturing method with step E1 and step F1 that is preferred because of the ease of synthesis, furthermore, is presented in the following reaction scheme. 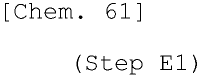 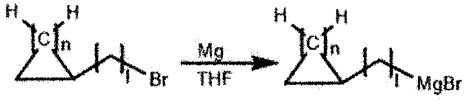 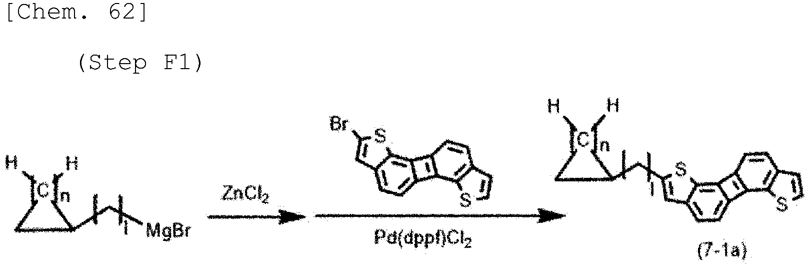 (In the formulae, n and l are synonymous with n and l indicated in formula (2).)

As another example of a method for manufacturing the aromatic compound according to the present invention, furthermore, an aromatic compound (7-1b) in which X⁸ and X⁹ in formula (7-1) are sulfur atoms, Y⁵ and Y⁶ are CH, R³⁸ and R⁴¹ are groups represented by formula (2), R³⁹, R⁴⁰, R⁴² and R⁴³ are hydrogen atoms, each of m and k in formula (2) is 0, and Z¹ to Z⁵ are hydrogen atoms, for example, can be manufactured through the steps of E1, described above, and F2, described below.

(Step F2); A step of producing the aromatic compound (7-1b) by allowing an alkylzinc chloride derived from the alkylmagnesium bromide obtained through step E1 to react with 2,7-dibromobiphenyleno[1,2-b:5,6-b']dithiophene in the presence of a palladium catalyst.

The details of each step are presented below.

### (Step F2)

The step F2 is a step of producing the aromatic compound (7-1b) by allowing an alkylzinc chloride derived from the alkylmagnesium bromide obtained through step E1 to react with 2,7-dibromobiphenyleno[1,2-b:5,6-b']dithiophene in the presence of a palladium catalyst.

For the conditions for the preparation of the alkylzinc chloride from the alkylmagnesium bromide, the process can be performed using zinc chloride, for example within a temperature range of 0°C to 25°C in a solvent such as THF or diethyl ether.

Examples of palladium catalysts in step F2 include [1,1'-bis(diphenylphosphino)ferrocene]dichloropalladium(II), tetrakis(triphenylphosphine)palladium and dichlorobis(triphenylphosphine)palladium. The reaction temperature can be, for example, within a range of 20°C to 60°C.

A more specific manufacturing method with step F2 that is preferred because of the ease of synthesis, furthermore, is presented in the following reaction scheme. 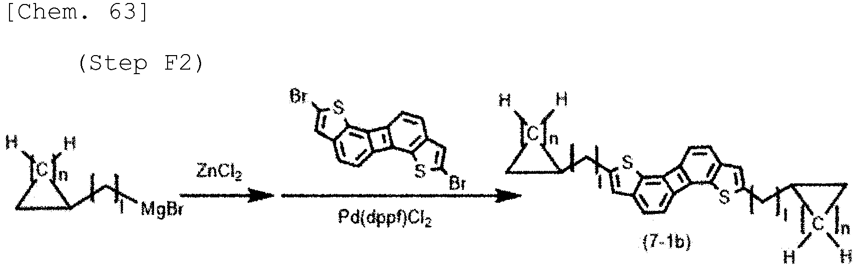 (In the formula, n and l are synonymous with n and l indicated in formula (2).)

Moreover, as another example of a method for manufacturing the aromatic compound according to the present invention, an aromatic compound (7-1d) in which X⁸ and X⁹ in formula (7-1) are sulfur atoms, Y⁵ and Y⁶ are CH, R³⁸ is a group represented by formula (2), R³⁹, R⁴⁰, R⁴² and R⁴³ are hydrogen atoms, each of m and k in formula (2) is 0, and Z¹ to Z⁵ are hydrogen atoms, for example, can be manufactured through the steps of E1, described above, and D2 and F3, described below.

(Step D2); A step of turning a dithienobiphenylene derivative (7-1a) into a monolithium salt using butyllithium and then synthesizing a monobromodithienobiphenylene derivative (7-1c) using a brominating agent.

(Step F3); A step of producing the aromatic compound (7-1d) by allowing a zinc chloride derived from a magnesium bromide to react with the monobromodithienobiphenylene derivative (7-1c) obtained in step D2 in the presence of a palladium catalyst.

The details of each step are presented below.

### (Step D2)

The step D2 is a step of turning a dithienobiphenylene derivative (7-1a) into a monolithium salt using 1 equivalent or more of butyllithium and allowing it to react with a brominating agent to produce a dibromo derivative.

For the conditions for the preparation of the monolithium salt, the process can be performed, for example, within a temperature range of -80°C to 30°C in a solvent such as THF or diethyl ether using 1.0 to 5.0 equivalents of n-butyllithium or tert-butyllithium.

The brominating agent can be, for example, tetrabromomethane or 1,2-dibromotetrachloroethane.

### (Step F3)

The step F3 is a method of producing the aromatic compound (7-1d) by allowing a zinc chloride derived from a magnesium bromide to react with the monobromodithienobiphenylene derivative (7-1c) obtained in step D2 in the presence of a palladium catalyst.

For the method for preparing the zinc chloride, the process can be performed using zinc chloride, for example within a temperature range of 0°C to 25°C in a solvent such as THF or diethyl ether.

Examples of palladium catalysts in step F3 include [1,1'-bis(diphenylphosphino)ferrocene]dichloropalladium(II), tetrakis(triphenylphosphine)palladium and dichlorobis(triphenylphosphine)palladium. The reaction temperature can be, for example, within a range of 20°C to 60°C.

A more specific manufacturing method with step D2 and step F3 that is preferred because of the ease of synthesis, furthermore, is presented in the following reaction scheme. 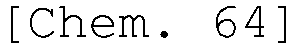 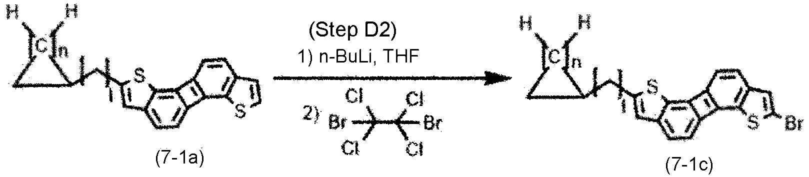 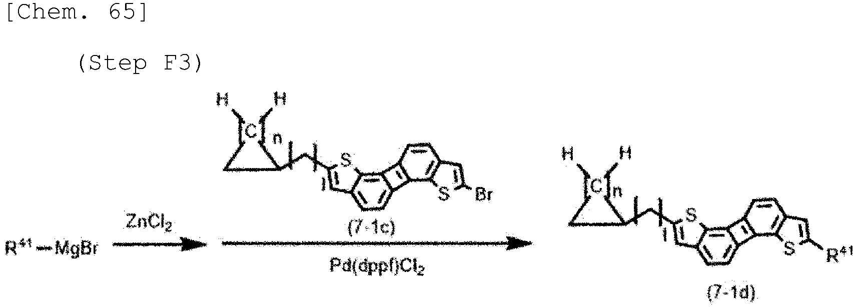 (In the formulae, n and l are synonymous with n and l indicated in formula (2), and R⁴¹ is synonymous with R⁴¹ indicated in formula (7-1).)

### [2. Solution for Forming an Organic Semiconductor Layer]

By dissolving the compound of the present invention in a solvent, the compound can be turned into a solution comprising it for forming an organic semiconductor layer. The solvent may be any solvent as long as the aromatic compound indicated by formula (1-I) or formula (1-II) can be dissolved in it. Of such solvents, organic solvents having a boiling point at atmospheric pressure of 100°C or above are particularly preferred because with such a solvent, an appropriate solvent drying rate can be achieved during the formation of the organic semiconductor layer.

There are no specific restrictions on solvents that can be used in the solution according to the present invention for forming an organic semiconductor layer. Examples include aromatic hydrocarbons, such as toluene, mesitylene, o-xylene, isopropylbenzene, pentylbenzene, cyclohexylbenzene, 1,2,4-trimethylbenzene, tetralin and indan; aromatic ethers, such as anisole, 2-methylanisole, 3-methylanisole, 2,3-dimethylanisole, 3,4-dimethylanisole, 2,6-dimethylanisole, ethyl phenyl ether, butyl phenyl ether, 1,2-methylenedioxybenzene and 1,2-ethylenedioxybenzene; aromatic halogen compounds, such as chlorobenzene, 1,2-dichlorobenzene, 1,3-dichlorobenzene, 1,4-dichlorobenzene, 1,2-difluorobenzene, 1,3-difluorobenzene and 1,4-difluorobenzene; heteroaromatics, such as thiophene, 3-chlorothiophene, 2-chlorothiophene, 3-methylthiophene, 2-methylthiophene, benzothiophene, 2-methylbenzothiophene, 2,3-dihydrobenzothiophene, furan, 3-methylfuran, 2-methylfuran, 2,5-dimethylfuran, benzofuran, 2-methylbenzofuran, 2,3-dihydrobenzofuran, thiazole, oxazole, benzothiazole, benzoxazole and pyridine; saturated hydrocarbons, such as hexane, cyclohexane, heptane, octane, nonane, decane, undecane, dodecane and decalin; glycols, such as dipropylene glycol dimethyl ether, dipropylene glycol diacetate, dipropylene glycol methyl-n-propyl ether, dipropylene glycol methyl ether acetate, 1,4-butanediol diacetate, 1,3-butylene glycol diacetate, 1,3-butylene glycol diacetate, 1,6-hexanediol diacetate, ethylene glycol monomethyl ether acetate, propylene glycol monomethyl ether acetate, ethylene glycol monobutyl ether acetate, diethylene glycol monoethyl ether acetate and diethylene glycol monobutyl ether acetate; esters, such as dimethyl phthalate, diethyl phthalate, dimethyl terephthalate, phenyl acetate, cyclohexanol acetate, 3-methoxybutyl acetate, tetrahydrofurfuryl acetate, tetrahydrofurfuryl propionate and γ-butyrolactone; and cyclic ethers, such as THF and 2-methoxymethyltetrahydrofuran. Of these, it is particularly preferred that the solvent be toluene, o-xylene, mesitylene, 1,2,4-trimethylbenzene, tetralin, indan, octane, nonane, decane, anisole, 2-methylanisole, 3-methylanisole, 2,3-dimethylanisole, 3,4-dimethylanisole, 2,6-dimethylanisole, ethyl phenyl ether, butyl phenyl ether, 1,2-methylenedioxybenzene, 1,2-ethylenedioxybenzene, chlorobenzene, 1,2-dichlorobenzene, 1,3-dichlorobenzene, 1,4-dichlorobenzene, 3-methylthiophene or benzothiazole because they have an adequate drying rate. More preferably, the solvent is toluene, o-xylene, mesitylene, tetralin, indan, octane, nonane, decane, anisole, 2-methylanisole, 3-methylanisole, 2,3-dimethylanisole, 3,4-dimethylanisole or 2,6-dimethylanisole.

It should be noted that the solvent used in the solution for forming an organic semiconductor layer may be one solvent used alone, or two or more solvents differing in characteristics such as boiling point, polarity and solubility parameters may be mixed and used.

For the temperature during the mixing and dissolution of the aromatic compound indicated by formula (1-I) or formula (1-II) in the solvent, it is preferred that the process be performed within a temperature range of 0°C to 80°C for the purpose of accelerating dissolution. More preferably, the process is performed within a temperature range of 10°C to 60°C.

The duration for which the aromatic compound indicated by formula (1-I) or formula (1-II) is dissolved and mixed in the solvent, furthermore, is preferably set to 1 minute to 1 hour so that a uniform solution is obtained.

When the concentration of the aromatic compound indicated by formula (1-I) or formula (1-II) in the solution for forming an organic semiconductor layer falls within the range of 0.1% to 10.0% by weight, the solution is easier to handle and better in terms of efficiency in forming an organic semiconductor layer. When the viscosity of the solution for forming an organic semiconductor layer falls within the range of 0.3 to 10 mPa·s, furthermore, the solution exhibits better spreadability.

It should be noted that this solution can be prepared at relatively low temperatures because the aromatic compound itself has adequate aggregability, and is also suitable for application to the manufacture of organic thin films by coating by virtue of its resistance to oxidation. In other words, the coating step can be simplified because there is no need to eliminate air from the atmosphere. In this solution, furthermore, a polymer can be allowed to present as a binder. Examples of such polymers include polystyrene, poly(α-methylstyrene), poly(4-methylstyrene), poly(1-vinylnaphthalene), poly(2-vinylnaphthalene), poly(styrene-block-butadiene-block-styrene), poly(styrene-block-isoprene-block-styrene), poly(vinyltoluene), poly(styrene-co-2,4-dimethylstyrene), poly(chlorostyrene), poly(styrene-co-α-methylstyrene), poly(styrene-co-butadiene), poly(ethylene-co-norbornene), polyphenylene ether, polycarbonate, polycarbazole, polytriarylamine, poly(9,9-dioctylfluorene-co-dimethyltriarylamine), poly(N-vinylcarbazole), polymethyl methacrylate, poly(styrene-co-methyl methacrylate), polyethyl methacrylate, poly-n-propyl methacrylate, polyisopropyl methacrylate, poly-n-butyl methacrylate, polyphenyl methacrylate, polymethyl acrylate, polyethyl acrylate, poly-n-propyl acrylate, polar cyclic polyolefins, polysulfones, acrylonitrile-styrene copolymers and methyl methacrylate-styrene copolymers. Of these, polymers such as polystyrene, poly(α-methylstyrene), poly(ethylene-co-norbornene) and polymethyl methacrylate are particularly preferred. The concentration of such a polymer binder is preferably from 0.001% to 10.0% by weight for adequate viscosity of the solution.

The glass transition temperature (Tg) of the polymer binder is preferably 105°C or above because this is more suitable for covering process temperatures during the manufacture of electronic devices. More preferably, the Tg is 120°C or above, particularly preferably 150°C or above.

The molecular weight of the polymer, furthermore, is preferably from 5,000 to 1,000,000 because this is suitable for obtaining an organic thin film transistor having higher carrier mobility. More preferably, the molecular weight is from 10,000 to 500,000, particularly preferably 20,000 to 100,000. It should be noted that in the present invention, the molecular weight of a polymer refers to a polystyrene-equivalent weight-average molecular weight (Mw).

The polymer is one that has effects as a general polymer binder and that improves the film-forming properties of the resulting organic semiconductor layer. Electrically insulating polymers and semiconducting polymers can also be used.

A polar cyclic polyolefin used as a polymer binder is, more specifically, more preferably a polymer indicated by formula (9) below. 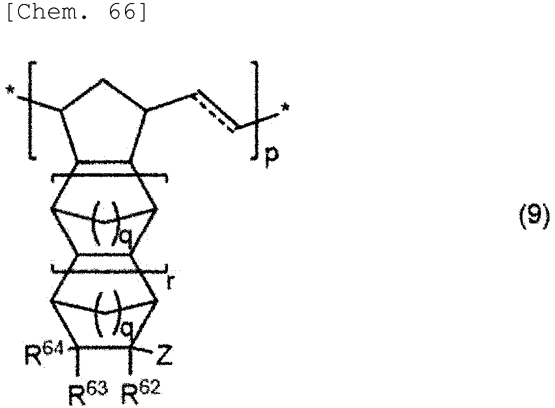 (In the formula, R⁶² to R⁶⁴ each independently denote one selected from the group consisting of a hydrogen atom, a halogen atom, a C1 to C20 alkyl group, a C6 to C20 aryl group, a C2 to C20 alkyloxycarbonyl group, a C7 to C20 aryloxycarbonyl group, a cyano group, a nitro group, a C1 to C20 alkoxy group, a C6 to C20 aryloxy group, a hydroxyl group, an amino group and a C1 to C20 alkylamino group. Z denotes one selected from the group consisting of a halogen atom, a C2 to C20 alkyloxycarbonyl group, a C7 to C20 aryloxycarbonyl group, a cyano group, a nitro group, a C1 to C20 alkoxy group, a C6 to C20 aryloxy group, a hydroxyl group, an amino group and a C1 to C20 alkylamino group. p denotes an integer of 20 to 5,000, and q and r each independently denote an integer of 0 to 2. The bond represented by a solid line and a dotted line indicates that it is a single bond or a double bond.)

R⁶² to R⁶⁴ in formula (9) each independently denote one selected from the group consisting of a hydrogen atom, a halogen atom, a C1 to C20 alkyl group, a C6 to C20 aryl group, a C2 to C20 alkyloxycarbonyl group, a C7 to C20 aryloxycarbonyl group, a cyano group, a nitro group, a C1 to C20 alkoxy group, a C6 to C20 aryloxy group, a hydroxyl group, an amino group and a C1 to C20 alkylamino group. A hydrogen atom and a C1 to C20 alkyl group are preferred for high heat resistance of this polymer.

Examples of C1 to C20 alkyl groups at R⁶² to R⁶⁴ include linear-chain or branched alkyl groups, such as a methyl group, an ethyl group, an n-propyl group, an n-butyl group, an isobutyl group, a sec-butyl group and an n-pentyl group. Examples of C6 to C20 aryl groups include a phenyl group, a p-tolyl group, a p-(n-hexyl)phenyl group, a p-(n-octyl)phenyl group and a p-(2-ethylhexyl)phenyl group. Examples of C2 to C20 alkyloxycarbonyl groups include a methyloxycarbonyl group, an ethyloxycarbonyl group and an n-propyloxycarbonyl group. Examples of C7 to C20 aryloxycarbonyl groups include a phenoxycarbonyl group and a 4-methylphenoxycarbonyl group. Examples of C1 to C20 alkoxy groups include a methoxy group, an ethoxy group and an n-propoxy group. Examples of C6 to C20 aryloxy groups include a phenoxy group and a 4-methylphenoxy. Examples of C1 to C20 alkylamino groups include a methylamino group, an ethylamino group and an n-propylamino group. Of these, furthermore, it is particularly preferred, for high heat resistance of this polymer, that substituent R⁶² be a methyl group, an ethyl group or an n-propyl group, and that substituents R⁶³ and R⁶⁴ be hydrogen atoms.

Z in formula (9) denotes one selected from the group consisting of a halogen atom, a C2 to C20 alkyloxycarbonyl group, a C7 to C20 aryloxycarbonyl group, a cyano group, a nitro group, a C1 to C20 alkoxy group, a C6 to C20 aryloxy group, a hydroxyl group, an amino group and a C1 to C20 alkylamino group.

Examples of C2 to C20 alkyloxycarbonyl groups at substituent Z include a methoxycarbonyl group, an ethoxycarbonyl group, an n-propoxycarbonyl group, an n-butoxycarbonyl group, an n-hexyloxycarbonyl group and a cyclohexyloxycarbonyl group, and examples of C7 to C20 aryloxycarbonyl groups include a phenoxycarbonyl group, a 4-methylphenoxycarbonyl group, a 2,4-dimethylphenoxycarbonyl group and a 4-ethylphenoxycarbonyl group. Examples of C1 to C20 alkoxy groups include a methoxy group and an ethoxy group. Examples of C6 to C20 aryloxy groups include a phenoxy group and a 4-methylphenoxy. Examples of C1 to C20 alkylamino groups include a methylamino group, an ethylamino group and an n-propylamino group. Z is preferably a C2 to C20 alkyloxycarbonyl group for high solubility and high heat resistance of this polymer.

p denotes an integer of 20 to 5,000. Preferably, p is from 40 to 2,000 because this is suitable for obtaining an organic thin film transistor having higher carrier mobility. q denotes an integer of 0 to 2, and preferably is 1. r denotes an integer of 0 to 2, and preferably is 0 or 1, more preferably 0.

The bond represented by a solid line and a dotted line indicates that it is a single bond or a double bond. Preferably, it is a single bond for thermal stability.

For a polysulfone used as a polymer binder, there are no specific restrictions as long as it has a polysulfone structure. More specific examples include polysulfones indicated by polysulfones 1 to 5 below. 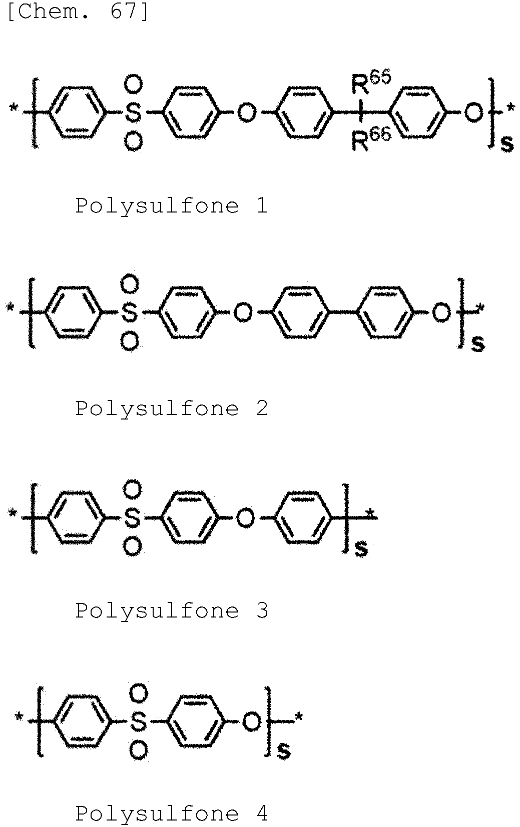 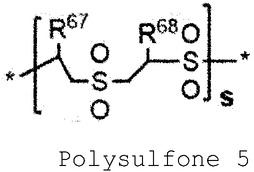 (In the formulae, substituents R⁶⁵ to R⁶⁸ each independently denote a C1 to C20 alkyl group, and s denotes an integer of 10 to 20,000.)

Examples of C1 to C20 alkyl groups at substituents R⁶⁵ to R⁶⁸ include linear-chain or branched alkyl groups, such as a methyl group, an ethyl group, an n-propyl group, an n-butyl group, an isobutyl group, an n-pentyl group, an n-hexyl group, an isohexyl group, an n-heptyl group, an n-octyl group, an n-nonyl group, an n-decyl group, an n-dodecyl group, an n-tetradecyl group, an n-octadecyl group, a 2-ethylhexyl group, a 3-ethylheptyl group, a 3-ethyldecyl group and a 2-hexyldecyl group.

s denotes an integer of 10 to 20,000, and preferably is an integer of 10 to 10,000.

An acrylonitrile-styrene copolymer used as a polymer binder is a copolymer of acrylonitrile and styrene in any proportions. An acrylonitrile-styrene copolymer preferably has a ratio of 10:90 to 50:50 as a ratio by weight between acrylonitrile and styrene because in that case the reliability of the finally obtained organic thin film transistor is improved; for example, the transistor exhibits good electrical properties and experiences a smaller change in threshold voltage when exposed to bias stress. More preferably, the ratio is from 20:80 to 40:60.

A methyl methacrylate-styrene copolymer used as a polymer binder is a copolymer of methyl methacrylate and styrene in any proportions. A methyl methacrylate-styrene copolymer preferably has a ratio of 1:99 to 90:10 as a molar ratio between methyl acrylate and styrene because in that case the reliability of the finally obtained organic thin film transistor is improved; for example, the transistor exhibits good electrical properties and experiences a smaller change in threshold voltage when exposed to bias stress. More preferably, the ratio is from 1:99 to 70:30.

A polymer used as a polymer binder can be a polymer whose surface energy has been adjusted using a surface treatment agent. The surface treatment agent can be a silane coupling agent, and specific examples thereof include 1,1,1,3,3,3-hexamethyldisilazane, phenyltrimethoxysilane, octyltrichlorosilane, β-phenethyltrichlorosilane and β-phenethyltrimethoxysilane.

The polymer may be one polymer used alone, or two or more polymers may be used as a mixture. It is, furthermore, also possible to mix and use polymers with different molecular weights.

### [3. Organic Semiconductor Layer]

For the coating method used when an organic semiconductor layer is formed using a solution comprising the compound of the present invention for forming an organic semiconductor layer, there are no specific restrictions as long as an organic semiconductor layer can be formed by the method. Examples include simple coating methods, such as spin coating, drop casting, dip coating and cast coating; and printing methods, such as a dispenser, inkjet printing, slit coating, blade coating, flexography, screen printing, gravure printing and offset printing. Of these, it is particularly preferred that the method be spin coating, drop casting or inkjet printing because in that case the solution can be easily and efficiently processed into an organic semiconductor layer.

By applying a solution according to the present invention for forming an organic semiconductor layer to form a coating of the solution for forming an organic semiconductor layer and then removing the solvent from the coating by drying, it is possible to form an organic semiconductor layer comprising the compound of the present invention in which the solution for forming an organic semiconductor layer is used.

When the solvent is removed by drying from the coating of the solution for forming an organic semiconductor layer, there are no specific restrictions on the drying conditions. For example, the drying and removal of the solvent can be performed under atmospheric pressure or under reduced pressure.

There are no specific restrictions on the temperature at which the solvent is removed by drying from the coating of the solution for forming an organic semiconductor layer. Preferably, however, the drying and removal process is performed within a temperature range of 10°C to 150°C because in that case the solvent can be efficiently removed by drying from the applied organic semiconductor layer, allowing for efficient formation of the organic semiconductor layer.

When the solvent is removed by drying from the coating of the solution for forming an organic semiconductor layer, the crystal growth of the aromatic compound indicated by formula (1-I) or formula (1-II) can be controlled by adjusting the vaporization rate of the solvent to be removed.

There are no restrictions on the thickness of the organic semiconductor layer formed by the solution according to the present invention for forming an organic semiconductor layer. Preferably, the thickness falls within the range of 1 nm to 1 µm because in that case good carrier movement is achieved. More preferably, the thickness falls within the range of 10 nm to 300 nm.

The resulting organic semiconductor layer, furthermore, may be subjected to annealing treatment at 40°C to 180°C after the organic semiconductor layer is formed.

The organic semiconductor layer formed from the solution according to the present invention for forming an organic semiconductor layer can be used in the form of an organic semiconductor device that includes this organic semiconductor layer, in particular an organic thin film transistor comprising the compound of the present invention that includes this organic semiconductor layer.

### [4. Organic Thin Film Transistor]

An organic thin film transistor can be obtained by stacking an organic semiconductor layer, with a source electrode and a drain electrode attached thereto, and a gate electrode on a substrate with an insulating layer interposed therebetween. By using, as the organic semiconductor layer, an organic semiconductor layer formed using a solution according to the present invention for forming an organic semiconductor layer, it is possible to make the organic thin film transistor one that exhibits excellent semiconductor properties and electric properties.

In Fig. 1, typical structures of organic thin film transistors categorized by cross-sectional shape are illustrated. In these drawings, 1001 is a bottom gate-top contact organic thin film transistor, 1002 is a bottom gate-bottom contact organic thin film transistor, 1003 is a top gate-top contact organic thin film transistor, and 1004 is a top gate-bottom contact organic thin film transistor. 1 indicates an organic semiconductor layer, 2 indicates a substrate, 3 indicates a gate electrode, 4 indicates a gate insulating layer, 5 indicates a source electrode, and 6 indicates a drain electrode. An organic semiconductor layer formed from a solution according to the present invention for forming an organic semiconductor layer can be applied to any type of organic thin film transistor.

For the substrate, there are no specific restrictions. Examples include plastic substrates, such as polyethylene terephthalate, polyethylene naphthalate, polymethyl methacrylate, polymethyl acrylate, polyethylene, polypropylene, polystyrene, cyclic polyolefin, fluorinated cyclic polyolefin, polyimide, polycarbonate, polyvinyl phenol, polyvinyl alcohol, poly(diisopropyl fumarate), poly(diethyl fumarate), poly(diisopropyl maleate), polyethersulfone, polyphenylene sulfide and cellulose triacetate; inorganic material substrates, such as glass, quartz, aluminum oxide, silicon, highly doped silicon, silicon oxide, tantalum dioxide, tantalum pentoxide and indium tin oxide; and metal substrates, such as gold, copper, chromium, titanium and aluminum. It should be noted that when highly doped silicon is used as the substrate, this substrate can also function as the gate electrode.

For the material for the gate electrode, there are no specific restrictions. Examples include inorganic materials, such as aluminum, gold, silver, copper, highly doped silicon, tin oxides, indium oxide, indium tin oxide, chromium, titanium, tantalum, graphene and carbon nanotubes; and organic materials, such as doped conductive polymers (e.g., PEDOT-PSS).

The inorganic materials listed above, furthermore, can also be used in the form of metal nanoparticle ink without causing problems. The solvent in that case is preferably a polar solvent, such as water, methanol, ethanol, 2-propanol, 1-butanol or 2-butanol; a C6 to C14 aliphatic hydrocarbon solvent, such as hexane, heptane, octane, decane, dodecane or tetradecane; or a C7 to C14 aromatic hydrocarbon solvent, such as toluene, xylene, mesitylene, ethylbenzene, pentylbenzene, hexylbenzene, octylbenzene, cyclohexylbenzene, tetralin, indan, anisole, 1,2-dimethoxybenzene, 1,3-dimethoxybenzene, 1,2-dimethylanisole, 2,3-dimethylanisole or 3,4-dimethylanisole, for adequate dispersibility. After application, the nanoparticle ink is preferably subjected to annealing treatment within a temperature range of 80°C to 200°C for the improvement of conductivity.

For the material for the gate insulating layer, there are no specific restrictions. Examples include inorganic materials, such as silicon oxide, silicon nitride, aluminum oxide, aluminum nitride, titanium oxide, tantalum dioxide, tantalum pentoxide, indium tin oxide, tin oxide, vanadium oxide, barium titanate and bismuth titanate; and the polymeric insulating materials of polymethyl methacrylate, polymethyl acrylate, polyimides, polyamic acid polycarbonate, polyvinyl phenol, polyvinyl alcohol, poly(diisopropyl fumarate), poly(diethyl fumarate), polyethylene terephthalate, polyethylene naphthalate, polyethyl cinnamate, polymethyl cinnamate, polyethyl crotonate, polyethersulfone, polypropylene-co-1-butene, polyisobutylene, polypropylene, polycyclopentane, polycyclohexane, polycyclohexane-ethylene copolymers, polyfluorinated cyclopentane, polyfluorinated cyclohexane, polyfluorinated cyclohexane-ethylene copolymers, BCB resin (trade name, CYCLOTENE; manufactured by The Dow Chemical Company), Cytop^{®}, Teflon^{®} and Parylene^{®}s, such as Parylene C. Of these, it is particularly preferred that the material for the gate insulating layer be a polymeric insulating material for which coating can be used (a polymeric gate insulating layer) because in that case the production process is easy and convenient.

For the solvent used to dissolve the polymeric insulating material, there are no specific restrictions. Examples include C6 to C14 aliphatic hydrocarbon solvents, such as hexane, heptane, octane, decane, dodecane and tetradecane; ether solvents, such as THF, 1,2-dimethoxyethane and dioxane; alcohol solvents, such as ethanol, isopropyl alcohol, 1-butanol, 2-butanol, 2-ethylhexanol and tetrahydrofurfuryl alcohol; ketone solvents, such as acetone, methyl ethyl ketone, diethyl ketone, diisopropyl ketone and acetophenone; ester solvents, such as ethyl acetate, γ-butyrolactone, cyclohexanol acetate, 3-methoxybutyl acetate, tetrahydrofurfuryl acetate and tetrahydrofurfuryl propionate; amide solvents, such as DMF and NMP; glycol solvents, such as dipropylene glycol dimethyl ether, dipropylene glycol diacetate, dipropylene glycol methyl-n-propyl ether, dipropylene glycol methyl ether acetate, 1,4-butanediol diacetate, 1,3-butylene glycol diacetate, 1,6-hexanediol diacetate, ethylene glycol monomethyl ether acetate, propylene glycol monomethyl ether acetate, ethylene glycol monobutyl ether acetate, diethylene glycol monoethyl ether acetate and diethylene glycol monobutyl ether acetate; and fluorinated solvents, such as perfluorohexane, perfluorooctane, 2-(pentafluoroethyl)hexane and 3-(pentafluoroethyl)heptane.

The concentration of the polymeric insulating material when dissolved in the solvent is, for example, from 0.1% to 10.0% by weight at temperatures of 20°C to 40°C. There are no restrictions on the thickness of the insulating layer obtained at that concentration. From the viewpoint of insulation resistance, it is preferred that the thickness be from 100 nm to 1 µm, more preferably from 150 nm to 900 nm.

The gate insulating layer can also be a gate insulating layer whose surface has undergone modification treatment with a silane, such as octadecyltrichlorosilane, decyltrichlorosilane, decyltrimethoxysilane, octyltrichlorosilane, octadecyltrimethoxysilane, β-phenethyltrichlorosilane, β-phenethyltrimethoxysilane, phenyltrichlorosilane or phenyltrimethoxysilane; a phosphonic acid, such as octadecylphosphonic acid, decylphosphonic acid or octylphosphonic acid; or a silylamine, such as hexamethyldisilazane. In general, performing surface treatment of a gate insulating layer increases the crystal particle size of the organic semiconductor material while also improving its molecular orientation. As a result, the advantageous outcomes of improvements in carrier mobility and current on-off ratio and a reduction in threshold voltage are achieved.

For the materials for the source electrode and the drain electrode, there are no specific restrictions. Materials that can be used are the same as those for the gate electrode, and the materials may be the same as or different from that for the gate electrode. It is also possible to stack different materials. To increase carrier injection efficiency, furthermore, the surfaces of the source electrode and the drain electrode may be subjected to surface treatment. Examples of surface treatment agents usable for the surface treatment include benzenethiol, pentafluorobenzenethiol, 4-fluorobenzenetiol and 4-methoxybenzenethiol.

The organic thin film transistor according to the present invention preferably has a carrier mobility of 0.10 cm²/V·sec or more for rapid operation.

The organic thin film transistor according to the present invention can be utilized in transistor organic semiconductor layer applications, for example in electronic paper, OLED displays, liquid crystal displays, IC tags (RFID tags), pressure sensors and biosensors; or as an electronic material, such as an OLED display material; an organic semiconductor laser material; an organic thin film solar cell material; a photonic crystal material; or a semiconductor material for imaging elements. Since the aromatic compound indicated by formula (1-I) or formula (1-II) forms a crystalline thin film, furthermore, the organic thin film transistor is preferably used in organic thin film transistor semiconductor layer applications.

### [Recapitulation]

A first aspect of the present invention is an aromatic compound indicated by either formula (1-I) or (1-II) above.

A second aspect of the present invention is the compound according to the first aspect, wherein of R¹ to R⁶, only one or only both of R¹ and R² are groups indicated by formula (2) above.

A third aspect of the present invention is the aromatic compound according to the first aspect or second aspect, wherein the aromatic compound indicated by formula (1-I) or (1-II) is a compound indicated by one selected from the group consisting of formulae (3-1) to (3-6) above.

A fourth aspect of the present invention is the aromatic compound according to the third aspect, wherein the aromatic compound indicated by formula (1-I) or (1-II) is a compound indicated by formula (3-1) or formula (3-2).

A fifth aspect of the present invention is the aromatic compound according to the third aspect or fourth aspect, wherein formula (4-2) above is formula (4-3) above.

A sixth aspect of the present invention is the aromatic compound according to the third aspect or fourth aspect, wherein formula (4-2) above is formula (4-4) above.

A seventh aspect of the present invention is the aromatic compound according to the first aspect or second aspect, wherein the aromatic compound indicated by formula (1-I) or (1-II) is a compound indicated by formula (5) or (5-2) above.

An eighth aspect of the present invention is the aromatic compound according to the seventh aspect, wherein formula (6-2) above is formula (6-3) above.

A ninth aspect of the present invention is the aromatic compound according to the seventh aspect, wherein formula (6-2) above is formula (6-4) above.

A tenth aspect of the present invention is the aromatic compound according to the first aspect or second aspect, wherein the aromatic compound indicated by formula (1-I) or (1-II) is a compound indicated by one selected from the group consisting of formulae (7-1) to (7-6) above.

An eleventh aspect of the present invention is the aromatic compound according to the tenth aspect, wherein each of R³⁸ and R⁴¹ is independently one selected from the group consisting of a group represented by formula (2) above, a hydrogen atom and a fluorine atom, and R³⁹, R⁴⁰, R⁴² to R⁴⁵, R⁷¹ and R⁷² are hydrogen atoms.

A twelfth aspect of the present invention is the aromatic compound according to the tenth aspect or eleventh aspect, wherein R³⁸ and R⁴¹ are groups represented by formula (2) above and R³⁹, R⁴⁰, R⁴² to R⁴⁵, R⁷¹ and R⁷² are hydrogen atoms.

A thirteenth aspect of the present invention is a solution for forming an organic semiconductor layer, the solution comprising the aromatic compound according to any of the first to twelfth aspects.

A thirteenth aspect of the present invention is an organic semiconductor layer comprising the aromatic compound according to any of the first to twelfth aspects.

A thirteenth aspect of the present invention is an organic thin film transistor comprising the aromatic compound according to any one of the first to twelfth aspects.

### EXAMPLES

The present invention will now be described in further detail with examples, but the present invention is not limited to these examples.

The identification of the products was performed using ¹H NMR spectrometry and liquid chromatography-mass spectrometry (LCMS).

### <¹H NMR Spectrometry>

Instrument; Manufactured by JEOL Ltd., (trade name) Delta V5 (400 MHz)
Measurement temperature; 23°C (if no temperature is specified)

### <Liquid Chromatography-Mass Spectrometry (LCMS)>

Instrument; Bruker Daltonics Inc., (trade name) microTOF focus
MS ionization; Atmospheric pressure chemical ionization (APCI)
LC conditions; The conditions specified in the Liquid Chromatography section below

The confirmation of the progress of the reactions, for example, was performed using thin-layer chromatography, gas chromatography (GC) and liquid chromatography (LC). The measurement of the purity of the aromatic compounds was also performed using liquid chromatography.

### <Thin-Layer Chromatography>

Merck KGaA's PLC Silica Gel 60 F254 0.5 mm for thin-layer chromatography was used, and hexane or/and toluene was used as the developing solvent.

### <Gas Chromatography>

Instrument; Manufactured by Shimadzu Corporation, (trade name) GC2014
Column; Manufactured by RESTEK Corporation, (trade name) Rxi-1HT, 30 m

### <Liquid Chromatography>

Instrument; Manufactured by Agilent Technologies, Inc., model; 1260 Infinity II
Column; Manufactured by Tosoh Corporation, (trade name) ODS-100V, 5 µm, 4.6 mm × 250 mm
Column temperature; 33°C
Eluent; Dichloromethane:acetonitrile = 2:8 (ratio by volume)
Flow rate; 1.0 ml/minute

The purification of the aromatic compounds was performed using a recycling preparative HPLC system.

### <Recycling Preparative HPLC>

Instrument; Manufactured by Japan Analytical Industry Co., Ltd., model; LC-9160II NEXT
Solvent; Tetrahydrofuran
Flow rate; 10 ml/minute

The measurement of the melting points of the aromatic compounds was performed using a DSC (differential scanning calorimeter).

### <DSC Measurement>

Instrument; Manufactured by SII NanoTechnology Inc., model; DSC6220
Temperature elevation/lowering rate; 10°C/min
Scan range; -10°C to 300°C

The evaluation of the transmission characteristics of the aromatic compounds was performed using a semiconductor parameter analyzer.

### <Transmission Characteristics Measurement>

Instrument; Manufactured by Keithley Instruments, Inc., model; 4200A-SCS
Drain voltage; -20 V
Gate voltage; 10 V to -20 V.

### Synthesis Example 1 Synthesis of (2-Bromoethyl)cyclohexane

In a nitrogen atmosphere, 5.03 g (39.2 mmol) of 2-cyclohexylethanol (Tokyo Chemical Industry Co., Ltd.) and 43 ml of toluene (FUJIFILM Wako Pure Chemical Corporation, dehydration grade) were added to a 200-ml two-neck flask. The solution was stirred for 20 minutes at room temperature with 1.70 ml (17.9 mmol) of phosphorus tribromide (FUJIFILM Wako Pure Chemical Corporation) added thereto, and the resulting mixture was stirred for 3 hours at 100°C. The reaction solution was poured into ice and neutralized with a saturated aqueous solution of sodium hydrogencarbonate, then toluene was added, and phase separation was performed. The organic phase was washed with water, dried over anhydrous sodium sulfate and concentrated under reduced pressure, giving 5.31 g of a colorless liquid of (2-bromoethyl)cyclohexane (yield, 68%).

¹H NMR (CDCl₃): δ = 3.44 (t, J = 7.3 Hz, 2H), 1.79-1.64 (m, 7H), 1.52-1.41 (m, 1H), 1.30-1.09 (m, 3H), 0.96-0.86 (m, 2H).

### ((2-Bromoethyl)cyclohexane)

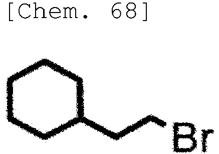

### Synthesis Example 2 Synthesis of 2-(2-Cyclohexylethyl)-4,4,5,5-tetramethyl-1,3,2-dioxaborolane

In a nitrogen atmosphere, 809 mg (1.40 mmol) of Xantphos (Tokyo Chemical Industry Co., Ltd.), 8.49 g (33.4 mmol) of bis(pinacolato)diboron (Tokyo Chemical Industry Co., Ltd.), 140 mg (1.42 mmol) of copper(I) chloride (FUJIFILM Wako Pure Chemical Corporation), 4.06 g (36.2 mmol) of potassium tert-butoxide (Tokyo Chemical Industry Co., Ltd.) and 20 ml of THF (FUJIFILM Wako Pure Chemical Corporation, dehydration grade) were added to a 200-ml Schlenk reaction vessel, and the resulting mixture was stirred for 30 minutes at room temperature. 19 mL of a solution of 5.31 g (27.8 mmol) of the (2-bromoethyl)cyclohexane synthesized in Synthesis Example 1 in THF (FUJIFILM Wako Pure Chemical Corporation, dehydration grade) was added with cooling on ice, and the resulting mixture was stirred for 25 minutes at 0°C and for 4 hours at room temperature. The reaction mixture was cooled on ice, water was added, then toluene was added, and phase separation was performed. The organic phase was washed with water, dried over anhydrous sodium sulfate and concentrated under reduced pressure. The resulting residue was purified by silica gel column chromatography (solvent; toluene), giving 5.46 g of a colorless liquid of 2-(2-cyclohexylethyl)-4,4,5,5-tetramethyl-1,3,2-dioxaborolane (yield, 81%).

¹H NMR (CDCl₃): δ = 1.72-1.59 (m, 5H), 1.32-1.26 (m, 2H), 1.25 (s, 12H), 1.22-1.08 (m, 4H), 0.88-0.74 (m, 4H).

### (2-(2-Cyclohexylethyl)-4,4,5,5-tetramethyl-1,3,2-dioxaborolane)

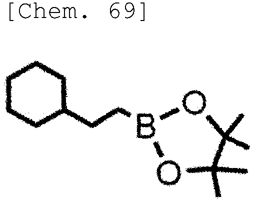

### Synthesis Example 3 Synthesis of 2-Hexylanthra[1,2-b:5,6-b']dithiophene

In a nitrogen atmosphere, 716 mg (purity, 65%; 1.26 mmol) of 2-bromoanthra[1,2-b:5,6-b']dithiophene synthesized by the method described in WO 2021/177417 (compound 22 in the publication), 67.0 mg (0.298 mmol) of palladium(II) acetate (FUJIFILM Wako Pure Chemical Corporation), 278 mg (0.596 mmol) of Ruphos (Tokyo Chemical Industry Co., Ltd.) and 487 mg (3.64 mmol) of potassium tert-butoxide (Tokyo Chemical Industry Co., Ltd.) and 509 mg (3.91 mmol) of hexylboronic acid (Tokyo Chemical Industry Co., Ltd.) were added to a 200-mL Schlenk tube. 45 mL of toluene (FUJIFILM Wako Pure Chemical Corporation, dehydration grade) and 3.0 mL of water were added to the tube, and the resulting mixture was stirred for 2.5 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane), giving 100 mg of a yellow solid of 2-hexylanthra[1,2-b:5,6-b']dithiophene (yield, 49%).

¹H NMR (CDCl₃): δ = 8.70 (s, 1H), 8.62 (s, 1H), 7.89 (d, J = 6.6 Hz, 1H), 7.87 (d, J = 6.4 Hz, 1H), 7.82 (d, J = 8.7 Hz, 1H), 7.73 (d, J = 8.7 Hz, 1H), 7.54 (d, J = 5.2 Hz, 1H), 7.49 (d, J = 5.1 Hz, 1H), 7.16 (s, 1H), 3.02 (t, J = 8.0 Hz, 2H), 1.91 (dt, J = 7.6 Hz, J = 7.6 Hz, 2H), 1.51-1.44 (m, 2H), 1.39-1.32 (m, 4H), 0.94-0.90 (m, 3H).

### (2-Hexylanthra[1,2-b:5,6-b']dithiophene)

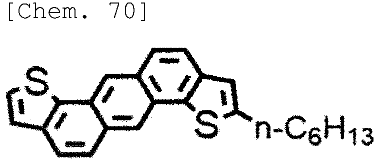

### Synthesis Example 4 2-Bromo-8-hexylanthra[1,2-b:5,6-b']dithiophene

In a nitrogen atmosphere, 246 mg (0.655 mmol) of the 2-hexylanthra[1,2-b:5,6-b']dithiophene synthesized in Synthesis Example 3 and 10 ml of THF (FUJIFILM Wako Pure Chemical Corporation, dehydration grade) were added to a 50-ml Schlenk reaction vessel. The mixture was cooled to -78°C and stirred for 3 hours at -78°C with 1.50 mL (2.40 mmol) 1.6 M normal-butyllithium (FUJIFILM Wako Pure Chemical Corporation) added thereto. 864 mg (2.65 mmol) of 1,2-dibromotetrachloroethane was added at -78°C, and the resulting mixture was stirred while being warmed to room temperature. 1 M hydrochloric acid was added, and the solid was filtered and washed with water and methanol, giving 234 mg of a yellow solid of 2-bromo-8-hexylanthra[1,2-b:5,6-b']dithiophene (yield, 78%).

¹H NMR (CDCl₃): δ = 8.59 (s, 1H), 8.52 (s, 1H), 7.87 (d, J = 6.0 Hz, 1H), 7.84 (d, J = 6.0 Hz, 1H), 7.73 (d, J = 8.9 Hz, 1H), 7.68 (d, J = 8.8 Hz, 1H), 7.46 (s, 1H), 7.16 (s, 1H), 3.01 (t, J = 7.3 Hz, 2H), 1.83 (dt, J = 7.6 Hz, J = 7.6 Hz, 2H), 1.49-1.43 (m, 2H), 1.39-1.32 (m, 4H), 0.94-0.90 (m, 3H).

### (2-Bromo-8-hexylanthra[1,2-b:5,6-b']dithiophene)

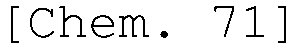

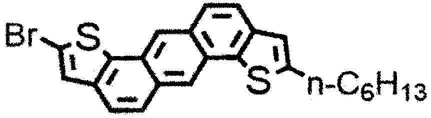

### Example 1 Synthesis of 2-(2-Cyclohexylethyl)anthra[1,2-b:5,6-b']dithiophene (compound 1)

In a nitrogen atmosphere, 605 mg (purity, 60%; 0.983 mmol) of 2-bromoanthra[1,2-b:5,6-b']dithiophene synthesized by the method described in WO 2021/177417 (compound 22 in the publication), 55.8 mg (0.249 mmol) of palladium(II) acetate (FUJIFILM Wako Pure Chemical Corporation), 231 mg (0.494 mmol) of Ruphos (Tokyo Chemical Industry Co., Ltd.) and 409 mg (3.64 mmol) of potassium tert-butoxide (Tokyo Chemical Industry Co., Ltd.) were added to a 200-mL Schlenk tube. 38 mL of a solution of 799 mg (3.36 mmol) of the 2-(2-cyclohexylethyl)-4,4,5,5-tetramethyl-1,3,2-dioxaborolane synthesized in Synthesis Example 2 in toluene (FUJIFILM Wako Pure Chemical Corporation, dehydration grade) and 3.0 mL of water were added to the tube, and the resulting mixture was stirred for 23 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from hexane/toluene = 1/1, giving 186 mg of a yellow solid of 2-(2-cyclohexylethyl)anthra[1,2-b:5,6-b']dithiophene (compound 1) (yield, 47%).

¹H NMR (CDCl₃): δ = 8.70 (s, 1H), 8.62 (s, 1H), 7.89 (d, J = 6.4 Hz, 1H), 7.87 (d, J = 6.4 Hz, 1H), 7.82 (d, J = 8.8 Hz, 1H), 7.72 (d, J = 8.7 Hz, 1H), 7.53 (d, J = 5.5 Hz, 1H), 7.48 (d, J = 5.0 Hz, 1H), 7.16 (s, 1H), 3.03 (t, J = 8.2 Hz, 2H), 1.87-1.64 (m, 7H), 1.47-1.36 (m, 1H), 1.32-1.13 (m, 3H), 1.05-0.95 (m, 2H).

Melting point: 161°C

### (Compound 1)

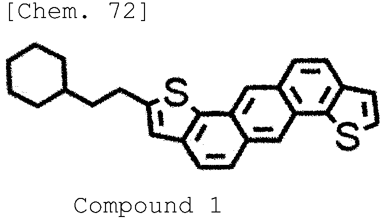

### Example 2 Synthesis of 2,8-Di(2-cyclohexylethyl)anthra[1,2-b:5,6-b']dithiophene (compound 2)

In a nitrogen atmosphere, 75.0 mg (0.167 mmol) of 2,8-dibromoanthra[1,2-b:5,6-b']dithiophene synthesized by the method described in WO 2021/177417 (compound 20 in the publication), 9.5 mg (0.042 mmol) of palladium(II) acetate (FUJIFILM Wako Pure Chemical Corporation), 42.0 mg (0.0900 mmol) of Ruphos (Tokyo Chemical Industry Co., Ltd.) and 149 mg (1.33 mmol) of potassium tert-butoxide (Tokyo Chemical Industry Co., Ltd.) were added to a 100-mL Schlenk tube. 6.5 mL of a solution of 274 mg (1.15 mmol) of the 2-(2-cyclohexylethyl)-4,4,5,5-tetramethyl-1,3,2-dioxaborolane synthesized in Synthesis Example 2 in toluene (FUJIFILM Wako Pure Chemical Corporation, dehydration grade) and 0.5 mL of water were added to the tube, and the resulting mixture was stirred for 25 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from hexane, giving 25.8 mg of a yellow solid of 2,8-di(2-cyclohexylethyl)anthra[1,2-b:5,6-b']dithiophene (compound 2) (yield, 30%).

¹H NMR (CDCl₃): δ = 8.58 (s, 2H), 7.84 (d, J = 8.8 Hz, 2H), 7.69 (d, J = 8.9 Hz, 2H), 7.15 (s, 2H), 3.03 (t, J = 7.2 Hz, 4H), 1.84-1.65 (m, 14H), 1.48-1.37 (m, 2H), 1.32-1.16 (m, 6H), 1.05-0.95 (m, 4H).

Melting point: 217°C

### (Compound 2)

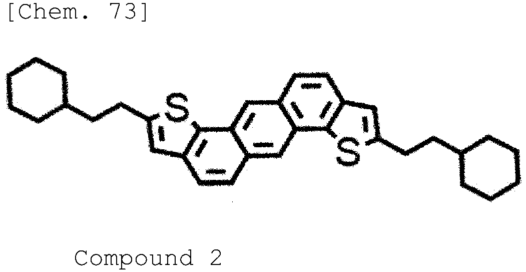

### Example 3 Synthesis of 2-Bromo-8-(2-cyclohexylethyl)anthra[1,2-b:5,6-b']dithiophene (compound 3)

In a nitrogen atmosphere, 150 mg (0.375 mmol) of compound 1, synthesized in Example 1, and 10 ml of THF (FUJIFILM Wako Pure Chemical Corporation, dehydration grade) were added to a 100-ml Schlenk reaction vessel. The mixture was cooled to -78°C and stirred for 10 minutes at -78°C and for 1 hour at room temperature with 0.50 mL (0.80 mmol) of 1.6 M normal-butyllithium (FUJIFILM Wako Pure Chemical Corporation) added thereto. After cooling to -78°C, 345 mg (1.06 mmol) of 1,2-dibromotetrachloroethane was added, and the resulting mixture was stirred while being warmed to room temperature. 1 M hydrochloric acid was added, and the solid was filtered and washed with water and methanol, giving 155 mg of a yellow solid of 2-bromo-8-(2-cyclohexylethyl)anthra[1,2-b:5,6-b']dithiophene (compound 3) (yield, 82%).

¹H NMR (CDCl₃): δ = 8.59 (s, 1H), 8.53 (s, 1H), 7.87 (d, J = 6.0 Hz, 1H), 7.85 (d, J = 6.0 Hz, 1H), 7.73 (d, J = 8.8 Hz, 1H), 7.69 (d, J = 8.9 Hz, 1H), 7.46 (s, 1H), 7.16 (s, 1H), 3.04 (t, J = 7.5 Hz, 2H), 1.86-1.66 (m, 7H), 1.46-1.38 (m, 1H), 1.32-1.12 (m, 3H), 1.05-0.98 (m, 2H).

### (Compound 3)

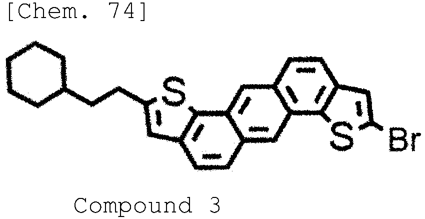

### Example 4 Synthesis of 2-(2-Cyclohexylethyl)-8-propylanthra[1,2-b:5,6-b']dithiophene (compound 4)

In a nitrogen atmosphere, 49.5 mg (0.103 mmol) of compound 3, synthesized in Example 3, 7.4 mg (0.033 mmol) of palladium(II) acetate (FUJIFILM Wako Pure Chemical Corporation), 30.6 mg (0.0656 mmol) of Ruphos (Tokyo Chemical Industry Co., Ltd.), 53.0 mg (0.472 mmol) of potassium tert-butoxide (Tokyo Chemical Industry Co., Ltd.) and 36.7 mg (0.417 mmol) of propylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 2.5 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 13.2 mg of a yellow solid of 2-(2-cyclohexylethyl)-8-propylanthra[1,2-b:5,6-b']dithiophene (compound 4) (yield, 28%).

¹H NMR (CDCl₃): δ = 8.59 (s, 2H), 7.85 (d, J = 8.7 Hz, 2H), 7.70 (d, J = 8.7 Hz, 1H), 7.70 (d, J = 8.7 Hz, 1H), 7.16 (s, 1H), 7.15 (s, 1H), 3.05-2.98 (m, 4H), 1.89-1.65 (m, 9H), 1.46-1.38 (m, 1H), 1.32-1.17 (m, 3H), 1.08 (t, J = 7.3 Hz, 3H), 1.04-0.96 (m, 2H).

Melting point: 144°C

### (Compound 4)

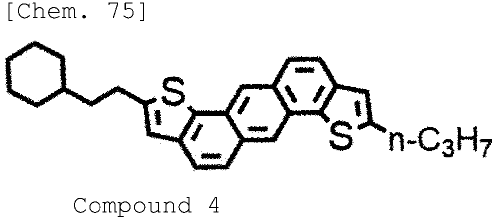

### Example 5 Synthesis of 2-(2-Cyclohexylethyl)-8-isobutylanthra[1,2-b:5,6-b']dithiophene (compound 5)

In a nitrogen atmosphere, 49.3 mg (0.103 mmol) of compound 3, synthesized in Example 3, 6.9 mg (0.031 mmol) of palladium(II) acetate (FUJIFILM Wako Pure Chemical Corporation), 29.2 mg (0.0626 mmol) of Ruphos (Tokyo Chemical Industry Co., Ltd.), 52.6 mg (0.469 mmol) of potassium tert-butoxide (Tokyo Chemical Industry Co., Ltd.) and 43.0 mg (0.422 mmol) of isobutylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 2 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 15.8 mg of a yellow solid of 2-(2-cyclohexylethyl)-8-isobutylanthra[1,2-b:5,6-b']dithiophene (compound 5) (yield, 33%).

¹H NMR (CDCl₃): δ = 8.59 (s, 2H), 7.85 (d, J = 8.8 Hz, 2H), 7.71 (d, J = 8.8 Hz, 1H), 7.70 (d, J = 8.8 Hz, 1H), 7.15 (s, 1H), 7.14 (s, 1H), 3.03 (t, J = 7.8 Hz, 2H), 2.88 (d, J = 7.2 Hz, 2H), 2.13-2.03 (m, 1H), 1.85-1.66 (m, 7H), 1.47-1.37 (m, 1H), 1.32-1.16 (m, 3H), 1.06 (s, 3H), 1.04 (s, 3H), 1.01-0.95 (m, 2H).

### Melting point: 166°C

### (Compound 5)

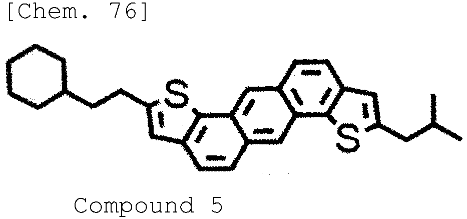

### Example 6 Synthesis of 2-Butyl-8-(2-cyclohexylethyl)anthra[1,2-b:5,6-b']dithiophene (compound 6)

In a nitrogen atmosphere, 29.7 mg (0.0619 mmol) of compound 3, synthesized in Example 3, 5.0 mg (0.022 mmol) of palladium(II) acetate (FUJIFILM Wako Pure Chemical Corporation), 20.5 mg (0.0439 mmol) of Ruphos (Tokyo Chemical Industry Co., Ltd.), 28.7 mg (0.256 mmol) of potassium tert-butoxide (Tokyo Chemical Industry Co., Ltd.) and 22.2 mg (0.218 mmol) butylboronic acid (Tokyo Chemical Industry Co., Ltd.) were added to a 50-mL Schlenk tube. 3.0 mL of toluene (FUJIFILM Wako Pure Chemical Corporation, dehydration grade) and 0.3 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from heptane, giving 10.2 mg of a yellow solid of 2-butyl-8-(2-cyclohexylethyl)anthra[1,2-b:5,6-b']dithiophene (compound 6) (yield, 36%).

¹H NMR (CDCl₃): δ = 8.58 (s, 2H), 7.84 (d, J = 8.8 Hz, 2H), 7.70 (d, J = 8.7 Hz, 2H), 7.16-7.14 (m, 2H), 3.05-3.00 (m, 4H), 1.86-1.64 (m, 9H), 1.52-1.47 (m, 2H), 1.45-1.38 (m, 1H), 1.31-1.16 (m, 3H), 1.05-0.95 (m, 5H).

Melting point: 140°C

### (Compound 6)

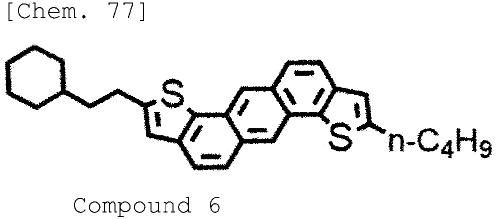

### Example 7 Synthesis of 2-(2-Cyclohexylethyl)-8-pentylanthra[1,2-b:5,6-b']dithiophene (compound 7)

In a nitrogen atmosphere, 25.2 mg (0.0526 mmol) of compound 3, synthesized in Example 3, 5.8 mg (0.026 mmol) of palladium(II) acetate (FUJIFILM Wako Pure Chemical Corporation), 24.0 mg (0.0514 mmol) of Ruphos (Tokyo Chemical Industry Co., Ltd.), 21.4 mg (0.185 mmol) potassium tert-butoxide (Tokyo Chemical Industry Co., Ltd.) and 21.4 mg (0.185 mmol) of pentylboronic acid (Tokyo Chemical Industry Co., Ltd.) were added to a 50-mL Schlenk tube. 2.5 mL of toluene (FUJIFILM Wako Pure Chemical Corporation, dehydration grade) and 0.25 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from heptane, giving 9.9 mg of a yellow solid of 2-(2-cyclohexylethyl)-8-pentylanthra[1,2-b:5,6-b']dithiophene (compound 7) (yield, 40%).

¹H NMR (CDCl₃): δ = 8.59 (s, 2H), 7.84 (d, J = 8.8 Hz, 2H), 7.70 (d, J = 8.8 Hz, 2H), 7.16-7.15 (m, 2H), 3.04 (d, J = 7.0 Hz, 2H), 3.00 (d, J = 7.0 Hz, 2H), 1.87-1.66 (m, 9H), 1.49-1.39 (m, 5H), 1.28-1.12 (m, 3H), 1.04-0.98 (m, 2H), 0.95 (d, J = 7.1 Hz, 2H).

Melting point: 148°C

### (Compound 7)

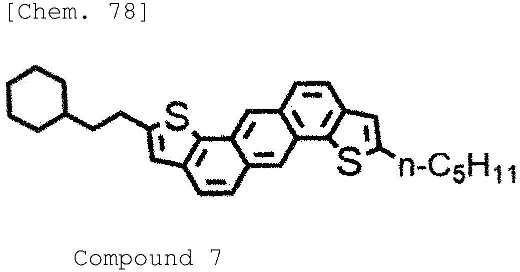

### Example 8 Synthesis of 2-(2-Cyclohexylethyl)-8-hexylanthra[1,2-b:5,6-b']dithiophene (compound 8)

In a nitrogen atmosphere, 60.5 mg (0.133 mmol) of the 2-bromo-8-hexylanthra[1,2-b:5,6-b']dithiophene synthesized in Synthesis Example 4, 9.2 mg (0.041 mmol) of palladium(II) acetate (FUJIFILM Wako Pure Chemical Corporation), 38.1 mg (0.0816 mmol) of Ruphos (Tokyo Chemical Industry Co., Ltd.) and 62.9 mg (0.560 mmol) of potassium tert-butoxide (Tokyo Chemical Industry Co., Ltd.) were added to a 50-mL Schlenk tube. 6.0 mL of a solution of 128 mg (0.539 mmol) of the 2-(2-cyclohexylethyl)-4,4,5,5-tetramethyl-1,3,2-dioxaborolane synthesized in Synthesis Example 2 in toluene (FUJIFILM Wako Pure Chemical Corporation, dehydration grade) and 0.5 mL of water were added to the tube, and the resulting mixture was stirred for 24 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from hexane, giving 18.2 mg of a yellow solid of 2-(2-cyclohexylethyl)-8-hexylanthra[1,2-b:5,6-b']dithiophene (compound 8) (yield, 28%).

¹H NMR (CDCl₃): δ = 8.58 (s, 2H), 7.84 (d, J = 8.8 Hz, 2H), 7.70 (d, J = 8.8 Hz, 2H), 7.15-7.14 (m, 2H), 3.04 (d, J = 7.2 Hz, 2H), 3.00 (d, J = 7.0 Hz, 2H), 1.85-1.69 (m, 9H), 1.47-1.17 (m, 12H), 1.04-0.89 (m, 5H).

Melting point: 133°C

### (Compound 8)

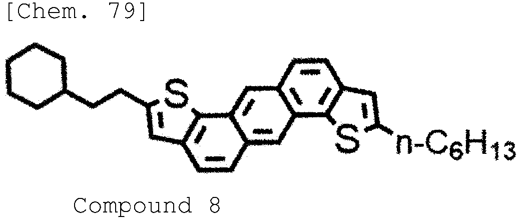

### Synthesis Example 5 Synthesis of (2-Bromoethyl)cyclopentane

In a nitrogen atmosphere, 4.55 g (39.8 mmol) of 2-cyclopentylethanol (Tokyo Chemical Industry Co., Ltd.) and 40 ml of toluene (FUJIFILM Wako Pure Chemical Corporation, dehydration grade) were added to a 200-ml two-neck flask. The solution was stirred for 20 minutes at room temperature with 1.70 ml (17.9 mmol) of phosphorus tribromide (FUJIFILM Wako Pure Chemical Corporation) added thereto, and the resulting mixture was stirred for 3 hours at 100°C. The reaction solution was poured into ice and neutralized with a saturated aqueous solution of sodium hydrogencarbonate, then toluene was added, and phase separation was performed. The organic phase was washed with water, dried over anhydrous sodium sulfate and concentrated under reduced pressure, giving 3.63 g of a colorless liquid of (2-bromoethyl)cyclopentane (yield, 51%).

¹H NMR (CDCl₃): δ = 3.42 (t, J = 7.8 Hz, 2H), 1.99-1.77 (m, 5H), 1.66-1.50 (m, 4H), 1.15-1.06 (m, 2H).

### ((2-Bromoethyl)cyclopentane)

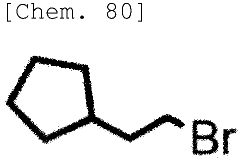

### Synthesis Example 6 Synthesis of 2-(2-Cyclopentylethyl)-4,4,5,5-tetramethyl-1,3,2-dioxaborolane

In a nitrogen atmosphere, 506 mg (0.874 mmol) of Xantphos (Tokyo Chemical Industry Co., Ltd.), 4.49 g (17.7 mmol) of bis(pinacolato)diboron (Tokyo Chemical Industry Co., Ltd.), 85.1 mg (0.860 mmol) of copper(I) chloride (FUJIFILM Wako Pure Chemical Corporation), 2.16 g (19.2 mmol) of potassium tert-butoxide (Tokyo Chemical Industry Co., Ltd.) and 12 ml of THF (FUJIFILM Wako Pure Chemical Corporation, dehydration grade) were added to a 100-ml Schlenk reaction vessel, and the resulting mixture was stirred for 1.5 hours at room temperature. 8 mL of a solution of 2.63 g (14.8 mmol) of the (2-bromoethyl)cyclopentane synthesized in Synthesis Example 5 in THF (FUJIFILM Wako Pure Chemical Corporation, dehydration grade) was added with cooling on ice, and the resulting mixture was stirred for 10 minutes at 0°C and for 20 hours at room temperature. The reaction mixture was cooled on ice, water was added, then toluene was added, and phase separation was performed. The organic phase was washed with water, dried over anhydrous sodium sulfate and concentrated under reduced pressure. The resulting residue was purified by silica gel column chromatography (solvent; toluene), giving 2.77 g of a colorless liquid of 2-(2-cyclopentylethyl)-4,4,5,5-tetramethyl-1,3,2-dioxaborolane (yield, 80%).

¹H NMR (CDCl₃): δ = 1.77-1.66 (m, 3H), 1.59-1.38 (m, 6H), 1.24 (s, 12H), 1.12-1.04 (m, 2H), 0.7 (t, J = 8.4 Hz, 2H).

### (2-(2-Cyclopentylethyl)-4,4,5,5-tetramethyl-1,3,2-dioxaborolane)

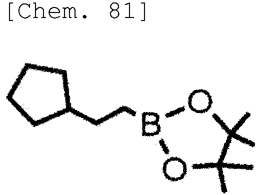

### Example 9 Synthesis of 2-(2-Cyclopentylethyl)anthra[1,2-b:5,6-b']dithiophene (compound 9)

In a nitrogen atmosphere, 603 mg (purity, 60%; 0.980 mmol) of 2-bromoanthra[1,2-b:5,6-b']dithiophene synthesized by the method described in WO 2021/177417 (compound 22 in the publication), 59.8 mg (0.266 mmol) of palladium(II) acetate (FUJIFILM Wako Pure Chemical Corporation), 249 mg (0.533 mmol) of Ruphos (Tokyo Chemical Industry Co., Ltd.) and 465 mg (4.14 mmol) of potassium tert-butoxide (Tokyo Chemical Industry Co., Ltd.) were added to a 200-mL Schlenk tube. 20 mL of a solution of 747 mg (3.36 mmol) of the 2-(2-cyclopentylethyl)-4,4,5,5-tetramethyl-1,3,2-dioxaborolane synthesized in Synthesis Example 6 in toluene (FUJIFILM Wako Pure Chemical Corporation, dehydration grade) and 3.0 mL of water were added to the tube, and the resulting mixture was stirred for 24 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane/toluene = 20/1, giving 128 mg of a yellow solid of 2-(2-cyclopentylethyl)anthra[1,2-b:5,6-b']dithiophene (compound 9) (yield, 34%).

¹H NMR (CDCl₃): δ = 8.70 (s, 1H), 8.62 (s, 1H), 7.89 (d, J = 6.2 Hz, 1H), 7.87 (d, J = 6.3 Hz, 1H), 7.82 (d, J = 8.8 Hz, 1H), 7.72 (d, J = 8.8 Hz, 1H), 7.54 (d, J = 5.6 Hz, 1H), 7.49 (d, J = 5.1 Hz, 1H), 7.17 (s, 1H), 3.05 (t, J = 7.7 Hz, 2H), 1.96-1.83 (m, 5H), 1.69-1.56 (m, 4H), 1.25-1.16 (m, 2H) .

### (Compound 9)

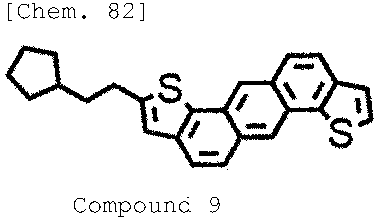

### Example 10 Synthesis of 2,8-Di(2-cyclopentylethyl)anthra[1,2-b:5,6-b']dithiophene (compound 10)

In a nitrogen atmosphere, 213 mg (0.476 mmol) of 2,8-dibromoanthra[1,2-b:5,6-b']dithiophene synthesized by the method described in WO 2021/177417 (compound 20 in the publication), 29.4 mg (0.131 mmol) of palladium(II) acetate (FUJIFILM Wako Pure Chemical Corporation), 126 mg (0.270 mmol) of Ruphos (Tokyo Chemical Industry Co., Ltd.) and 275 mg (2.45 mmol) of potassium tert-butoxide (Tokyo Chemical Industry Co., Ltd.) were added to a 200-mL Schlenk tube. 20.0 mL of a solution of 458 mg (2.04 mmol) of the 2-(2-cyclopentylethyl)-4,4,5,5-tetramethyl-1,3,2-dioxaborolane synthesized in Synthesis Example 6 in toluene (FUJIFILM Wako Pure Chemical Corporation, dehydration grade) and 2.0 mL of water were added to the tube, and the resulting mixture was stirred for 25 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from heptane, giving 57.5 mg of a yellow solid of 2,8-di(2-cyclopentylethyl)anthra[1,2-b:5,6-b']dithiophene (compound 10) (yield, 25%).

¹H NMR (CDCl₃): δ = 8.58 (s, 2H), 7.84 (d, J = 8.7 Hz, 2H), 7.70 (d, J = 8.9 Hz, 2H), 7.16 (s, 2H), 3.04 (t, J = 8.4 Hz, 4H), 1.97-1.83 (m, 10H), 1.70-1.56 (m, 8H), 1.25-1.16 (m, 4H).

Melting point: 188°C

### (Compound 10)

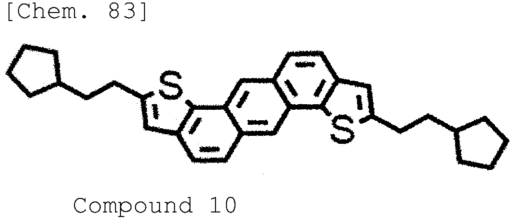

### Example 11 Synthesis of 2-Bromo-8-(2-cyclopentylethyl)anthra[1,2-b:5,6-b']dithiophene (compound 11)

In a nitrogen atmosphere, 150 mg (0.389 mmol) of compound 10, synthesized in Example 10, and 10 ml of THF (FUJIFILM Wako Pure Chemical Corporation, dehydration grade) were added to a 100-ml Schlenk reaction vessel. The mixture was cooled to -78°C and stirred for 15 minutes at -78°C and for 1 hour at room temperature with 0.80 mL (1.28 mmol) of 1.6 M normal-butyllithium (FUJIFILM Wako Pure Chemical Corporation) added thereto. After cooling to -78°C, 466 mg (1.43 mmol) of 1,2-dibromotetrachloroethane was added, and the resulting mixture was stirred while being warmed to room temperature. 1 M hydrochloric acid was added, and the solid was filtered and washed with water and methanol, giving 145 mg of a yellow solid of 2-bromo-8-(2-cyclopentylethyl)anthra[1,2-b:5,6-b']dithiophene (compound 11) (yield, 77%).

¹H NMR (CDCl₃): δ = 8.59 (s, 1H), 8.52 (s, 1H), 7.87 (d, J = 5.9 Hz, 1H), 7.84 (d, J = 5.7 Hz, 1H), 7.73 (d, J = 8.8 Hz, 1H), 7.68 (d, J = 8.8 Hz, 1H), 7.46 (s, 1H), 7.17 (s, 1H), 3.03 (t, J = 7.6 Hz, 2H), 1.98-1.83 (m, 5H), 1.70-1.55 (m, 4H), 1.25-1.16 (m, 2H).

### (Compound 11)

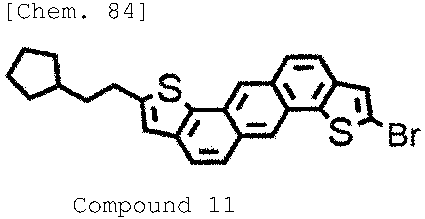

### Example 12 Synthesis of 2-(2-Cyclopentylethyl)-8-isobutylanthra[1,2-b:5,6-b']dithiophene (compound 12)

In a nitrogen atmosphere, 45.9 mg (0.0986 mmol) of compound 11, synthesized in Example 11, 5.5 mg (0.024 mmol) of palladium(II) acetate (FUJIFILM Wako Pure Chemical Corporation), 23.3 mg (0.0499 mmol) of Ruphos (Tokyo Chemical Industry Co., Ltd.), 35.4 mg (0.315 mmol) of potassium tert-butoxide (Tokyo Chemical Industry Co., Ltd.) and 25.8 mg (0.253 mmol) of isobutylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 24 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 12.6 mg of a yellow solid of 2-(2-cyclohexylethyl)-8-isobutylanthra[1,2-b:5,6-b']dithiophene (compound 12) (yield, 29%).

¹H NMR (CDCl₃): δ = 8.56 (s, 2H), 7.85 (d, J = 8.8 Hz, 2H), 7.71 (d, J = 9.0 Hz, 1H), 7.70 (d, J = 8.8 Hz, 1H), 7.16 (s, 1H), 7.14 (s, 1H), 3.04 (t, J = 8.5 Hz, 2H), 2.88 (d, J = 7.2 Hz, 2H), 2.11-2.03 (m, 1H), 1.97-1.83 (m, 5H), 1.71-1.54 (m, 4H), 1.25-1.16 (m, 2H), 1.05 (d, J = 6.6 Hz, 6H).

Melting point: 151°C

### (Compound 12)

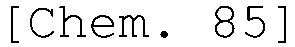

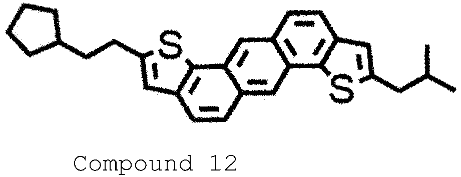

### Example 13 Synthesis of 2-(2-Cyclopentylethyl)-8-hexylanthra[1,2-b:5,6-b']dithiophene (compound 13)

In a nitrogen atmosphere, 58.9 mg (0.130 mmol) of the 2-bromo-8-hexylanthra[1,2-b:5,6-b']dithiophene synthesized in Synthesis Example 4, 9.5 mg (0.042 mmol) of palladium(II) acetate (FUJIFILM Wako Pure Chemical Corporation), 40.6 mg (0.0870 mmol) of Ruphos (Tokyo Chemical Industry Co., Ltd.) and 53.5 mg (0.477 mmol) of potassium tert-butoxide (Tokyo Chemical Industry Co., Ltd.) were added to a 50-mL Schlenk tube. 6.0 mL of a solution of 87.1 mg (0.389 mmol) of the 2-(2-cyclopentylethyl)-4,4,5,5-tetramethyl-1,3,2-dioxaborolane synthesized in Synthesis Example 6 in toluene (FUJIFILM Wako Pure Chemical Corporation, dehydration grade) and 0.5 mL of water were added to the tube, and the resulting mixture was stirred for 27 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from hexane, giving 10.7 mg of a yellow solid of 2-(2-cyclopentylethyl)-8-hexylanthra[1,2-b:5,6-b']dithiophene (compound 13) (yield, 17%).

¹H NMR (CDCl₃): δ = 8.58 (s, 2H), 7.84 (d, J = 8.8 Hz, 2H), 7.70 (d, J = 8.8 Hz, 2H), 7.16-7.15 (m, 2H), 3.03 (t, J = 7.8 Hz, 2H), 3.01 (t, J = 7.8 Hz, 2H), 1.97-1.79 (m, 7H), 1.71-1.55 (m, 4H), 1.49-1.43 (m, 2H), 1.40-1.32 (m, 4H), 1.25-1.16 (m, 2H), 0.93-0.89 (m, 3H).

Melting point: 134°C

### (Compound 13)

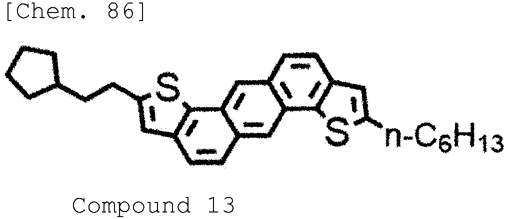

### Example 14 Synthesis of 2-(2-Cyclohexylethyl)-8-(2-cyclopentylethyl)anthra[1,2-b:5,6-b']dithiophene (compound 14)

In a nitrogen atmosphere, 46.7 mg (0.100 mmol) of compound 11, synthesized in Example 11, 5.5 mg (0.025 mmol) of palladium(II) acetate (FUJIFILM Wako Pure Chemical Corporation), 22.5 mg (0.0482 mmol) of Ruphos (Tokyo Chemical Industry Co., Ltd.) and 49.4 mg (0.440 mmol) of potassium tert-butoxide (Tokyo Chemical Industry Co., Ltd.) were added to a 50-mL Schlenk tube. 5.0 mL of a solution of 90.2 mg (0.379 mmol) of the 2-(2-cyclohexylethyl)-4,4,5,5-tetramethyl-1,3,2-dioxaborolane synthesized in Synthesis Example 2 in toluene (FUJIFILM Wako Pure Chemical Corporation, dehydration grade) and 0.5 mL of water were added to the tube, and the resulting mixture was stirred for 27 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane). The resulting solid was purified by recrystallization from heptane, giving 13.8 mg of a yellow solid of 2-(2-cyclohexylethyl)-8-(2-cyclopentylethyl)anthra[1,2-b:5,6-b']dithiophene (compound 14) (yield, 27%).

1H NMR (CDCl₃): δ = 8.58 (s, 2H), 7.84 (d, J = 8.8 Hz, 2H), 7.70 (d, J = 8.9 Hz, 2H), 7.16-7.15 (m, 2H), 3.03 (t, J = 7.5 Hz, 4H), 1.96-1.82 (m, 7H), 1.77-1.57 (m, 8H), 1.47-1.37 (m, 1H), 1.32-1.16 (m, 6H), 1.05-0.95 (m, 2H).

Melting point: 186°C

### (Compound 14)

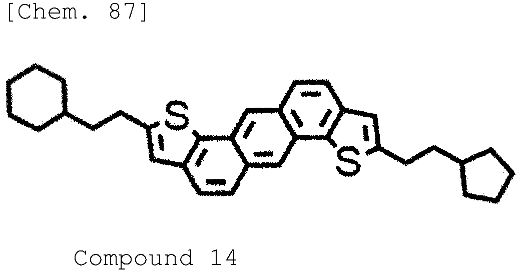

### Synthesis Example 7 Synthesis of 2-(3-Cyclohexylpropyl)-4,4,5,5-tetramethyl-1,3,2-dioxaborolane

In a nitrogen atmosphere, 50.0 mL (45.0 mmol) of an 8.5% borane/THF solution (Tokyo Chemical Industry Co., Ltd.) and 3.45 mL (22.5 mmol) of allylcyclohexane (Tokyo Chemical Industry Co., Ltd.) were added to a 200-ml two-neck flask with cooling on ice, and the resulting mixture was stirred for 1 hour at 0°C and for 1.5 hours at room temperature. 6.1 mL of water was added with cooling on ice, and the resulting mixture was stirred for 2 hours at room temperature. The resulting mixture was concentrated under reduced pressure, ethyl acetate and an aqueous solution of sodium hydrogencarbonate were added, and phase separation was performed. The organic phase was washed with saline solution, dried over anhydrous sodium sulfate and concentrated under reduced pressure. In this manner, (3-cyclohexylpropyl)boronic acid was synthesized.

In a nitrogen atmosphere, the resulting boronic acid was added to a 300-mL two-neck flask and stirred at room temperature with 3.32 g (28.1 mmol) of pinacol (Tokyo Chemical Industry Co., Ltd.) and 25.0 mL of diethyl ether (FUJIFILM Wako Pure Chemical Corporation, dehydration grade) added thereto. 3.51 g (29.1 mmol) of magnesium sulfate (FUJIFILM Wako Pure Chemical Corporation) was added to the flask, and the resulting mixture was stirred for 4 hours at room temperature. The solid was isolated by filtration under reduced pressure, and the filtrate was concentrated under reduced pressure. The resulting residue was purified by silica gel column chromatography (solvent; toluene), giving 2.53 g of a colorless liquid of 2-(2-cyclohexylpropyl)-4,4,5,5-tetramethyl-1,3,2-dioxaborolane (yield, 29%).

### (2-(3-Cyclohexylpropyl)-4,4,5,5-tetramethyl-1,3,2-dioxaborolane)

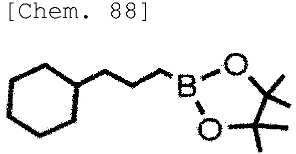

### Example 15 Synthesis of 2-(3-Cyclohexylpropyl)anthra[1,2-b:5,6-b']dithiophene (compound 15)

In a nitrogen atmosphere, 706 mg of a mixture of 2-bromoanthra[1,2-b:5,6-b']dithiophene and 2,8-dibromoanthra[1,2-b:5,6-b']dithiophene synthesized by the methods described in WO 2021/177417 (compound 22 and compound 20, respectively, in the publication), 85.0 mg (0.379 mmol) of palladium(II) acetate (FUJIFILM Wako Pure Chemical Corporation), 358 mg (0.767 mmol) of Ruphos (Tokyo Chemical Industry Co., Ltd.) and 705 mg (6.28 mmol) of potassium tert-butoxide (Tokyo Chemical Industry Co., Ltd.) were added to a 200-mL Schlenk tube. 60 mL of a solution of 1.48 g (5.88 mmol) of the 2-(2-cyclohexylpropyl)-4,4,5,5-tetramethyl-1,3,2-dioxaborolane synthesized in Synthesis Example 7 in toluene (FUJIFILM Wako Pure Chemical Corporation, dehydration grade) and 6.0 mL of water were added to the tube, and the resulting mixture was stirred for 2 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was separated by recycling HPLC and purified by recrystallization from heptane/toluene = 10/1, giving 247 mg of a yellow solid of 2-(3-cyclohexylpropyl)anthra[1,2-b:5,6-b']dithiophene (compound 15).

¹H NMR (CDCl₃): δ = 8.70 (s, 1H), 8.62 (s, 1H), 7.89 (d, J = 8.7 Hz, 1H), 7.87 (d, J = 8.7 Hz, 1H), 7.82 (d, J = 8.9 Hz, 1H), 7.73 (d, J = 8.8 Hz, 1H), 7.54 (d, J = 5.1 Hz, 1H), 7.49 (d, J = 5.1 Hz, 1H), 7.16 (s, 1H), 3.00 (t, J = 7.2 Hz, 2H), 1.88-1.81 (m, 2H), 1.78-1.65 (m, 5H), 1.38-1.11 (m, 6H), 0.96-0.88 (m, 2H).

### (Compound 15)

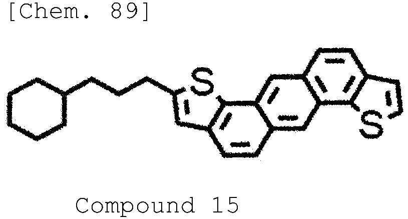

### Example 16 Synthesis of 2,8-Bis(3-cyclohexylpropyl)anthra[1,2-b:5,6-b']dithiophene (compound 16)

The other component obtained in the recycling HPLC purification in Example 15 was purified by recrystallization from heptane/toluene = 10/1, giving 86.7 mg of a yellow solid of 2,8-bis(3-cyclohexylpropyl)anthra[1,2-b:5,6-b']dithiophene (compound 16).

¹H NMR (CDCl₃): δ = 8.59 (s, 2H), 7.84 (d, J = 8.7 Hz, 2H), 7.70 (d, J = 8.8 Hz, 2H), 7.15 (s, 2H), 3.00 (t, J = 7.3 Hz, 4H), 1.88-1.80 (m, 4H), 1.77-1.64 (m, 10H), 1.38-1.10 (m, 12H), 0.96-0.86 (m, 4H).

Melting point: 169°C

### (Compound 16)

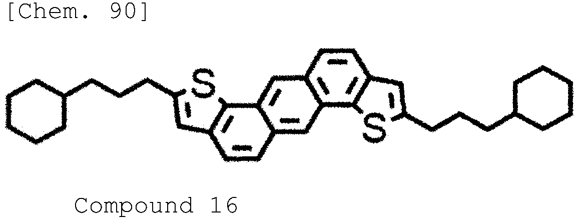

### Example 17 Synthesis of 2-Bromo-8-(3-cyclohexylpropyl)anthra[1,2-b:5,6-b']dithiophene (compound 17)

In a nitrogen atmosphere, 203 mg (0.489 mmol) of compound 15, synthesized in Example 15, and 10 ml of THF (FUJIFILM Wako Pure Chemical Corporation, dehydration grade) were added to a 50-ml Schlenk reaction vessel. The mixture was cooled to -78°C and stirred for 2 hours at -78°C with 1.10 mL (1.76 mmol) of 1.6 M normal-butyllithium (FUJIFILM Wako Pure Chemical Corporation) added thereto. After cooling to -78°C, 638 mg (1.96 mmol) of 1,2-dibromotetrachloroethane was added, and the resulting mixture was stirred while being warmed to room temperature. 1 M hydrochloric acid was added, and the solid was filtered and washed with water and methanol, giving 227 mg of a yellow solid of 2-bromo-8-(3-cyclohexylpropyl)anthra[1,2-b:5,6-b']dithiophene (compound 17) (yield, 93%).

¹H NMR (CDCl₃): δ = 8.59 (s, 1H), 8.52 (s, 1H), 7.86 (d, J = 8.7 Hz, 1H), 7.85 (d, J = 8.7 Hz, 1H), 7.73 (d, J = 8.8 Hz, 1H), 7.69 (d, J = 8.8 Hz, 1H), 7.46 (s, 1H), 7.16 (s, 1H), 3.00 (t, J = 7.4 Hz, 2H), 1.88-1.80 (m, 2H), 1.77-1.64 (m, 5H), 1.38-1.10 (m, 6H), 0.96-0.87 (m, 2H).

### (Compound 17)

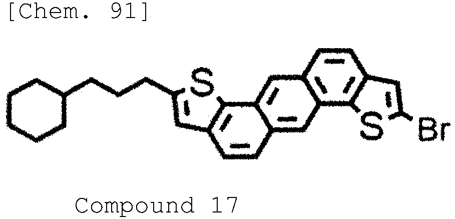

### Example 18 Synthesis of 2-(3-Cyclohexylpropyl)-8-hexylanthra[1,2-b:5,6-b']dithiophene (compound 18)

In a nitrogen atmosphere, 51.0 mg (0.103 mmol) of compound 17, synthesized in Example 17, 5.00 mg (0.0223 mmol) of palladium(II) acetate (FUJIFILM Wako Pure Chemical Corporation), 21.8 mg (0.0467 mmol) of Ruphos (Tokyo Chemical Industry Co., Ltd.), 41.7 mg (0.372 mmol) of potassium tert-butoxide (Tokyo Chemical Industry Co., Ltd.) and 44.5 mg (0.342 mmol) of hexylboronic acid (Tokyo Chemical Industry Co., Ltd.) were added to a 50-mL Schlenk tube. 6.0 mL of toluene (FUJIFILM Wako Pure Chemical Corporation, dehydration grade) and 0.60 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 22.7 mg of a yellow solid of 2-(3-cyclohexylpropyl)-8-hexylanthra[1,2-b:5,6-b']dithiophene (compound 18) (yield, 44%).

¹H NMR (CDCl₃): δ = 8.59 (s, 2H), 7.84 (d, J = 8.7 Hz, 2H), 7.70 (d, J = 8.8 Hz, 2H), 7.16 (s, 2H), 3.02 (t, J = 7.9 Hz, 2H), 2.98 (t, J = 8.3 Hz, 2H), 1.87-1.79 (m, 4H), 1.77-1.63 (m, 5H), 1.51-1.43 (m, 2H), 1.38-1.13 (m, 10H), 0.96-0.87 (m, 5H).

Melting point: 132°C

### (Compound 18)

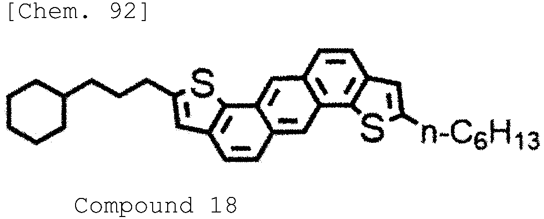

### Synthesis Example 8 Synthesis of (2-Cyclohexylethyl)magnesium Bromide

In a nitrogen atmosphere, 174 mg (7.15 mmol) of magnesium (FUJIFILM Wako Pure Chemical Corporation, turnings) was added to a 30-mL two-neck flask and stirred in a vacuum. In a nitrogen atmosphere, 15 mL of a solution of 1.15 g (6.01 mmol) of the (2-bromoethyl)cyclohexane synthesized in Synthesis Example 1 in THF (FUJIFILM Wako Pure Chemical Corporation, dehydration grade) was added, and the resulting mixture was stirred for 3 and a half hours at room temperature. By removing the solid by filtration, a 0.4 M (2-cyclohexylethyl)magnesium bromide/THF solution was obtained.

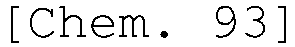

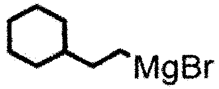

### Example 19 Synthesis of 2-(2-Cyclohexylethyl)biphenyleno[1,2-b:5,6-b']dithiophene

### (compound 19)

In a nitrogen atmosphere, 203 mg (1.49 mmol) of zinc chloride (FUJIFILM Wako Pure Chemical Corporation) and 5 mL of THF (FUJIFILM Wako Pure Chemical Corporation, dehydration grade) were added to a 50-mL Schlenk tube and stirred with cooling on ice. 1.50 mL (0.600 mmol) of the 0.4 M (2-cyclohexylethyl)magnesium bromide/THF solution synthesized in Synthesis Example 8 was added with cooling on ice, and the resulting mixture was stirred for 30 minutes at 0°C and for 2 hours at room temperature. In this manner, a zinc reagent solution was prepared.

To this solution, 120 mg (purity, 86%; 0.300 mmol) of 2-bromobiphenyleno[1,2-b:5,6-b']dithiophene synthesized by the method described in WO 2021/177417 (compound 21 in the publication) and 12.5 mg (0.0171 mmol) of [1,1'-bis(diphenylphosphino)ferrocene]dichloropalladium(II) (Sigma-Aldrich Co. LLC) were added. The mixture was stirred for 19 hours at room temperature. The reaction mixture was cooled on ice, 1 M hydrochloric acid was added, then toluene was added, and phase separation was performed. The organic phase was washed with water, dried over anhydrous sodium sulfate and concentrated under reduced pressure. The resulting residue was purified by silica gel chromatography (solvent; toluene). The resulting solid was purified by recycling HPLC, and the product was purified by recrystallization from heptane, giving 59.6 mg of a red-orange solid of 2-(2-cyclohexylethyl)biphenyleno[1,2-b:5,6-b']dithiophene (compound 19) (yield, 53%).

1H NMR (CDCl3): δ = 7.18 (d, J = 7.6 Hz, 1H), 7.18 (d, J = 6.0 Hz, 1H), 7.03 (d, J = 5.6 Hz, 1H), 7.01 (d, J = 7.6 Hz, 1H), 6.70 (d, J = 7.6 Hz, 1H), 6.70 (s, 1H), 6.66 (d, J = 7.6 Hz, 1H), 2.80 (t, J = 7.6 Hz, 2H), 1.78-1.57 (m, 7H), 1.38-1.10 (m, 4H), 0.99-0.86 (m, 2H).

Melting point: 134°C

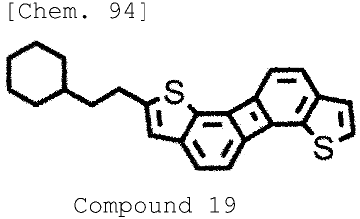

### Example 20 Synthesis of 2,7-Bis(2-cyclohexylethyl)biphenyleno[1,2-b:5,6-b']dithiophene (compound 20)

In a nitrogen atmosphere, 164 mg (1.20 mmol) of zinc chloride (FUJIFILM Wako Pure Chemical Corporation) and 4 mL of THF (FUJIFILM Wako Pure Chemical Corporation, dehydration grade) were added to a 100-mL Schlenk tube and stirred with cooling on ice. 1.20 mL (0.480 mmol) of the 0.4 M (2-cyclohexylethyl)magnesium bromide/THF solution synthesized in Synthesis Example 8 was added with cooling on ice, and the resulting mixture was stirred for 17 hours at room temperature. In this manner, a zinc reagent solution was prepared.

To this solution, 50.7 mg (0.120 mmol) of 2,7-dibromobiphenyleno[1,2-b:5,6-b']dithiophene synthesized by the method described in WO 2021/177417 (compound 3 in the publication) and 8.78 mg (0.012 mmol) of [1,1'-bis(diphenylphosphino)ferrocene]dichloropalladium(II) (Sigma-Aldrich Co. LLC) were added. The mixture was stirred for 6 and a half hours at room temperature. The reaction mixture was cooled on ice, 1 M hydrochloric acid was added, then toluene was added, and phase separation was performed. The organic phase was washed with water, dried over anhydrous sodium sulfate and concentrated under reduced pressure.

The resulting residue was purified by silica gel chromatography (solvent; toluene). The resulting solid was purified by recycling HPLC, and the product was purified by recrystallization from hexane/toluene = 2/1. The product was further purified by silica gel chromatography (solvent; hexane), and the product was purified by recrystallization from hexane, giving 10.2 mg of a red-orange solid of 2,7-bis(2-cyclohexylethyl)biphenyleno[1,2-b:5,6-b']dithiophene (compound 20) (yield, 17%).

1H NMR (CDCl3): δ = 7.00 (d, J = 7.2 Hz, 2H), 6.69 (s, 2H), 6.63 (d, J = 8.0 Hz, 2H), 2.80 (t, J = 7.6 Hz, 4H), 1.78-1.64 (m, 10H), 1.63-1.57 (m, 4H), 1.38-1.28 (m, 2H), 1.26-1.11 (m, 6H), 0.99-0.90 (m, 4H).

Melting point: 174°C

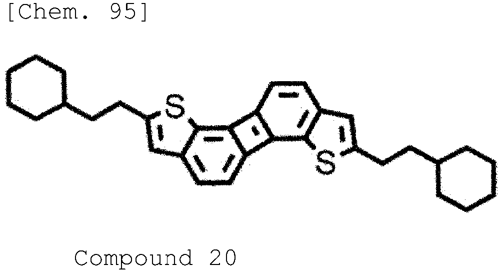

### Example 21 Synthesis of 2-Bromo-7-(2-cyclohexylethyl)biphenyleno[1,2-b:5,6-b']dithiophene (compound 21)

In a nitrogen atmosphere, 44.8 mg (0.120 mmol) of compound 19, synthesized in Example 19, and 5 ml of THF (FUJIFILM Wako Pure Chemical Corporation, dehydration grade) were added to a 50-ml Schlenk reaction vessel. The mixture was cooled to -78°C and stirred for 1 hour at -78°C with 0.224 mL (0.359 mmol) of 1.6 M normal-butyllithium (FUJIFILM Wako Pure Chemical Corporation) added thereto. After cooling to -78°C, 1 mL of 135 mg (0.416 mmol) of 1,2-dibromotetrachloroethane in THF (FUJIFILM Wako Pure Chemical Corporation, dehydration grade) was added, and the resulting mixture was stirred while being warmed to room temperature. 1 M hydrochloric acid was added, and the solid was filtered and washed with water and methanol, giving 38.2 mg of a red-orange solid of 2-bromo-7-(2-cyclohexylethyl)biphenyleno[1,2-b:5,6-b']dithiophene (compound 21) (yield, 69%).

1H NMR (CDCl3): δ = 7.03 (d, J = 7.2 Hz, 2H), 7.03 (s, 1H), 6.71 (s, 1H), 6.64 (d, J = 7.2 Hz, 1H), 6.63 (d, J = 7.2 Hz, 1H), 2.80 (t, J = 7.6 Hz, 2H), 1.75-1.57 (m, 7H), 1.35-1.14 (m, 4H), 0.98-0.90 (m, 2H).

Melting point: 142°C

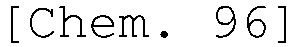

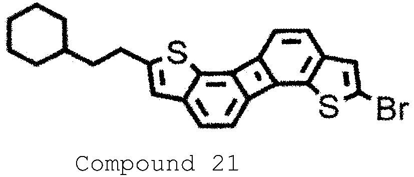

### Example 22 Synthesis of 2-(2-Cyclohexylethyl)hexylbiphenyleno[1,2-b:5,6-b']dithiophene (compound 22)

In a nitrogen atmosphere, 52.5 mg (0.385 mmol) of zinc chloride (FUJIFILM Wako Pure Chemical Corporation) and 4 mL of THF (FUJIFILM Wako Pure Chemical Corporation, dehydration grade) were added to a 50-mL Schlenk tube and stirred with cooling on ice. 0.0679 mL (0.136 mmol) of a 2.0 M hexylmagnesium bromide/THF solution (Sigma-Aldrich Co. LLC) was added with cooling on ice, and the resulting mixture was stirred for 20 minutes at 0°C and for 30 minutes at room temperature. In this manner, a zinc reagent solution was prepared.

To this solution, 30.8 mg (0.0679 mmol) of compound 21, synthesized in Example 21, and 6.10 mg (0.00834 mmol) of [1,1'-bis(diphenylphosphino)ferrocene]dichloropalladium(II) (Sigma-Aldrich Co. LLC) were added. The mixture was stirred for 5 hours at room temperature. The reaction mixture was cooled on ice, 1 M hydrochloric acid was added, then toluene was added, and phase separation was performed. The organic phase was washed with water, dried over anhydrous sodium sulfate and concentrated under reduced pressure. The resulting residue was purified by silica gel chromatography (solvent; toluene). The resulting solid was purified by recycling HPLC, and the product was purified by recrystallization from heptane, giving 10.9 mg of a red-orange solid of 2-(2-cyclohexylethyl)hexylbiphenyleno[1,2-b:5,6-b']dithiophene (compound 22) (yield, 34%).

1H NMR (CDCl3): δ = 6.99 (d, J = 6.8 Hz, 2H), 6.70 (s, 1H), 6.69 (s, 1H), 6.63 (d, J = 7.2 Hz, 2H), 2.80 (t, J = 7.2 Hz, 2H), 2.78 (t, J = 7.2 Hz, 2H), 1.78-1.64 (m, 7H), 1.63-1.57 (m, 2H), 1.42-1.10 (m, 10H), 0.98-0.88 (m, 5H).

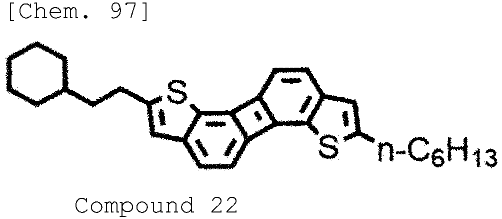

### Synthesis Example 9 Synthesis of (2-Cyclopentylethyl)magnesium Bromide

In a nitrogen atmosphere, 177 mg (7.27 mmol) of magnesium (FUJIFILM Wako Pure Chemical Corporation, turnings) was added to a 30-mL two-neck flask and stirred in a vacuum. In a nitrogen atmosphere, 15 mL of a solution of 1.06 g (6.00 mmol) of the (2-bromoethyl)cyclopentane synthesized in Synthesis Example 5 in THF (FUJIFILM Wako Pure Chemical Corporation, dehydration grade) was added, and the resulting mixture was stirred for 2 hours at room temperature. By removing the solid by filtration, a 0.4 M (2-cyclopentylethyl)magnesium bromide/THF solution was obtained.

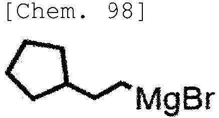

### Example 23 Synthesis of 2,7-Bis(2-cyclopentylethyl)biphenyleno[1,2-b:5,6-b']dithiophene (compound 23)

In a nitrogen atmosphere, 182 mg (1.34 mmol) of zinc chloride (FUJIFILM Wako Pure Chemical Corporation) and 4 mL of THF (FUJIFILM Wako Pure Chemical Corporation, dehydration grade) were added to a 50-mL Schlenk tube and stirred with cooling on ice. 1.20 mL (0.480 mmol) of the 0.4 M (2-cyclopentylethyl)magnesium bromide/THF solution synthesized in Synthesis Example 9 was added with cooling on ice, and the resulting mixture was stirred for 15 hours at room temperature. In this manner, a zinc reagent solution was prepared.

To this solution, 50.9 mg (0.121 mmol) of 2,7-dibromobiphenyleno[1,2-b:5,6-b']dithiophene synthesized by the method described in WO 2021/177417 (compound 3 in the publication) and 8.8 mg (0.012 mmol) of [1,1'-bis(diphenylphosphino)ferrocene]dichloropalladium(II) (Sigma-Aldrich Co. LLC) were added. The mixture was stirred for 25 and a half hours at room temperature. The reaction mixture was cooled on ice, 1 M hydrochloric acid was added, then toluene was added, and phase separation was performed. The organic phase was washed with water, dried over anhydrous sodium sulfate and concentrated under reduced pressure.

The resulting residue was purified by silica gel chromatography (solvent; toluene). The resulting solid was purified by recycling HPLC, and the product was purified by recrystallization from hexane/toluene = 4/1, giving 14.0 mg of a red-orange solid of 2,7-bis(2-cyclopentylethyl)biphenyleno[1,2-b:5,6-b']dithiophene (compound 23) (yield, 25%).

1H NMR (CDCl3): δ = 6.99 (d, J = 7.6 Hz, 2H), 6.70 (s, 2H), 6.63 (d, J = 8.0 Hz, 2H), 2.80 (t, J = 7.6 Hz, 4H), 1.92-1.77 (m, 6H), 1.75-1.69 (m, 4H), 1.67-1.59 (m, 4H), 1.57-1.48 (m, 4H), 1.18-1.10 (m, 4H).

Melting point: 152°C

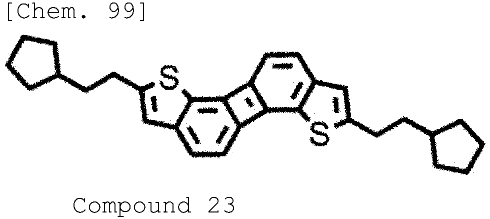

### Example 24 Evaluation of Solubility

Toluene and n-octane were each added to predetermined amounts of the aromatic compounds obtained in Examples 1, 4, 5, 6, 7, 8, 9, 12, 13, 14, 15, 18, 19, 20, 22 and 23 to give compositions for film formation. The weight of each organic solvent required to completely dissolve the aromatic compound at room temperature (25°C) was measured, and solubility (% by weight) was calculated. The determination of the point of complete dissolution was confirmed by visual observation. The solubility of the evaluated aromatic compounds is presented in Table 1.

**[Table 1]**

| Solubility of Aromatic Compounds according to the Present Invention | | |
|---|---|---|
| Aromatic compound | Solubility [% by weight] | |
| | Toluene | n-octane |
| Compound 1 (Example 1) | ≥1.0 | ≥0.1 |
| Compound 4 (Example 4) | ≥1.0 | ≥0.5 |
| Compound 5 (Example 5) | ≥1.0 | ≥0.2 |
| Compound 6 (Example 6) | ≥1.0 | ≥0.5 |
| Compound 7 (Example 7) | ≥1.0 | ≥0.2 |
| Compound 8 (Example 8) | ≥1.0 | ≥0.5 |
| Compound 9 (Example 9) | ≥1.0 | ≥1.0 |
| Compound 12 (Example 12) | ≥1.0 | ≥0.5 |
| Compound 13 (Example 13) | ≥1.0 | ≥0.5 |
| Compound 14 (Example 14) | ≥1.0 | ≥1.0 |
| Compound 15 (Example 15) | ≥1.0 | ≥0.5 |
| Compound 18 (Example 18) | ≥1.0 | ≥0.2 |
| Compound 19 (Example 19) | ≥1.0 | ≥0.5 |
| Compound 20 (Example 20) | ≥1.0 | ≥0.5 |
| Compound 23 (Example 23) | ≥1.0 | ≥0.2 |

### Example 25 Production 1 of Solution for Forming an Organic Semiconductor Layer, Organic Semiconductor Layer and Organic Thin Film Transistor

In air, 1.74 mg of the 2,8-di(2-cyclohexylethyl)anthra[1,2-b:5,6-b']dithiophene synthesized in Example 2 (compound 2) and 868 mg of toluene (FUJIFILM Wako Pure Chemical Industries Corporation, pure grade) were added to a 10-ml sample tube. After dissolution by heating to 50°C, the solution was allowed to cool at room temperature (25°C), whereby a solution for forming an organic semiconductor layer was prepared. This solution for forming an organic semiconductor layer remained in solution form even after 10 hours at 25°C (the concentration of compound 2 was 0.20% by weight), confirming that compound 2 is a compound suitable for film formation by drop casting and inkjet printing.

Using the resulting solution for forming an organic semiconductor layer, a bottom gate-bottom contact p-type organic thin film transistor was fabricated. The materials (base materials) and film formation methods for the individual structural components are presented in Table 2.

**[Table 2]**

| Structural component | Base material | Film formation method |
|---|---|---|
| Substrate | Glass | - |
| Gate electrode | Silver | Vacuum deposition |
| Gate insulating layer | Parylene C | CVD |
| Source and drain electrodes | Gold (W/L = 500/100 µm) | Vacuum deposition |
| Surface treatment agent | Pentafluorobenzenethiol | Dipping |
| Organic semiconductor | Compound 2 | Drop casting |

The evaluation of the transmission characteristics of this transistor element was performed, and the hole carrier mobility was found to be 1.46 cm²/V·sec.

### Example 26 Production 2 of Solution for Forming an Organic Semiconductor Layer, Organic Semiconductor Layer and Organic Thin Film Transistor

A solution for forming an organic semiconductor layer was prepared by the same method as in Example 25, except that the 2-(2-cyclohexylethyl)-8-hexylanthra[1,2-b:5,6-b']dithiophene synthesized in Example 8 (compound 8) was used. This solution for forming an organic semiconductor layer remained in solution form even after 10 hours at 25°C (the concentration of compound 8 was 0.20% by weight), confirming that compound 8 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 1.67 cm²/V·sec.

### Example 27 Production 3 of Solution for Forming an Organic Semiconductor Layer, Organic Semiconductor Layer and Organic Thin Film Transistor

A solution for forming an organic semiconductor layer was prepared by the same method as in Example 25, except that the 2,8-di(2-cyclopentylethyl)anthra[1,2-b:5,6-b']dithiophene synthesized in Example 10 (compound 10) was used. This solution for forming an organic semiconductor layer remained in solution form even after 10 hours at 25°C (the concentration of compound 10 was 0.20% by weight), confirming that compound 10 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 3.33 cm²/V·sec.

### Example 28 Production 4 of Solution for Forming an Organic Semiconductor Layer, Organic Semiconductor Layer and Organic Thin Film Transistor

A solution for forming an organic semiconductor layer was prepared by the same method as in Example 25, except that the 2-(2-cyclopentylethyl)-8-isobutylanthra[1,2-b:5,6-b']dithiophene synthesized in Example 12 (compound 12) was used. This solution for forming an organic semiconductor layer remained in solution form even after 10 hours at 25°C (the concentration of compound 12 was 0.20% by weight), confirming that compound 12 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.22 cm²/V·sec.

### Example 29 Production 5 of Solution for Forming an Organic Semiconductor Layer, Organic Semiconductor Layer and Organic Thin Film Transistor

A solution for forming an organic semiconductor layer was prepared by the same method as in Example 25, except that the 2-(2-cyclopentylethyl)-8-hexylanthra[1,2-b:5,6-b']dithiophene synthesized in Example 13 (compound 13) was used. This solution for forming an organic semiconductor layer remained in solution form even after 10 hours at 25°C (the concentration of compound 13 was 0.20% by weight), confirming that compound 13 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 1.17 cm²/V·sec.

### Example 30 Production 6 of Solution for Forming an Organic Semiconductor Layer, Organic Semiconductor Layer and Organic Thin Film Transistor

A solution for forming an organic semiconductor layer was prepared by the same method as in Example 25, except that the 2-(2-cyclohexylethyl)-8-(2-cyclopentylethyl)anthra[1,2-b:5,6-b']dithiophene synthesized in Example 14 (compound 14) was used. This solution for forming an organic semiconductor layer remained in solution form even after 10 hours at 25°C (the concentration of compound 14 was 0.20% by weight), confirming that compound 14 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.20 cm²/V·sec.

### Example 31 Production 7 of Solution for Forming an Organic Semiconductor Layer, Organic Semiconductor Layer and Organic Thin Film Transistor

A solution for forming an organic semiconductor layer was prepared by the same method as in Example 25, except that the 2-(3-cyclohexylpropyl)-8-hexylanthra[1,2-b:5,6-b']dithiophene synthesized in Example 18 (compound 18) was used. This solution for forming an organic semiconductor layer remained in solution form even after 10 hours at 25°C (the concentration of compound 18 was 0.20% by weight), confirming that compound 18 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.48 cm²/V·sec.

### Example 32 Production 8 of Solution for Forming an Organic Semiconductor Layer, Organic Semiconductor Layer and Organic Thin Film Transistor

A solution for forming an organic semiconductor layer was prepared by the same method as in Example 25, except that the 2,7-bis(2-cyclohexylethyl)biphenyleno[1,2-b:5,6-b']dithiophene synthesized in Example 20 (compound 20) was used. This solution for forming an organic semiconductor layer remained in solution form even after 10 hours at 25°C (the concentration of compound 20 was 0.2% by weight), confirming that compound 20 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.22 cm²/V·sec.

### Example 33 Synthesis of 2-(3-Cyclohexylpropyl)-8-butylanthra[1,2-b:5,6-b']dithiophene (compound 24)

In a nitrogen atmosphere, 40.5 mg (0.0821 mmol) of compound 17, synthesized in Example 17, 5.70 mg (0.0250 mmol) of palladium(II) acetate (FUJIFILM Wako Pure Chemical Corporation), 23.7 mg (0.0508 mmol) of Ruphos (Tokyo Chemical Industry Co., Ltd.), 33.6 mg (0.299 mmol) of potassium tert-butoxide (Tokyo Chemical Industry Co., Ltd.) and 29.3 mg (0.287 mmol) of butylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.50 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 21.8 mg of a yellow solid of 2-(3-cyclohexylpropyl)-8-butylanthra[1,2-b:5,6-b']dithiophene (compound 24) (yield, 56%).

¹H NMR (CDCl3): δ = 8.58 (s, 2H), 7.84 (d, J = 8.7 Hz, 2H), 7.70 (d, J = 8.7 Hz, 2H), 7.15 (s, 2H), 3.03 (t, J = 7.5 Hz, 2H), 2.99 (t, J = 7.6 Hz, 2H), 1.87-1.80 (m, 4H), 1.78-1.64 (m, 5H), 1.54-1.45 (m, 2H), 1.38-1.13 (m, 6H), 1.00 (t, J = 7.4 Hz, 3H), 1.02-0.88 (m, 2H).

### (Compound 24)

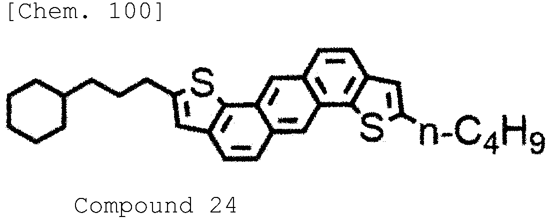

### Example 34 Synthesis of 2-(3-Cyclohexylpropyl)-8-pentylanthra[1,2-b:5,6-b']dithiophene (compound 25)

In a nitrogen atmosphere, 40.7 mg (0.0825 mmol) of compound 17, synthesized in Example 17, 6.20 mg (0.0280 mmol) of palladium(II) acetate (FUJIFILM Wako Pure Chemical Corporation), 26.7 mg (0.0572 mmol) of Ruphos (Tokyo Chemical Industry Co., Ltd.), 38.4 mg (0.342 mmol) of potassium tert-butoxide (Tokyo Chemical Industry Co., Ltd.) and 35.3 mg (0.304 mmol) of pentylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.50 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 21.2 mg of a yellow solid of 2-(3-cyclohexylpropyl)-8-pentylanthra[1,2-b:5,6-b']dithiophene (compound 25) (yield, 53%).

¹H NMR (CDCl₃): δ = 8.59 (s, 2H), 7.84 (d, J = 8.8 Hz, 2H), 7.70 (d, J = 8.7 Hz, 2H), 7.15 (s, 2H), 3.00 (m, 4H), 1.88-1.80 (m, 4H), 1.77-1.64 (m, 5H), 1.46-1.13 (m, 10H), 0.96-0.88 (m, 5H).

### (Compound 25)

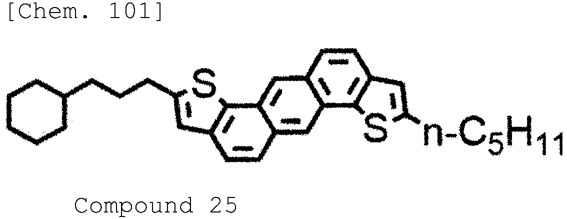

### Example 35 Synthesis of 2-(3-Cyclohexylpropyl)-8-octylanthra[1,2-b:5,6-b']dithiophene (compound 26)

In a nitrogen atmosphere, 44.0 mg (0.0892 mmol) of compound 17, synthesized in Example 17, 6.80 mg (0.0303 mmol) of palladium(II) acetate (FUJIFILM Wako Pure Chemical Corporation), 27.6 mg (0.0591 mmol) of Ruphos (Tokyo Chemical Industry Co., Ltd.), 41.0 mg (0.365 mmol) of potassium tert-butoxide (Tokyo Chemical Industry Co., Ltd.) and 51.2 mg (0.324 mmol) of octylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.50 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 29.4 mg of a yellow solid of 2-(3-cyclohexylpropyl)-8-octylanthra[1,2-b:5,6-b']dithiophene (compound 26) (yield, 62%).

¹H NMR (CDCl3): δ = 8.59 (s, 2H), 7.84 (d, J = 8.8 Hz, 2H), 7.70 (d, J = 8.8 Hz, 2H), 7.15 (s, 2H), 3.00 (m, 4H), 1.88-1.79 (m, 4H), 1.76-1.64 (m, 5H), 1.50-1.13 (m, 16H), 0.96-0.88 (m, 5H).

### (Compound 26)

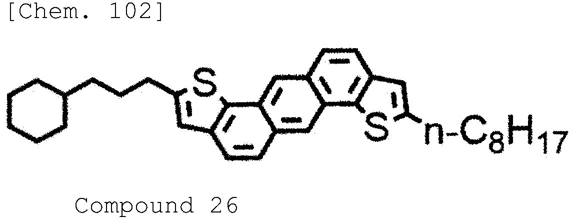

### Synthesis Example 10 Synthesis of 2-(3-Cyclopentylpropyl)-4,4,5,5-tetramethyl-1,3,2-dioxaborolane

In a nitrogen atmosphere, 30.0 mL (27.0 mmol) of an 8.5% borane/THF solution (Tokyo Chemical Industry Co., Ltd.) and 2.00 mL (14.3 mmol) of allylcyclopentane (Tokyo Chemical Industry Co., Ltd.) were added to a 100-ml two-neck flask with cooling on ice, and the resulting mixture was stirred for 50 minutes at 0°C and for 1.5 hours at room temperature. 4.0 mL of water was added with cooling on ice, and the resulting mixture was stirred for 2 hours at room temperature. The resulting mixture was concentrated under reduced pressure, ethyl acetate and an aqueous solution of sodium hydrogencarbonate were added, and phase separation was performed. The organic phase was washed with saline solution, dried over anhydrous sodium sulfate and concentrated under reduced pressure. In this manner, (3-cyclopentylpropyl)boronic acid was synthesized.

In a nitrogen atmosphere, the resulting boronic acid was added to a 200-mL two-neck flask and stirred at room temperature with 2.09 g (17.7 mmol) of pinacol (Tokyo Chemical Industry Co., Ltd.) and 15.0 mL of diethyl ether (FUJIFILM Wako Pure Chemical Corporation, dehydration grade) added thereto. 2.34 g (19.4 mmol) of magnesium sulfate (FUJIFILM Wako Pure Chemical Corporation) was added to the flask, and the resulting mixture was stirred for 4 hours at room temperature. The solid was isolated by filtration under reduced pressure, and the filtrate was concentrated under reduced pressure. The resulting residue was purified by silica gel column chromatography (solvent; toluene), giving 1.03 g of a colorless liquid of 2-(3-cyclopentylpropyl)-4,4,5,5-tetramethyl-1,3,2-dioxaborolane (yield, 27%).

### (2-(3-Cyclopentylpropyl)-4,4,5,5-tetramethyl-1,3,2-dioxaborolane)

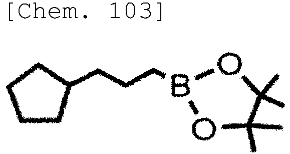

### Example 36 Synthesis of 2-(3-Cyclopentylpropyl)anthra[1,2-b:5,6-b']dithiophene (compound 27)

In a nitrogen atmosphere, 261 mg of a mixture of 2-bromoanthra[1,2-b:5,6-b']dithiophene and 2,8-dibromoanthra[1,2-b:5,6-b']dithiophene synthesized by the methods described in WO 2021/177417 (compound 22 and compound 20, respectively, in the publication), 32.3 mg (0.144 mmol) of palladium(II) acetate (FUJIFILM Wako Pure Chemical Corporation), 133 mg (0.285 mmol) of Ruphos (Tokyo Chemical Industry Co., Ltd.) and 267 mg (2.38 mmol) of potassium tert-butoxide (Tokyo Chemical Industry Co., Ltd.) were added to a 200-mL Schlenk tube. 20 mL of a solution of 534 mg (2.70 mmol) of the 2-(2-cyclopentylpropyl)-4,4,5,5-tetramethyl-1,3,2-dioxaborolane synthesized in Synthesis Example 10 in toluene (FUJIFILM Wako Pure Chemical Corporation, dehydration grade) and 2.0 mL of water were added to the tube, and the resulting mixture was stirred for 4 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was separated by recycling HPLC and purified by recrystallization from heptane, giving 82.8 mg of a yellow solid of 2-(3-cyclopentylpropyl)anthra[1,2-b:5,6-b']dithiophene (compound 27).

¹H NMR (CDCl3): δ = 8.70 (s, 1H), 8.62 (s, 1H), 7.89 (d, J = 8.8 Hz, 1H), 7.87 (d, J = 8.7 Hz, 1H), 7.82 (d, J = 8.7 Hz, 1H), 7.73 (d, J = 8.8 Hz, 1H), 7.54 (d, J = 5.1 Hz, 1H), 7.49 (d, J = 5.4 Hz, 1H), 7.17 (s, 1H), 3.02 (t, J = 7.3 Hz, 2H), 1.89-1.78 (m, 5H), 1.63-1.45 (m, 6H), 1.16-1.08 (m, 2H).

Melting point: 136°C

### (Compound 27)

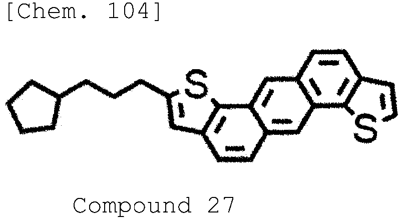

### Example 37 Synthesis of 2,8-Bis(3-cyclopentylpropyl)anthra[1,2-b:5,6-b']dithiophene (compound 28)

The other component obtained in the recycling HPLC purification in Example 36 was purified by recrystallization from heptane, giving 12.5 mg of a yellow solid of 2,8-bis(3-cyclopentylpropyl)anthra[1,2-b:5,6-b']dithiophene (compound 28).

¹H NMR (CDCl3): δ = 8.59 (s, 2H), 7.85 (d, J = 8.8 Hz, 2H), 7.70 (d, J = 8.8 Hz, 2H), 7.15 (s, 2H), 3.02 (t, J = 7.2 Hz, 4H), 1.88-1.76 (m, 10H), 1.65-1.44 (m, 12H), 1.38-1.10 (m, 12H), 1.15-1.09 (m, 4H).

Melting point: 143°C

### (Compound 28)

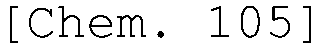

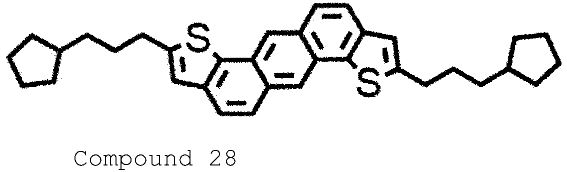

### Example 38 Synthesis of 2-Bromo-8-(3-cyclopentylpropyl)anthra[1,2-b:5,6-b']dithiophene (compound 29)

In a nitrogen atmosphere, 80.2 mg (0.193 mmol) of compound 27, synthesized in Example 36, and 5.0 ml of THF (FUJIFILM Wako Pure Chemical Corporation, dehydration grade) were added to a 50-ml Schlenk reaction vessel. The mixture was cooled to -78°C and stirred for 2 hours at -78°C with 1.00 mL (1.60 mmol) of 1.6 M normal-butyllithium (FUJIFILM Wako Pure Chemical Corporation) added thereto. After cooling to -78°C, 719 mg (2.21 mmol) of 1,2-dibromotetrachloroethane was added, and the resulting mixture was stirred while being warmed to room temperature. 1 M hydrochloric acid was added, and the solid was filtered and washed with water and methanol, giving 79.2 mg of a yellow solid of 2-bromo-8-(3-cyclopentylpropyl)anthra[1,2-b:5,6-b']dithiophene (compound 29) (yield, 82%).

¹H NMR (CDCl₃): δ = 8.59 (s, 1H), 8.52 (s, 1H), 7.86 (d, J = 8.7 Hz, 1H), 7.84 (d, J = 8.7 Hz, 1H), 7.73 (d, J = 8.7 Hz, 1H), 7.68 (d, J = 8.9 Hz, 1H), 7.46 (s, 1H), 7.16 (s, 1H), 3.01 (t, J = 7.4 Hz, 2H), 1.89-1.76 (m, 7H), 1.66-1.44 (m, 6H), 1.18-1.07 (m, 2H).

### (Compound 29)

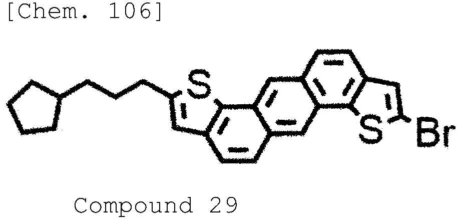

### Example 39 Synthesis of 2-(3-Cyclopentylpropyl)-8-pentylanthra[1,2-b:5,6-b']dithiophene (compound 30)

In a nitrogen atmosphere, 34.0 mg (0.0709 mmol) of compound 29, synthesized in Example 38, 5.60 mg (0.0249 mmol) of palladium(II) acetate (FUJIFILM Wako Pure Chemical Corporation), 23.0 mg (0.0493 mmol) of Ruphos (Tokyo Chemical Industry Co., Ltd.), 32.8 mg (0.292 mmol) of potassium tert-butoxide (Tokyo Chemical Industry Co., Ltd.) and 28.4 mg (0.245 mmol) of pentylboronic acid (Tokyo Chemical Industry Co., Ltd.) were added to a 50-mL Schlenk tube. 4.0 mL of toluene (FUJIFILM Wako Pure Chemical Corporation, dehydration grade) and 0.40 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 12.1 mg of a yellow solid of 2-(3-cyclopentylpropyl)-8-pentylanthra[1,2-b:5,6-b']dithiophene (compound 30) (yield, 36%).

¹H NMR (CDCl₃): δ = 8.59 (s, 2H), 7.84 (d, J = 8.8 Hz, 2H), 7.70 (d, J = 8.8 Hz, 2H), 7.15 (s, 2H), 3.01 (t, J = 7.5 Hz, 4H), 1.88-1.75 (m, 8H), 1.63-1.38 (m, 9H), 1.14-1.09 (m, 2H), 0.94 (t, J = 7.1 Hz, 3H).

### (Compound 30)

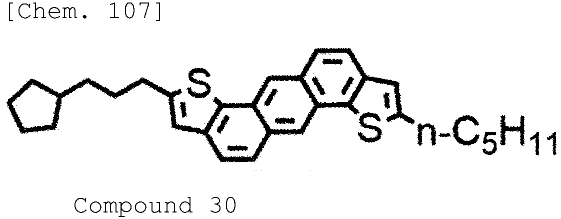

### Example 40 Synthesis of 2-(3-Cyclopentylpropyl)-8-hexylanthra[1,2-b:5,6-b']dithiophene (compound 31)

In a nitrogen atmosphere, 35.3 mg (0.0736 mmol) of compound 29, synthesized in Example 38, 4.80 mg (0.0213 mmol) of palladium(II) acetate (FUJIFILM Wako Pure Chemical Corporation), 20.6 mg (0.0441 mmol) of Ruphos (Tokyo Chemical Industry Co., Ltd.), 35.1 mg (0.313 mmol) of potassium tert-butoxide (Tokyo Chemical Industry Co., Ltd.) and 34.6 mg (0.266 mmol) of hexylboronic acid (Tokyo Chemical Industry Co., Ltd.) were added to a 50-mL Schlenk tube. 4.0 mL of toluene (FUJIFILM Wako Pure Chemical Corporation, dehydration grade) and 0.40 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 10.1 mg of a yellow solid of 2-(3-cyclopentylpropyl)-8-hexylanthra[1,2-b:5,6-b']dithiophene (compound 31) (yield, 28%).

¹H NMR (CDCl₃): δ = 8.59 (s, 2H), 7.84 (d, J = 8.7 Hz, 2H), 7.70 (d, J = 8.8 Hz, 2H), 7.15 (s, 2H), 3.02 (t, J = 7.4 Hz, 4H), 1.88-1.75 (m, 7H), 1.62-1.43 (m, 8H), 1.40-1.31 (m, 4H), 1.16-1.07 (m, 2H), 0.92 (t, J = 6.8 Hz, 3H).

### (Compound 31)

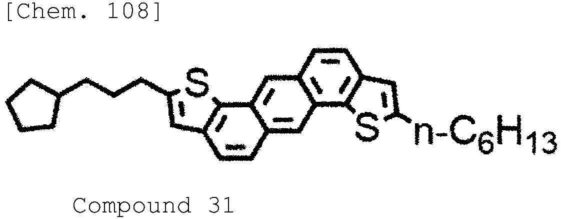

### Example 41 Synthesis of 2-(Cyclohexylmethyl)anthra[1,2-b:5,6-b']dithiophene (compound 32)

In a nitrogen atmosphere, 411 mg of a mixture of 2-bromoanthra[1,2-b:5,6-b']dithiophene and 2,8-dibromoanthra[1,2-b:5,6-b']dithiophene synthesized by the methods described in WO 2021/177417 (compound 22 and compound 20, respectively, in the publication), 88.7 mg (0.395 mmol) of palladium(II) acetate (FUJIFILM Wako Pure Chemical Corporation), 378 mg (0.285 mmol) of Ruphos (Tokyo Chemical Industry Co., Ltd.), 573 mg (4.03 mmol) of cyclohexylmethylboronic acid (FUJIFILM Wako Pure Chemical Corporation) and 476 mg (4.24 mmol) of potassium tert-butoxide (Tokyo Chemical Industry Co., Ltd.) were added to a 100-mL Schlenk tube. 30 mL of toluene and 1.5 mL of water were added to the tube, and the resulting mixture was stirred for 20 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was separated by recycling HPLC and purified by recrystallization from heptane, giving 171 mg of a yellow solid of 2-(cyclohexylmethyl)anthra[1,2-b:5,6-b']dithiophene (compound 32).

¹H NMR (CDCl₃): δ = 8.70 (s, 1H), 8.62 (s, 1H), 7.89 (d, J = 8.8 Hz, 1H), 7.87 (d, J = 8.7 Hz, 1H), 7.82 (d, J = 8.8 Hz, 1H), 7.73 (d, J = 8.7 Hz, 1H), 7.53 (d, J = 4.9 Hz, 1H), 7.49 (d, J = 5.1 Hz, 1H), 7.14 (s, 1H), 2.08 (d, J = 7.1 Hz, 2H), 1.89-1.73 (m, 2H), 1.77-1.67 (m, 4H), 1.33-1.18 (m, 3H), 1.10-1.01 (m, 2H).

Melting point: 137°C

### (Compound 32)

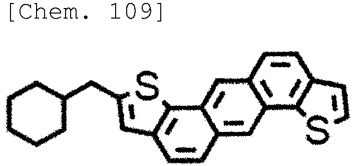

### Compound 32

### Example 42 Synthesis of 2,8-Bis(cyclohexylmethyl)anthra[1,2-b:5,6-b']dithiophene (compound 33)

The other component obtained in the recycling HPLC purification in Example 41 was purified by recrystallization from heptane, giving 23.8 mg of a yellow solid of 2,8-bis(cyclohexylmethyl)anthra[1,2-b:5,6-b']dithiophene (compound 33).

¹H NMR (CDCl3): δ = 8.58 (s, 2H), 7.84 (d, J = 8.7 Hz, 2H), 7.70 (d, J = 8.8 Hz, 2H), 7.13 (s, 2H), 2.88 (d, J = 6.9 Hz, 4H), 1.89-1.82 (m, 4H), 1.77-1.67 (m, 8H), 1.33-1.17 (m, 6H), 1.10-1.00 (m, 4H).

Melting point: 220°C

### (Compound 33)

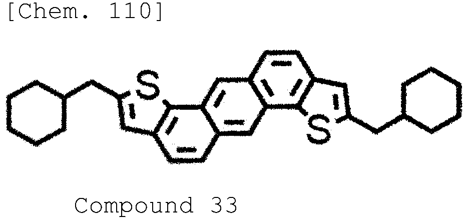

### Example 43 Synthesis of 2-Bromo-8-(cyclohexylmethyl)anthra[1,2-b:5,6-b']dithiophene (compound 34)

In a nitrogen atmosphere, 152 mg (0.394 mmol) of compound 32, synthesized in Example 41, and 5.0 ml of THF (FUJIFILM Wako Pure Chemical Corporation, dehydration grade) were added to a 50-ml Schlenk reaction vessel. The mixture was cooled to -78°C and stirred for 3 hours at -78°C with 0.80 mL (1.30 mmol) of 1.6 M normal-butyllithium (FUJIFILM Wako Pure Chemical Corporation) added thereto. After cooling to -78°C, 445 mg (1.37 mmol) of 1,2-dibromotetrachloroethane was added, and the resulting mixture was stirred while being warmed to room temperature. 1 M hydrochloric acid was added, and the solid was filtered and washed with water and methanol, giving 163 mg of a yellow solid of 2-bromo-8-(cyclohexylmethyl)anthra[1,2-b:5,6-b']dithiophene (compound 34) (yield, 88%).

¹H NMR (CDCl₃): δ = 8.59 (s, 1H), 8.54 (s, 1H), 7.86 (d, J = 8.8 Hz, 1H), 7.85 (d, J = 8.8 Hz, 1H), 7.73 (d, J = 8.7 Hz, 1H), 7.69 (d, J = 8.9 Hz, 1H), 7.68 (s, 1H), 7.14 (s, 1H), 2.89 (d, J = 7.1 Hz, 2H), 1.89-1.82 (m, 2H), 1.76-1.67 (m, 4H), 1.34-1.17 (m, 3H), 1.10-1.01 (m, 2H).

### (Compound 34)

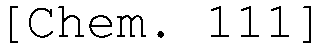

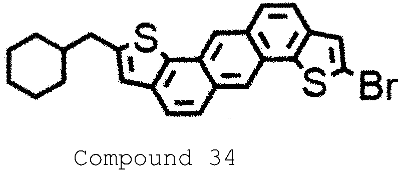

### Example 44 Synthesis of 2-(Cyclohexylmethyl)-8-hexylanthra[1,2-b:5,6-b']dithiophene (compound 35)

In a nitrogen atmosphere, 40.6 mg (0.0872 mmol) of compound 34, synthesized in Example 43, 6.70 mg (0.0298 mmol) of palladium(II) acetate (FUJIFILM Wako Pure Chemical Corporation), 27.7 mg (0.0594 mmol) of Ruphos (Tokyo Chemical Industry Co., Ltd.), 33.9 mg (0.302 mmol) of potassium tert-butoxide (Tokyo Chemical Industry Co., Ltd.) and 34.0 mg (0.262 mmol) of hexylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.50 mL of water were added to the tube, and the resulting mixture was stirred for 3.5 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 21.5 mg of a yellow solid of 2-(cyclohexylmethyl)-8-hexylanthra[1,2-b:5,6-b']dithiophene (compound 35) (yield, 52%).

¹H NMR (CDCl₃): δ = 8.58 (s, 2H), 7.84 (d, J = 8.7 Hz, 2H), 7.70 (d, J = 8.8 Hz, 2H), 7.15 (s, 1H), 7.13 (s, 1H), 3.02 (t, J = 7.4 Hz, 2H), 2.88 (d, J = 7.0 Hz, 2H), 1.88-1.79 (m, 4H), 1.76-1.66 (m, 4H), 1.52-1.43 (m, 2H), 1.40-1.36 (m, 4H), 1.35-1.19 (m, 3H), 1.10-1.00 (m, 2H), 0.92 (t, J = 6.9 Hz, 3H).

Melting point: 133°C

### (Compound 35)

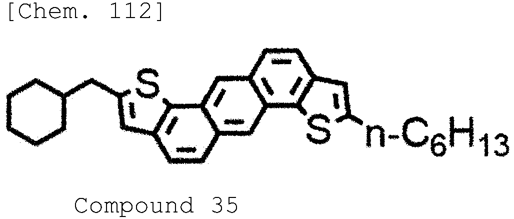

### Example 45 Synthesis of 2-(Cyclohexylmethyl)-8-octylanthra[1,2-b:5,6-b']dithiophene (compound 36)

In a nitrogen atmosphere, 43.6 mg (0.0937 mmol) of compound 34, synthesized in Example 43, 7.80 mg (0.0347 mmol) of palladium(II) acetate (FUJIFILM Wako Pure Chemical Corporation), 34.5 mg (0.0739 mmol) of Ruphos (Tokyo Chemical Industry Co., Ltd.), 33.6 mg (0.300 mmol) of potassium tert-butoxide (Tokyo Chemical Industry Co., Ltd.) and 44.3 mg (0.280 mmol) of octylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.50 mL of water were added to the tube, and the resulting mixture was stirred for 4 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (solvent; hexane:toluene = 2/1). The resulting solid was purified by recycling HPLC, and the product was purified by recrystallization from heptane, giving 30.6 mg of a yellow solid of 2-(cyclohexylmethyl)-8-octylanthra[1,2-b:5,6-b']dithiophene (compound 36) (yield, 65%).

¹H NMR (CDCl₃): δ = 8.58 (s, 2H), 7.84 (d, J = 8.7 Hz, 2H), 7.70 (d, J = 8.8 Hz, 2H), 7.15 (s, 1H), 7.12 (s, 1H), 3.02 (t, J = 7.5 Hz, 2H), 2.88 (d, J = 7.1 Hz, 2H), 1.88-1.70 (m, 8H), 1.49-1.20 (m, 13H), 1.09-1.00 (m, 2H), 0.90 (t, J = 6.6 Hz, 3H).

Melting point: 130°C

### (Compound 36)

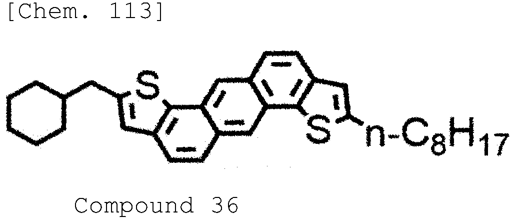

### Example 46 Synthesis of 2-(2-Cyclohexylethyl)-8-phenylanthra[1,2-b:5,6-b']dithiophene (compound 37)

In a nitrogen atmosphere, 32.9 mg (0.0686 mmol) of compound 3, synthesized in Example 3, 4.80 mg (0.021 mmol) of palladium(II) acetate (FUJIFILM Wako Pure Chemical Corporation), 19.9 mg (0.0426 mmol) of Ruphos (Tokyo Chemical Industry Co., Ltd.), 27.5 mg (0.245 mmol) of potassium tert-butoxide (Tokyo Chemical Industry Co., Ltd.) and 25.9 mg (0.212 mmol) of phenylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 4 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (toluene). The resulting solid was purified by recycling HPLC, and the product was purified by recrystallization from toluene/heptane, giving 19.2 mg of a yellow solid of 2-(2-cyclohexylethyl)-8-phenylanthra[1,2-b:5,6-b']dithiophene (compound 37) (yield, 58%).

¹H NMR (CDCl₃): δ = 8.68 (s, 1H), 8.61 (s, 1H), 7.90-7.78 (m, 5H), 7.73 (d, J = 8.8 Hz, 1H), 7.70 (s, 1H), 7.49-7.45 (m, 2H), 7.38-7.34 (m, 1H), 7.17 (s, 1H), 3.04 (t, J = 7.5 Hz, 2H), 1.85-1.66 (m, 7H), 1.47-1.37 (m, 1H), 1.32-1.14 (m, 3H), 1.05-0.96 (m, 2H).

Melting point: 229°C

### (Compound 37)

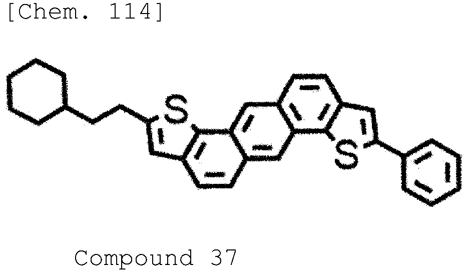

### Example 47 Synthesis of 2-(2-Cyclohexylethyl)-8-(p-tolyl)anthra[1,2-b:5,6-b']dithiophene (compound 38)

In a nitrogen atmosphere, 34.3 mg (0.0715 mmol) of compound 3, synthesized in Example 3, 5.40 mg (0.0241 mmol) of palladium(II) acetate (FUJIFILM Wako Pure Chemical Corporation), 22.3 mg (0.0478 mmol) of Ruphos (Tokyo Chemical Industry Co., Ltd.), 28.5 mg (0.254 mmol) of potassium tert-butoxide (Tokyo Chemical Industry Co., Ltd.) and 31.6 mg (0.232 mmol) of 4-methylphenylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 3 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (toluene). The resulting solid was purified by recycling HPLC, and the product was purified by recrystallization from toluene/heptane, giving 22.1 mg of a yellow solid of 2-(2-cyclohexylethyl)-8-(p-tolyl)anthra[1,2-b:5,6-b']dithiophene (compound 38) (yield, 62%).

¹H NMR (CDCl₃): δ = 8.66 (s, 1H), 8.60 (s, 1H), 7.89-7.86 (m, 2H), 7.77 (d, J = 8.8 Hz, 1H), 7.73-7.69 (m, 3H), 7.65 (s, 1H), 7.28 (m, 2H), 7.16 (s, 1H), 3.03 (t, J = 7.8 Hz, 2H), 2.42 (s, 3H), 1.85-1.66 (m, 7H), 1.48-1.37 (m, 1H), 1.32-1.14 (m, 3H), 1.05-0.96 (m, 2H).

Melting point: 240°C

### (Compound 38)

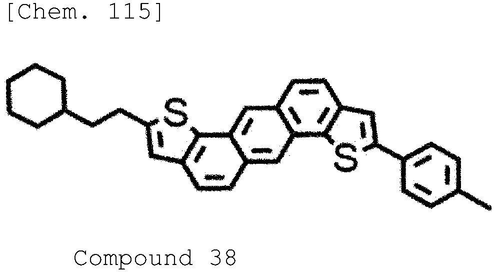

### Example 48 Synthesis of 2-(2-Cyclohexylethyl)-8-(4-hexylphenyl)anthra[1,2-b:5,6-b']dithiophene (compound 39)

In a nitrogen atmosphere, 40.0 mg (0.0834 mmol) of compound 3, synthesized in Example 3, 5.00 mg (0.0223 mmol) of palladium(II) acetate (FUJIFILM Wako Pure Chemical Corporation), 20.3 mg (0.0435 mmol) of Ruphos (Tokyo Chemical Industry Co., Ltd.), 37.0 mg (0.330 mmol) of potassium tert-butoxide (Tokyo Chemical Industry Co., Ltd.) and 58.4 mg (0.283 mmol) of 4-hexylphenylboronic acid (Tokyo Chemical Industry Co., Ltd.) were added to a 50-mL Schlenk tube. 5.0 mL of toluene (FUJIFILM Wako Pure Chemical Corporation, dehydration grade) and 0.5 mL of water were added to the tube, and the resulting mixture was stirred for 4 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (toluene). The resulting solid was purified by recycling HPLC, and the product was purified by recrystallization from toluene/heptane, giving 18.7 mg of a yellow solid of 2-(2-cyclohexylethyl)-8-(4-hexylphenyl)anthra[1,2-b:5,6-b']dithiophene (compound 39) (yield, 39%).

¹H NMR (CDCl₃): δ = 8.67 (s, 1H), 8.60 (s, 1H), 7.89-7.86 (m, 2H), 7.77 (d, J = 8.8 Hz, 1H), 7.73-7.70 (m, 3H), 7.65 (s, 1H), 7.29 (m, 2H), 7.16 (s, 1H), 3.03 (t, J = 7.8 Hz, 2H), 2.67 (t, J = 7.6 Hz, 2H), 1.87-1.63 (m, 9H), 1.44-1.17 (m, 10H), 1.05-0.95 (m, 2H), 0.93-0.89 (m, 3H).

Melting point: 196°C

### (Compound 39)

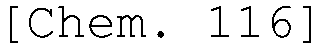

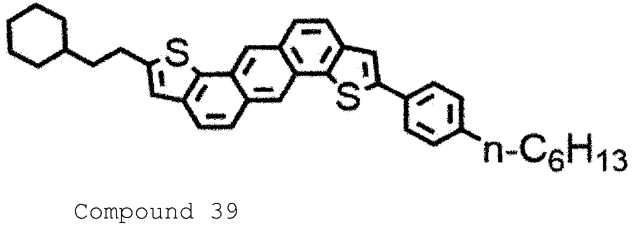

### Example 49 Production 9 of Solution for Forming an Organic Semiconductor Layer, Organic Semiconductor Layer and Organic Thin Film Transistor

A solution for forming an organic semiconductor layer was prepared by the same method as in Example 25, except that the 2-(3-cyclohexylpropyl)-8-pentylanthra[1,2-b:5,6-b']dithiophene synthesized in Example 34 (compound 25) was used. This solution for forming an organic semiconductor layer remained in solution form even after 10 hours at 25°C (the concentration of compound 25 was 0.2% by weight), confirming that compound 25 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 1.18 cm²/V·sec.

### Example 50 Production 10 of Solution for Forming an Organic Semiconductor Layer, Organic Semiconductor Layer and Organic Thin Film Transistor

A solution for forming an organic semiconductor layer was prepared by the same method as in Example 25, except that the 2-(3-cyclopentylpropyl)-8-hexylanthra[1,2-b:5,6-b']dithiophene synthesized in Example 40 (compound 31) was used. This solution for forming an organic semiconductor layer remained in solution form even after 10 hours at 25°C (the concentration of compound 31 was 0.2% by weight), confirming that compound 31 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.49 cm²/V·sec.

### Synthesis Example 11 Synthesis of 4,4,5,5-Tetramethyl-2-(4-octylphenylethyl)-1,3,2-dioxaborolane

In a nitrogen atmosphere, 52.0 mL (46.8 mmol) of an 8.5% borane/THF solution (Tokyo Chemical Industry Co., Ltd.) and 5.60 mL (23 mmol) of 4-n-octylstyrene (Tokyo Chemical Industry Co., Ltd.) were added to a 100-ml two-neck flask with cooling on ice, and the resulting mixture was stirred for 1 hour at 0°C and for 2 hours at room temperature. 6.2 mL of water was added with cooling on ice, and the resulting mixture was stirred for 2 hours at room temperature. The resulting mixture was concentrated under reduced pressure, ethyl acetate and an aqueous solution of sodium hydrogencarbonate were added, and phase separation was performed. The organic phase was washed with saline solution, dried over anhydrous sodium sulfate and concentrated under reduced pressure. In this manner, (4-octylphenylethyl)boronic acid was synthesized.

In a nitrogen atmosphere, the resulting boronic acid was added to a 300-mL recovery flask and stirred at room temperature with 5.78 g (48.7 mmol) of pinacol (Tokyo Chemical Industry Co., Ltd.) and 40.0 mL of diethyl ether (FUJIFILM Wako Pure Chemical Corporation, dehydration grade) added thereto. 6.09 g (50.6 mmol) of magnesium sulfate (FUJIFILM Wako Pure Chemical Corporation) was added to the flask, and the resulting mixture was stirred for 4 hours at room temperature. The solid was isolated by filtration under reduced pressure, and the filtrate was concentrated under reduced pressure. The resulting residue was purified by silica gel column chromatography (solvent; toluene/hexane), giving 2.40 g of a colorless liquid of 4,4,5,5-tetramethyl-2-(4-octylphenylethyl)-1,3,2-dioxaborolane (yield, 29%).

¹H NMR (CDCl₃): δ = 7.12 (d, J = 8.1 Hz, 2H), 7.07 (d, J = 8.1 Hz, 2H), 2.72 (t, J = 8.1 Hz, 2H), 2.56 (t, J = 7.6 Hz, 2H), 1.62-1.55 (m, 2H), 1.34-1.26 (m, 10H), 1.23 (s, 12H), 1.14 (t, J = 8.3 Hz, 2H), 0.89 (t, J = 6.7 Hz, 3H). 4,4,5,5-Tetramethyl-2-(4-octylphenylethyl)-1,3,2-dioxaborolane

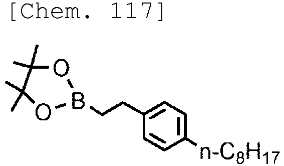

### Example 51 Synthesis of 2-(2-Cyclohexylethyl)-8-(4-octylphenylethyl)anthra[1,2-b:5,6-b']dithiophene (compound 40)

In a nitrogen atmosphere, 37.7 mg (0.0786 mmol) of compound 3, synthesized in Example 3, 6.20 mg (0.0276 mmol) of palladium(II) acetate (FUJIFILM Wako Pure Chemical Corporation), 25.8 mg (0.0553 mmol) of Ruphos (Tokyo Chemical Industry Co., Ltd.), 32.5 mg (0.290 mmol) of potassium tert-butoxide (Tokyo Chemical Industry Co., Ltd.) and 103 mg (0.299 mmol) of the 4,4,5,5-tetramethyl-2-(4-octylphenylethyl)-1,3,2-dioxaborolane synthesized in Synthesis Example 11 were added to a 50-mL Schlenk tube. 5.0 mL of toluene (FUJIFILM Wako Pure Chemical Corporation, dehydration grade) and 0.5 mL of water were added to the tube, and the resulting mixture was stirred for 20 hours at 80°C. The reaction mixture was allowed to cool at room temperature, undissolved residue was removed using diatomaceous earth (FUJIFILM Wako Pure Chemical Corporation), and the resulting solution was concentrated under reduced pressure. The resulting residue was purified by silica gel column chromatography (toluene/hexane = 1/2). The resulting solid was purified by recycling HPLC, and the product was purified by recrystallization from heptane, giving 25.8 mg of a yellow solid of 2-(2-cyclohexylethyl)-8-(4-octylphenylethyl)anthra[1,2-b:5,6-b']dithiophene (compound 40) (yield, 53%).

¹H NMR (CDCl₃): δ = 8.59 (s, 2H), 7.85 (d, J = 8.8 Hz, 1H), 7.84 (dd, J = 8.8 Hz, 1H), 7.70 (d, J = 8.8 Hz, 1H), 7.69 (d, J = 8.7 Hz, 1H), 7.19-7.12 (m, 6H), 3.31 (t, J = 7.4 Hz, 2H), 3.11 (t, J = 8.6 Hz, 2H), 3.03 (t, J = 7.2 Hz, 2H), 2.59 (t, J = 7.6 Hz, 2H), 1.85-1.81 (m, 2H), 1.76-1.58 (m, 7H), 1.47-1.37 (m, 1H), 1.36-1.16 (m, 13H), 1.05-0.95 (m, 2H), 0.89 (t, J = 6.5 Hz, 3H).

Melting point: 161°C

### (Compound 40)

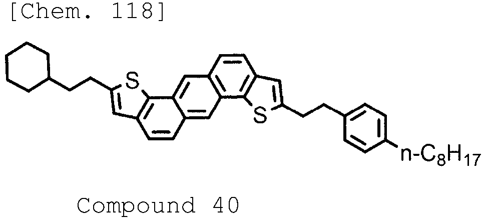

### Example 52 Production 11 of Solution for Forming an Organic Semiconductor Layer, Organic Semiconductor Layer and Organic Thin Film Transistor

A solution for forming an organic semiconductor layer was prepared by the same method as in Example 25, except that the 2-(2-cyclohexylethyl)-8-(4-octylphenylethyl)anthra[1,2-b:5,6-b']dithiophene synthesized in Example 51 (compound 40) was used. This solution for forming an organic semiconductor layer remained in solution form even after 10 hours at 25°C (the concentration of compound 40 was 0.2% by weight), confirming that compound 40 is a compound suitable for film formation by drop casting and inkjet printing.

Using the resulting solution for forming an organic semiconductor layer, a bottom gate-bottom contact p-type organic thin film transistor was fabricated using the materials and film formation methods for the individual structural components presented in Example 25.

The evaluation of the transmission characteristics of this transistor element was performed, and the hole carrier mobility was found to be 0.66 cm²/V·sec.

### Comparative Example 1

### (Production of Solution for Forming an Organic Semiconductor Layer)

In air, 0.44 mg of 2,7-diphenyl[1]benzothieno[3,2-b][1]benzothiophene (Sigma-Aldrich Co. LLC) and 434 mg of toluene (FUJIFILM Wako Pure Chemical Industries Corporation, pure grade) were added to a 10-ml sample tube. The resulting mixture was heated to 50°C and allowed to cool at room temperature (25°C), with solid precipitation observed. It was found that this compound is a compound unsuitable for film formation by drop casting and inkjet printing due to its low solubility.

### Comparative Example 2

### (Production of Solution for Forming an Organic Semiconductor Layer)

In air, a solution for forming an organic semiconductor layer was prepared in a 10-ml sample tube by the same method as in Example 25 using 2,8-dioctylanthra[1,2-b:5,6-b']dithiophene synthesized by the method described in WO 2021/177417. This solution for forming an organic semiconductor layer remained in solution form even after 10 hours at 25°C (0.20% by weight), confirming that this compound is a compound suitable for film formation by drop casting and inkjet printing.

### (Production of Organic Semiconductor Layer and Organic Thin Film Transistor)

Using this solution for forming an organic semiconductor layer, the fabrication of a bottom gate-bottom contact p-type organic thin film transistor was attempted using the materials and film formation methods for the individual structural components presented in Example 25. No thin film, however, was formed, preventing the fabrication of a bottom gate-bottom contact p-type organic thin film transistor.

### Comparative Example 3

For 2,8-di(2-phenylethyl)anthra[1,2-b:5,6-b']dithiophene, 2-(2-phenylethyl)anthra[1,2-b:5,6-b']dithiophene and 2-(2-(4-methylphenyl)ethyl)anthra[1,2-b:5,6-b']dithiophene synthesized by the methods described in WO 2021/177417 (compound 10, compound 15 and compound 16, respectively, in the publication), solubility in octane was evaluated by the method in Example 24. The solubility was less than 0.10% by weight for all of them; it was found that these are compounds with low solubility in octane.

### Industrial Applicability

The aromatic compound according to the present invention provides high carrier mobility and is also superior in heat resistance and solubility. The compound, therefore, is expected to be applied as a material for semiconductor devices, typified by organic thin film transistors.

### Reference Signs List

1: Organic semiconductor layer
2: Substrate
3: Gate electrode
4: Gate insulating layer
5: Source electrode
6: Drain Electrode
1001: Bottom gate-top contact organic thin film transistor
1002: Bottom gate-bottom contact organic thin film transistor
1003: Top gate-top contact organic thin film transistor
1004: Top gate-bottom contact organic thin film transistor

## Claims

1. An aromatic compound indicated by either formula (1-I) or (1-II) below. 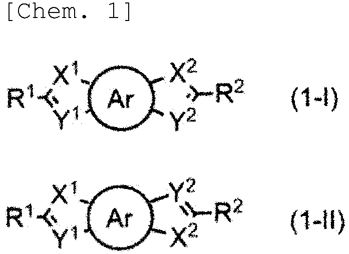
[(In the formula, Ar denotes a monocyclic ring or a fused ring system of two to six rings. X¹ and X² each independently denote one selected from the group consisting of an oxygen atom, a sulfur atom, a selenium atom, NR³ and CR⁴=CR⁵. Y¹ and Y² each independently denote either CR⁶ or a nitrogen atom. R¹ to R⁶ each independently denote one in the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group or a group represented by formula (2) below, with at least one of R¹ to R⁶ being a group represented by formula (2) below.) 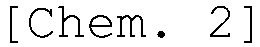 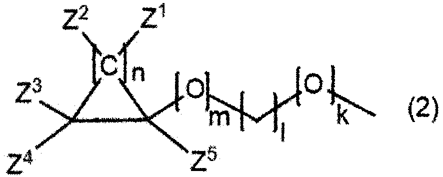
(In the formula, k and m each independently denote 0 or 1, n denotes an integer of 1 to 8, and l denotes an integer of 1 to 20. Z¹ to Z⁵ indicate one selected from the group consisting of a hydrogen atom, a halogen atom and a C1 to C20 alkyl group, being the same or different at each occurrence.)]

2. The aromatic compound according to Claim 1, wherein of R¹ to R⁶, only one or only both of R¹ and R² are groups indicated by the formula (2).

3. The aromatic compound according to Claim 1, wherein the aromatic compound indicated by formula (1-I) or (1-II) is a compound indicated by one selected from the group consisting of formulae (3-1) to (3-6) below. 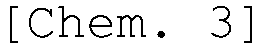 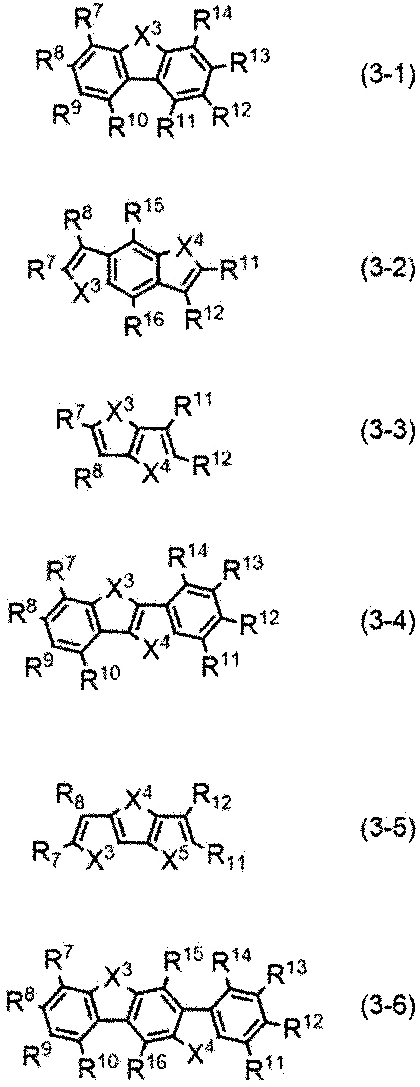
[(In the formulae, X³, X⁴ and X⁵ each independently denote one selected from the group consisting of an oxygen atom, a sulfur atom, a selenium atom, a single bond, NR¹⁷ and CR¹⁸=CR¹⁹. Of combinations of adjacent two of R⁷ to R¹⁰, only one constitutes formula (4) below, and of combinations of adjacent two of R¹¹ to R¹⁴, only one constitutes formula (4-2) below, each forming a five-membered ring or a six-membered ring. Those of R⁷ to R¹⁴ that do not constitute formula (4) below or formula (4-2) below, and R¹⁵ to R¹⁹, each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2).) 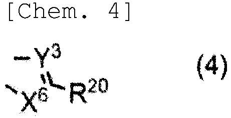
(In the formula, X⁶ denotes one selected from the group consisting of an oxygen atom, a sulfur atom, a selenium atom, CR²¹=CR²² and NR²³, Y³ denotes either CR²⁴ or a nitrogen atom. R²¹ to R²⁴ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2), and R²⁰ is a group represented by the formula (2).) 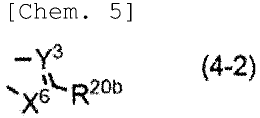
(In the formula, X⁶ and Y³ are synonymous with X⁶ and Y³ in the formula (4), and R^{20b} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2).)]

4. The aromatic compound according to Claim 3, wherein the aromatic compound indicated by formula (1-I) or (1-II) is a compound indicated by the formula (3-1) or the formula (3-2).

5. The aromatic compound according to Claim 3, wherein the formula (4-2) is formula (4-3) below. 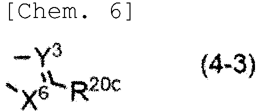 (In the formula, X⁶ and Y³ are synonymous with X⁶ and Y³ in the formula (4), and R^{20c} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a group represented by a C4 to C26 aryl group.)

6. The aromatic compound according to Claim 3, wherein the formula (4-2) is formula (4-4) below. 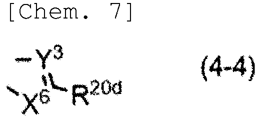 (In the formula, X⁶ and Y³ are synonymous with X⁶ and Y³ in the formula (4), and R^{20d} is a group represented by the formula (2).)

7. The aromatic compound according to Claim 1, wherein the aromatic compound indicated by formula (1-I) or (1-II) is a compound indicated by formula (5) below or formula (5-2) below. 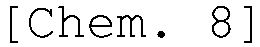 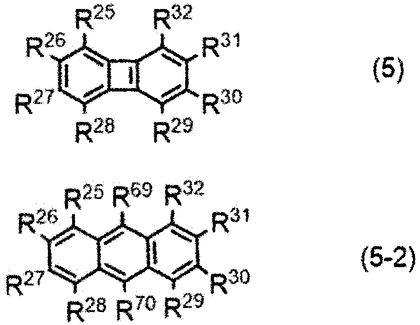
[(In the formula, only one of combinations of adjacent two of R²⁵ to R²⁸ constitutes formula (6) below, and only one of combinations of adjacent two of R²⁹ to R³² constitutes formula (6-2) below, each forming a five-membered ring or a six-membered ring. Those of R²⁵ to R³² that do not constitute formula (6) below or formula (6-2) below, and R⁶⁹ and R⁷⁰, each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2).) 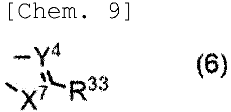
(In the formula, X⁷ denotes an oxygen atom, a sulfur atom, a selenium atom, CR³⁴=CR³⁵ or NR³⁶. Y⁴ denotes either CR³⁷ or a nitrogen atom. R³⁴ to R³⁷ each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2), and R³³ is a group represented by the formula (2).) 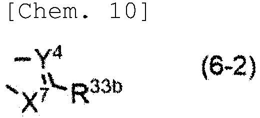
(In the formula, X⁷ and Y⁴ are synonymous with X⁷ and Y⁴ in the formula (6), and R^{33b} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2).)]

8. The aromatic compound according to Claim 7, wherein the formula (6-2) is formula (6-3) below. 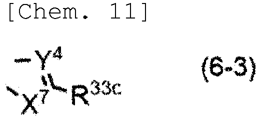 (In the formula, X⁷ and Y⁴ are synonymous with X⁷ and Y⁴ in the formula (6), and R^{33c} denotes one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group and a C4 to C26 aryl group.)

9. The aromatic compound according to Claim 7, wherein the formula (6-2) is formula (6-4) below. 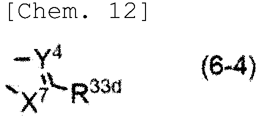 (In the formula, X⁷ and Y⁴ are synonymous with X⁷ and Y⁴ in the formula (6), and R^{33d} is a group represented by the formula (2).)

10. The aromatic compound according to Claim 1, wherein the aromatic compound indicated by formula (1-I) or (1-II) is a compound indicated by one selected from the group consisting of formulae (7-1) to (7-6) below. 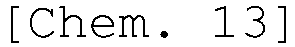 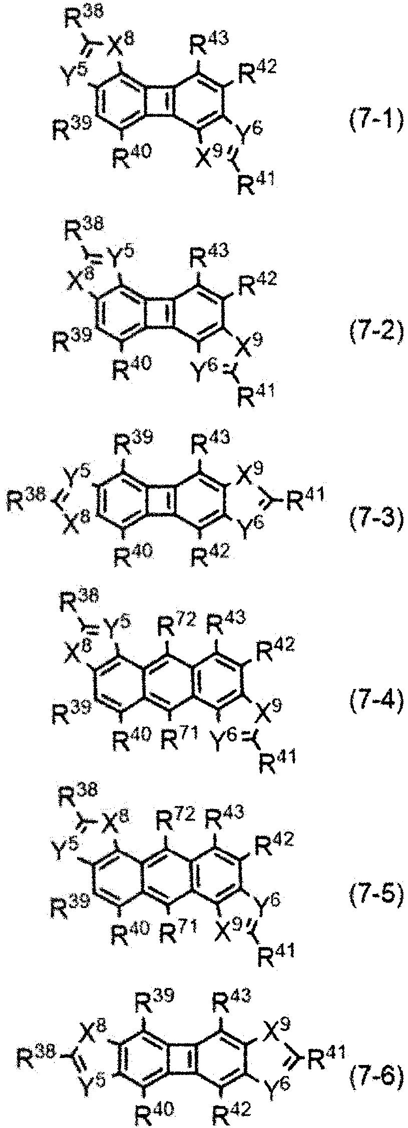 (In the formulae, X⁸ and X⁹ each independently denote an oxygen atom, a sulfur atom, a selenium atom or NR⁴⁴. Y⁵ and Y⁶ each independently denote CR⁴⁵ or a nitrogen atom. R³⁸ to R⁴⁵, R⁷¹ and R⁷² each independently denote one selected from the group consisting of a hydrogen atom, a halogen atom, a C1 to C20 alkyl group, a C2 to C20 alkenyl group, a C2 to C20 alkynyl group, a C4 to C22 alkadienyl group, a C4 to C22 alkadiynyl group, a C4 to C26 aryl group and a group represented by the formula (2), with at least one of R³⁸ or R⁴¹ being a group represented by the formula (2).)

11. The aromatic compound according to Claim 10, wherein each of R³⁸ and R⁴¹ is independently one selected from the group consisting of a group represented by the formula (2), a hydrogen atom and a fluorine atom, and R³⁹, R⁴⁰, R⁴² to R⁴⁵, R⁷¹ and R⁷² are hydrogen atoms.

12. The aromatic compound according to Claim 10, wherein R³⁸ and R⁴¹ are groups represented by the formula (2) and R³⁹, R⁴⁰, R⁴² to R⁴⁵, R⁷¹ and R⁷² are hydrogen atoms.

13. A solution for forming an organic semiconductor layer, the solution comprising the aromatic compound according to any one of Claims 1 to 12.

14. An organic semiconductor layer comprising the aromatic compound according to any one of Claims 1 to 12.

15. An organic thin film transistor comprising the aromatic compound according to any one of Claims 1 to 12.
